# EUROPÄISCHE PATENTSCHRIFT

(11) **EP 1 451 177 B1**
(45) Veröffentlichungstag und Bekanntmachung des Hinweises auf die Patenterteilung: **21.03.2007**
(21) Anmeldenummer: 02798318.8
(22) Anmeldetag: 02.12.2002
(51) Int. Cl.: C07D 403/04, C07D 409/14, C07D 401/14, C07D 413/14, C07D 403/14, C07D 405/14, C07D 493/08, A61K 31/4025, A61K 31/404, A61P 25/06, A61P 25/28

(54) **SUBSTITUIERTE 2-PYRROLIDIN-2-YL-1H-INDOL-DERIVATIVE FÜR DIE BEHANDLUNG VON MIGRÄNE**
SUBSTITUTED 2-PYRROLIDINE-2-YL-1H-INDOLE DERIVATIVES FOR THE TREATMENT OF MIGRAINE
DERIVES SUBSTITUES DE 2-PYRROLIDIN-2-YL-1H-INDOLE POUR LE TRAITEMENT DE LA MIGRAINE

(30) Priorität: 06.12.2001 DE 10159922
(43) Veröffentlichungstag der Anmeldung: 01.09.2004
(73) Patentinhaber: Grünenthal GmbH, 52078 Aachen (DE)
(72) Erfinder: SUNDERMANN, Corinna, 52074 Aachen (DE); SUNDERMANN, Bernd, 52074 Aachen (DE); BUSCHMANN, Helmut, E-08960 Sant Just Desvern (Barcelona) (ES); HENNIES, Hagen-Heinrich, 52152 Simmerath (DE)
(86) Internationale Anmeldenummer: PCT/EP2002/013613
(87) Internationale Veröffentlichungsnummer: WO 2003/048156

(56) Entgegenhaltungen:
- WO-A-93/11106
- WO-A-93/14087
- WO-A-95/06636
- FUERSTNER A ET AL: "SITE SELECTIVE FORMATION OF LOW-VALENT TITANIUM REAGENTS: AN INSTANT PROCEDURE FOR THE REDUCTIVE COUPLING OF OXO AMIDES TO INDOLES" JOURNAL OF ORGANIC CHEMISTRY, AMERICAN CHEMICAL SOCIETY. EASTON, US, Bd. 59, Nr. 18, 1994, Seiten 5215-5229, XP002044965 ISSN: 0022-3263

## Beschreibung

Die Erfindung betrifft substituierte 2-Pyrrolidin-2-yl-1H-indol-Derivate, Verfahren zur ihrer Herstellung, Arzneimittel, die diese Verbindungen enthalten, und die Verwendung der erfindungsgemäßen 2-Pyrrolidin-2-yl-1H-indol-Derivate zur Herstellung eines Medikaments zur NO-Synthase-Inhibierung und zur Behandlung von u.a. Migräne.

Stickstoffmonoxid (NO) reguliert zahlreiche physiologische Prozesse, unter anderem die Neurotransmission, Relaxation und Proliferation von glatter Muskulatur, die Adhäsion und Aggregation von Thrombozyten sowie die Gewebeverletzung und Entzündung. Aufgrund der Vielzahl von Signalfunktionen wird NO mit einer Reihe von Krankheiten in Verbindung gebracht (s. z.B. L. J. Ignarro, *Angew. Chem.* (1999), 111, 2002-2013 und F. Murad, *Angew. Chem. Int. Ed.* (1999), 111, 1976-1989). Eine wichtige Rolle bei der therapeutischen Beeinflussung dieser Krankheiten spielt dabei das für die physiologische Bildung von NO verantwortliche Enzym, die NO-Synthase (NOS). Bislang wurden drei verschiedene Isoformen der NO-Synthase, nämlich die beiden konstitutiven Formen nNOS und eNOS sowie die induzierbare Form iNOS, identifiziert (A. J. Hobbs, A. Higgs, S. Moncada, *Annu. Rev. Pharmacol. Toxicol.* (1999), 39, 191-220; I. C. Green, P.-E. Chabrier, DDT (1999), 4, 47-49; P.-E. Chabrier et al., *Cell. Mol. Life Sci.* (1999), 55, 1029-1035).

Die Hemmung der NO-Synthase eröffnet neue Therapieansätze für verschiedene Krankheiten, die mit NO in Zusammenhang stehen (A. J. Hobbs et al., *Annu. Rev. Pharmacol. Toxicol.* (1999), 39, 191-220; I. C. Green, P.-E. Chabrier, DDT (1999), 4, 47-49; P.-E. Chabrier et al., *Cell. Mol. Life Sci.* (1999), 55, 1029-1035), wie beispielsweise Migräne (L. L. Thomsen, J. Olesen, *Clinical Neuroscience* (1998), 5, 28-33; L. H. Lassen et al., *The Lancet* (1997), 349, 401-402), septischer Schock, neurodegenerative Erkrankungen wie Multiple Sklerose, Morbus Parkinson, Morbus Alzheimer oder Morbus Huntington, Entzündungen, Entzündungsschmerz, cerebrale Ischämie, Diabetes, Meningitis und Arteriosklerose. Darüber hinaus kann die NOS-Inhibierung einen Effekt auf die Wundheilung, auf Tumoren und auf die Angiogenese haben sowie eine unspezifische Immunität gegen Mikroorganismen bewirken (A. J. Hobbs et al., *Annu. Rev. Pharmacol. Toxicol.* (1999), 39, 191-220).

Bislang bekannte Wirkstoffe, die die NO-Synthase hemmen, sind neben L-NMMA und L-NAME - d.h. Analoga des L-Arginins, aus dem in-vivo unter Beteiligung von NOS NO und Citrullin gebildet werden - u.a. S-Methyl-L-citrullin, Aminoguanidin, S-Methylisoharnstoff, 7-Nitroindazol und 2-Mercaptoethylguanidin (A. J. Hobbs et al., *Annu. Rev. Pharmacol. Toxicol.* (1999), 39, 191-220).

WO 95/06636 offenbart Pyrrolidin-indolderivate für die Behandlung von Migräne, Schmerzen und Depressionen. Bei diesen Verbindungen ist der Pyrrolidinring in 3-Position mit dem Indolring verknüpft.

Der vorliegenden Erfindung lag demgegenüber als eine Aufgabe zugrunde, neue wirksame NOS-Inhibitoren zur Verfügung zu stellen.

Überraschenderweise wurde gefunden, daß substituierte 2-Pyrrolidin-2-yl-1 H-indol-Derivate der allgemeinen Formel (I) 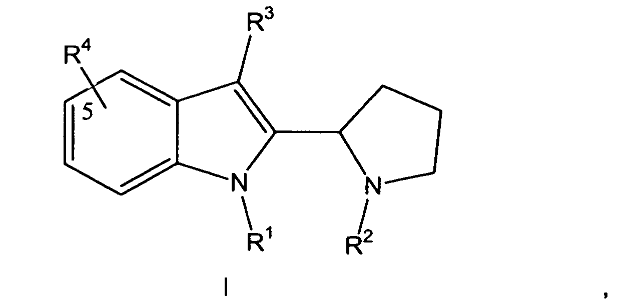 wobei
- R¹: H, Alkyl, Cycloalkyl, Alkyl-Cycloalkyl, Alkyl-Aryl oder Alkyl-Heterocyclyl bedeutet;
- R²: H, Alkyl, Cycloalkyl, Alkyl-Cycloalkyl, Alkyl-Aryl, Alkyl-Heterocyclyl, C(=O)-R²⁰ oder SO₂-R²¹ bedeutet;
- R³: Alkyl, Aryl oder Heterocyclyl bedeutet;
- R⁴: H, F, Cl, Br, I, -CN, OR⁴⁰, Alkyl, Cycloalkyl oder NO₂ bedeutet;
- R²⁰: Alkyl, Cycloalkyl, Alkyl-Cycloalkyl, Aryl, Alkyl-Aryl, Heterocyclyl, Alkyl-Heterocyclyl, OR²⁰⁰ oder NHR²⁰¹ bedeutet;
- R²¹: Alkyl, Aryl, Alkyl-Aryl, Heterocyclyl oder Alkyl-Heterocyclyl bedeutet;
- R⁴⁰: H, Alkyl, Cycloalkyl, Alkyl-Cycloalkyl, Aryl oder Alkyl-Aryl bedeutet;
- R²⁰⁰: Alkyl, Cycloalkyl, Alkyl-Cycloalkyl, Aryl, Alkyl-Aryl, Heterocyclyl oder Alkyl-Heterocyclyl bedeutet; und
- R²⁰¹: Alkyl, Aryl, Alkyl-Aryl, Heterocyclyl oder Alkyl-Heterocyclyl bedeutet;
wobei die Verbindungen der Formel (I) in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate; vorliegen;
sehr wirksame NOS-Inhibitoren darstellen.

Diese Verbindungen sind neu mit Ausnahme von 2-(3-Phenyl-1 H-indol-2-yl)-1-(trifluoracetyl)-pyrrolidin (A. Fürstner et al., *J. Org. Chem.* (1994), 59, 5215-5229); α-Methyl-2-(1-methyl-2-pyrrolidinyl)-indol-3-essigsäuremethylester, 3-(1-Cyanoethyl)-2-(1-methyl-2-pyrrolidinyl)-indol und 2-(1-Methyl-2-pyrrolidinyl)-3-vinylindol (G. H. Foster, J. Harley-Mason, *Chem. Commun.* (1968), 1440-1441); die als solche im Stand der Technik beschrieben wurden, ohne daß eine NOS-inhibierende (oder irgendeine andere pharmakologische oder therapeutische) Wirkung dieser Substanzen offenbart worden wäre. Daher sind auch diese letztgenannten Verbindungen Gegenstand dieser Erfindung, soweit ihre Verwendung in einem Arzneimittel und insbesondere zur Herstellung eines Medikaments zur Inhibierung von NO-Synthase und zur Behandlung von Migräne, septischem Schock, Multipler Sklerose, Morbus Parkinson, Morbus Alzheimer, Morbus Huntington, Entzündungen, Entzündungsschmerz, cerebraler Ischämie, Diabetes, Meningitis, Arteriosklerose und/oder für die Wundheilung betroffen sind.

Die Begriffe "Alkyl", "C₁₋₆-Alkyl", "C₁₋₄-Alkyl" bzw. "C₁₋₃-Alkyl" umfassen im Sinne dieser Erfindung acyclische gesättigte oder ungesättigte Kohlenwasserstoffreste, die verzweigt oder geradkettig sowie unsubstituiert oder einfach substituiert oder mehrfach gleich oder verschieden substituiert sein können, mit (wie im Fall von C₁₋₆-Alkyl) 1 bis 6 (d.h. 1, 2, 3, 4, 5 oder 6), mit (wie im Fall von C₁₋₄-Alkyl) 1 bis 4 (d.h. 1, 2, 3 oder 4) bzw. mit (wie im Fall von C₁₋₃-Alkyl) 1 bis 3 (d.h. 1, 2 oder 3) C-Atomen. Dabei umfaßt der Begriff "Alkyl" "Alkanyle", "Alkenyle" und "Alkinyle". "Alkenyle" weisen mindestens eine C-C-Doppelbindung (aber keine C-C-Dreifachbindung) und "Alkinyle" mindestens eine C-C-Dreifachbindung auf, während "Alkanyle" keine C-C-Mehrfachbindungen aufweisen. "C₁₋₄-Alkanyle" sind Alkanyle mit 1, 2, 3 oder 4 Kohlenstoffatomen, "C₁₋₈-Alkanyle" Alkanyle mit 1, 2, 3, 4, 5, 6, 7 oder 8 Kohlenstoffatomen, während "C₂₋₈-Alkenyle" Alkenyle mit 2, 3, 4, 5, 6, 7 oder 8 C-Atomen sind. Vorteilhaft ist Alkyl aus der Gruppe ausgewählt, die Methyl, Ethyl, n-Propyl, 2-Propyl, n-Butyl, isoButyl, sek-Butyl, tert-Butyl, n-Pentyl, iso-Pentyl, neo-Pentyl, n-Hexyl, 2-Hexyl, n-Heptyl, n-Octyl, n-Nonyl, n-Decyl, n-Dodecyl; Ethenyl (Vinyl), Ethinyl, Propenyl (-CH₂CH=CH₂, -CH=CH-CH₃, -C(=CH₂)-CH₃), Propinyl (-CH₂-C≡CH, -C≡C-CH₃), Butenyl, Butinyl, Pentenyl, Pentinyl, Hexenyl, Hexinyl, Octenyl und Octinyl umfaßt.

"Cycloalkyl" bedeutet im Sinne dieser Erfindung einen alicyclischen gesättigten oder ungesättigten Kohlenwasserstoff-Rest, wobei der Rest unsubstituiert oder einfach substituiert oder mehrfach gleich oder verschieden substituiert und ggf. benzokondensiert sein kann; ferner umfaßt der Ausdruck "Cycloalkyl" auch bi-, tri- oder polycyclische Alicyclen, z.B. Adamantyl. "C₃₋₈-Cycloalkyl" steht für ein Cycloalkyl mit 3, 4, 5, 6, 7 oder 8 C-Atomen im Cyclus. Beispielhaft steht Cycloalkyl für Cyclopropyl, Cyclobutyl, Cyclopentyl, Cyclohexyl, Cycloheptyl, Cyclooctyl, Cyclopentenyl, Cyclohexenyl, Cycloheptenyl, Cyclooctenyl oder Adamantyl.

Unter dem Ausdruck "Aryl" ist für die Zwecke der vorliegenden Erfindung ein Rest zu verstehen, der aus der Gruppe, die Phenyl, Naphthyl, Anthracenyl und Biphenyl umfaßt, ausgewählt ist und unsubstituiert oder einfach oder mehrfach gleich oder verschieden substituiert ist. Die ArylReste können auch mit weiteren gesättigten, (partiell) ungesättigten oder aromatischen Ringsystemen kondensiert sein. Jeder Aryl-Rest kann unsubstituiert oder einfach oder mehrfach substituiert vorliegen, wobei die Aryl-Substituenten gleich oder verschieden und in jeder beliebigen Position des Aryls sein können.

Der Ausdruck "Heterocyclyl" steht für einen monocyclischen oder polycyclischen organischen Rest, in dem mindestens ein Cyclus 1 Heteroatom oder 2, 3, 4 oder 5 gleiche oder verschiedene Heteroatome enthält, das/die aus der Gruppe, die N, O und S enthält, ausgewählt ist/sind, wobei der Rest gesättigt oder ungesättigt ist und unsubstituiert oder einfach substituiert oder mehrfach gleich oder verschieden substituiert ist. Beispiele für Heterocyclyl-Reste im Sinne dieser Erfindung sind monocyclische fünf-, sechs- oder siebengliedrige organische Reste mit 1 Heteroatom oder 2, 3, 4 oder 5 gleichen oder verschiedenen Heteroatomen, bei dem/denen es sich um Stickstoff, Sauerstoff und/oder Schwefel handelt, und deren benzokondensierte Analoga. Eine Untergruppe der Heterocyclyl-Reste bilden die "Heteroaryl"-Reste, bei denen es sich um solche Heterocyclyle handelt, in denen der mindestens eine Cyclus, der das/die Heteroatom/e enthält, heteroaromatisch ist. Jeder Heteroaryl-Rest kann unsubstituiert oder einfach substituiert oder mehrfach gleich oder verschieden substituiert vorliegen. Beispiele für Heterocyclyl-Reste im Sinne der vorliegenden Erfindung sind Pyrrolidinyl, Tetrahydrofuryl, Piperidinyl, Piperazinyl, Morpholinyl, Imidazolidinyl und Benzopiperidinyl. Beispiele für Heterocyclyle, die zugleich Heteroaryl-Reste darstellen, sind Pyrrolyl, Pyrazolyl, Imidazolyl, Pyridazinyl, Pyrimidinyl, Pyrazinyl und insbesondere Furanyl, Thienyl, Isoxazolyl und Pyridinyl sowie deren benzokondensierte Analoga. Alle diese Reste können jeweils unsubstituiert oder einfach oder mehrfach gleich oder verschieden substituiert vorliegen.

Die Ausdrücke "Alkyl-Cycloalkyl", "(C₁₋₃-Alkyl)-C₃₋₈-Cycloalkyl", "Alkyl-Heterocyclyl", "Alkyl-Aryl", "C₁₋₄-Alkanyl-Aryl" und "C₁₋₄-Alkenyl-Aryl" bedeuten für die Zwecke der vorliegenden Erfindung, daß der Cycloalkyl-, Heterocyclyl- bzw. Aryl-Rest über eine Alkyl-Gruppe bzw. Alkanyl- oder Alkenyl-Gruppe an die mit ihm substituierte Verbindung gebunden ist.

Im Zusammenhang mit "Alkyl", "Alkanyl", "Alkenyl", "Alkinyl" und "Cycloalkyl" versteht man unter dem Begriff "substituiert" im Sinne dieser Erfindung die Substitution eines Wasserstoffatoms durch beispielsweise F, Cl, Br, I, -CN, NH₂, NH-Alkyl, NH-Alkyl-Aryl, N(Alkyl)₂, NO, NO₂, SH, S-Alkyl, S-CF₃, OH, O-Alkyl, O-CF₃, O-Aryl, O-Alkyl-Aryl, O-Alkyl-OH, O-Alkyl-O-Alkyl, O-Alkyl-O-Alkyl-OH, CO₂H, CO₂-Alkyl, CO₂-Alkyl-Aryl, SO-Alkyl, SO₂-Alkyl, Cycloalkyl, Aryl oder Heterocyclyl, wobei unter mehrfach substituierten Resten solche Reste zu verstehen sind, die entweder an verschiedenen oder an gleichen Atomen mehrfach, z.B. zwei- oder dreifach, substituiert sind, beispielsweise dreifach am gleichen C-Atom wie im Falle von CF₃, oder -CH₂CF₃ oder an verschiedenen Stellen wie im Falle von -CH(OH)-CH=CCl-CH₂Cl. Die Mehrfachsubstitution kann mit dem gleichen oder mit verschiedenen Substituenten erfolgen.

In Bezug auf "Aryl", "Heterocyclyl" sowie "Heteroaryl" versteht man im Sinne dieser Erfindung unter "einfach substituiert" oder "mehrfach substituiert" die ein- oder mehrfache, z.B. zwei-, drei- oder vierfache, Substitution eines oder mehrerer Wasserstoffatome des Ringsystems durch einen geeigneten Substituenten. Soweit die Bedeutung dieser geeigneten Substituenten im Zusammenhang mit "Aryl", "Heterocyclyl" oder "Heteroaryl" nicht an anderer Stelle der Beschreibung oder in den Ansprüchen definiert ist, sind geeignete Substituenten F, Cl, Br, l, -CN, NH₂, NH-Alkyl, NH-Aryl, NH-Alkyl-Aryl, N(Alkyl)₂, NO, NO₂, SH, S-Alkyl, S-CF₃, OH, O-Alkyl, O-CF₃, O-Cycloalkyl, O-Aryl, O-Alkyl-Cycloalkyl, O-Alkyl-Aryl, CHO, C(=O)C₁₋₆-Alkyl, C(=O)CF₃, C(=O)Aryl, C(=O)-C₁₋₆-Alkyl-Aryl, CO₂H, CO₂Alkyl, S(O)-Alkyl, SO₂-Alkyl, CF₃, Alkyl, Cycloalkyl, Aryl und/oder Heterocyclyl; an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten.

"Benzokondensiert" bedeutet für die Zwecke der vorliegenden Erfindung, daß ein Benzol-Ring an einen anderen Cyclus ankondensiert ist.

Pharmazeutisch annehmbare Salze bzw. physiologisch verträgliche Salze im Sinne dieser Erfindung sind solche Salze der erfindungsgemäßen Verbindungen gemäß der allgemeinen Formel (I), die bei pharmazeutischer Verwendung physiologisch- insbesondere bei Anwendung am Säugetier und/oder Menschen - verträglich sind. Solche pharmazeutisch annehmbaren Salze können beispielsweise mit anorganischen oder organischen Säuren oder für den Fall, daß die erfindungsgemäßen Verbindungen Säuren, insbesondere Carbonsäuren sind, mit Basen gebildet werden.

Vorzugsweise werden die pharmazeutisch annehmbaren Salze der erfindungsgemäßen Verbindungen gemäß der allgemeinen Formel (I) mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure gebildet. Handelt es sich bei den erfindungsgemäßen Verbindungen um Säuren, insbesondere Carbonsäuren, können die pharmazeutisch annehmbaren Salze auch durch Umsetzung mit Basen, wie z.B. Natriumhydroxid, Natriumhydrogencarbonat oder Natriumcarbonat, gebildet werden. Bei den gebildeten Salzen handelt es sich u.a. um Hydrochloride, Hydrobromide, Phosphate, Carbonate, Hydrogencarbonate, Formiate, Acetate, Oxalate, Succinate, Tartrate, Fumarate, Citrate und Glutaminate bzw. um Natrium-Salze. Besonders bevorzugt sind die Hydrochlorid-Salze. Ebenfalls bevorzugt sind die Hydrate der erfindungsgemäßen Verbindungen, die z.B. durch Kristallisation aus wäßriger Lösung erhalten werden können.

Alle erfindungsgemäßen Verbindungen enthalten mindestens ein Asymmetriezentrum, nämlich das mit einem Stern (*) gekennzeichnete C-2-Atom in Nachbarschaft des Pyrrolidin-N-Atoms in Formel (I*):

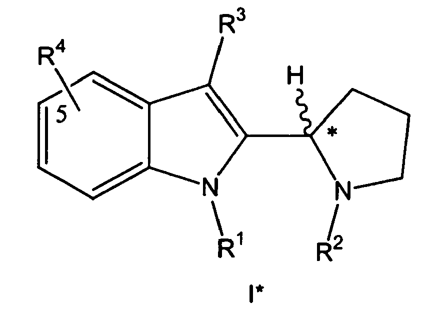

Daher können die erfindungsgemäßen Verbindungen der allgemeinen Formel (I) in Form ihrer Racemate, in Form der reinen Enantiomeren und/oder Diastereomeren oder in Form von Mischungen dieser Enantiomeren bzw. Diastereomeren vorliegen, und zwar sowohl in Substanz als auch als pharmazeutisch annehmbare Salze oder Solvate dieser Verbindungen. Die Mischungen können in jedem beliebigen Mischungsverhältnis der Stereoisomeren vorliegen. Bevorzugt liegen die Verbindungen der allgemeinen Formel (I) als enantiomerenreine Verbindungen vor.

Bevorzugte Verbindungen der allgemeinen Formel (I) sind solche, in denen
- R¹: H, Alkyl, Cycloalkyl oder Alkyl-Cycloalkyl bedeutet;
- R²: C(=O)-R²⁰ oder SO₂-R²¹ bedeutet;
- R³: Aryl bedeutet;
- R⁴: H, F, Cl, Br, I, -CN, OR⁴⁰, Alkyl, Cycloalkyl oder NO₂, jeweils in 5-Position des Indolrings, bedeutet;
- R²⁰: Alkyl, Cycloalkyl, Alkyl-Cycloalkyl, Aryl, Alkyl-Aryl, Heterocyclyl oder Alkyl-Heterocyclyl bedeutet;
- R²¹: Alkyl, Aryl, Alkyl-Aryl, Heterocyclyl oder Alkyl-Heterocyclyl bedeutet;
und
- R⁴⁰: H, Alkyl, Cycloalkyl, Alkyl-Cycloalkyl, Aryl oder Alkyl-Aryl bedeutet.

Besonders bevorzugt sind Verbindungen der allgemeinen Formel (I), worin
- R¹: H oder Alkyl bedeutet;
- R²: C(=O)-R²⁰ oder SO₂-R²¹ bedeutet;
- R³: Phenyl bedeutet;
- R⁴: H, F, Cl, Br oder I, jeweils in 5-Position des Indolrings, bedeutet;
- R²⁰: C₁₋₈-Alkanyl, C₂₋₈-Alkenyl, C₁₋₄-Alkanyl-CO₂-C₁₋₄-Alkyl, C₁₋₄-Alkanyl-O-R²⁰², C₁₋₄-Alkanyl-NR²⁰³R²⁰⁴, C₃₋₈-Cycloalkyl, 1-Adamantyl, 2-Adamantyl, -(C₁₋₃-Alkyl)-C₃₋₈-Cycloalkyl, Aryl¹, Aryl², C₁₋₄-Alkanyl-Aryl³, C₂₋₄-Alkenyl-Aryl⁴; oder unsubstituiertes oder substituiertes Furanyl, Pyrazolyl, Isoxazolyl oder Pyridinyl; oder 4,7,7-Trimethyloxabicyclo[2.2.1]heptan-3-on, -CH₂-Imidazolidin-2,4-dion bedeutet;
- R²¹: Aryl⁵, unsubstituiertes oder substituiertes Thienyl bedeutet oder für 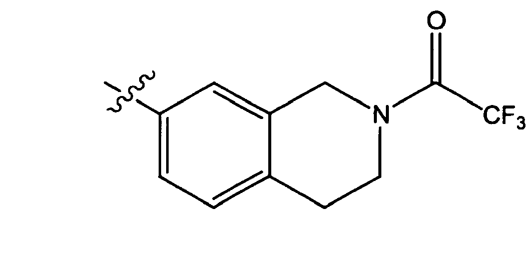 steht;
- R²⁰²: H, C₁₋₄-Alkanyl, C₁₋₄-Alkanyl-OH, C₁₋₄-Alkanyl-O-C₁₋₄-Alkanyl oder Aryl bedeutet;
- R²⁰³ und R²⁰⁴: unabhängig voneinander C₁₋₆-Alkyl bedeuten oder mit dem N-Atom ein 5-, 6- oder 7-gliedriges gesättigtes Heterocyclyl bilden;
- Aryl¹: für 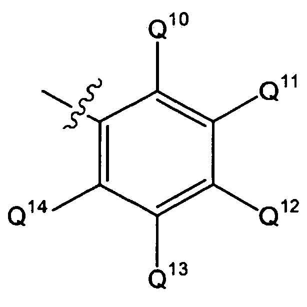 steht;
- Aryl²: für 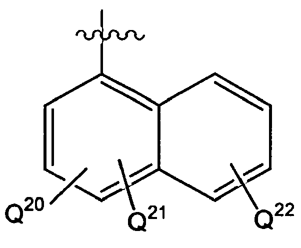 steht;
- Aryl³: für 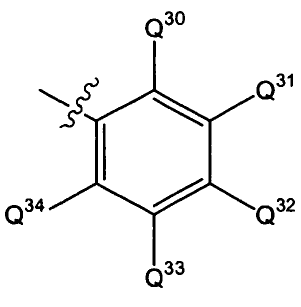 steht;
- Aryl⁴: für 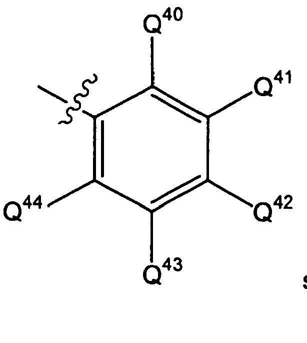 steht;
- Aryl⁵: für 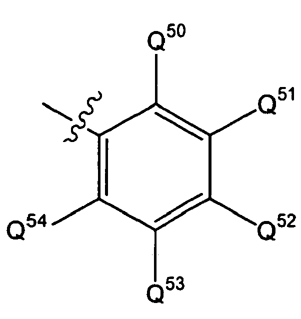 steht; und
Q¹⁰, Q¹¹, Q¹², Q¹³, Q¹⁴, Q²⁰, Q²¹, Q²², Q³⁰, Q³¹, Q³², Q³³, Q³⁴, Q⁴⁰, Q⁴¹, Q⁴², Q⁴³, Q⁴⁴, Q⁵⁰, Q⁵¹, Q⁵², Q⁵³ und Q⁵⁴
unabhängig voneinander H, F, Cl, Br, I, -CN, -NO₂, C₁₋₄-Alkyl, OH, O-C₁₋₄-Alkyl, SH oder S-C₁₋₄-Alkyl bedeuten.

Ganz besonders bevorzugte substituierte 2-Pyrrolidin-2-yl-1H-indol-Derivate sind solche, bei denen
- R¹: H, Methyl, But-2-in oder Diethylaminoethyl bedeutet;
- R²: C(=O)-R²⁰ oder SO₂-R²¹ bedeutet;
- R³: Phenyl bedeutet;
- R⁴: 5-H oder 5-Cl bedeutet;
- R²⁰: Methyl, Ethyl, n-Propyl, n-Butyl, 1-Methylpropyl, 2-Methylpropyl, tert-Butyl, n-Pentyl, 1-Methylbutyl, 2,2-Dimethylpropyl, 1-Propylbutyl, 1-Ethylpentyl, Prop-1-enyl, Prop-2-enyl, But-1-enyl, But-2-enyl, But-3-enyl, -(CH₂)₂-CO₂-Methyl, -(CH₂)₂-CO₂-Ethyl, -CH₂-O-CH₃, -CH₂-O-(CH₂)₂-O-CH₃, -CH₂-O-Phenyl, -CH₂-O-(4-Chlorphenyl), -CH(CH₃)-O-Phenyl, -(CH₂)₃-O-Phenyl, Dimethylaminomethyl, Cyclopropyl, Cyclobutyl, Cyclopentyl, Cyclohexyl, 1-Adamantyl, -CH₂-Cyclopropyl, -CH₂-Cyclopentyl, Aryl¹, 1-Naphthyl, CH₂-Aryl³, -CH(C₂H₅)-Aryl³, -CH=CH-Aryl⁴, Furan-2-yl, Furan-3-yl, 5-tert-Butyl-2-methyl-furan-3-yl, 2,5-Dimethyl-furan-3-yl, Pyrazol-4-yl, Pyrazol-5-yl, 1-tert-Butyl-3-methyl-pyrazol-5-yl, 1-Phenyl-3-propyl-pyrazol-4-yl, Isoxazol-3-yl, Isoxazol-4-yl, Isoxazol-5-yl, 5-Methyl-4-(2-chlorphenyl)-isoxazol-3-yl, 5-Methyl-3-(2-chlor-6-fluorphenyl)-isoxazol-4-yl, Pyridin-2-yl, Pyridin-3-yl, Pyridin-4-yl, 2-Chlorpyridin-3-yl, 6-Chlorpyridin-3-yl, 2-(CH₃-S-)-pyridin-3-yl, 2-Methyl-6-trifluormethyl-pyridin-3-yl, 4,7,7-Trimethyloxabicyclo[2.2.1]heptan-3-on oder -CH₂-Imidazolidin-2,4-dion bedeutet;
- R²¹: Aryl⁵, Thien-2-yl, Thien-3-yl oder 5-Chlor-thien-2-yl bedeutet oder für 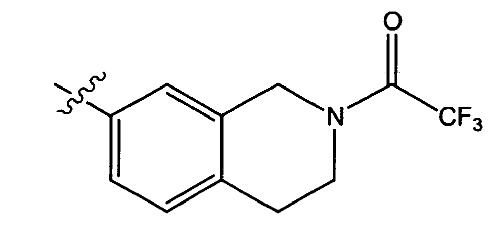 steht;
- Aryl¹: für 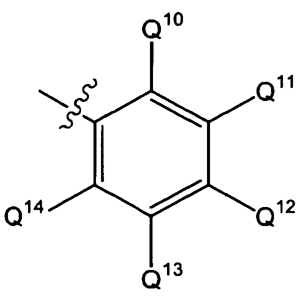 steht;
- Aryl³: für 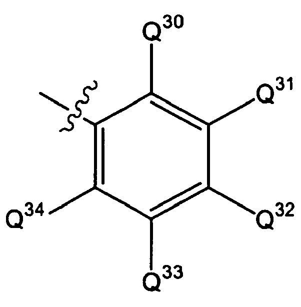 steht;
- Aryl⁴: für 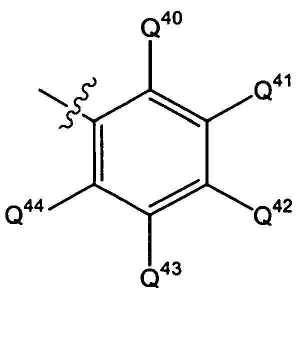 steht;
- Aryl⁵: für 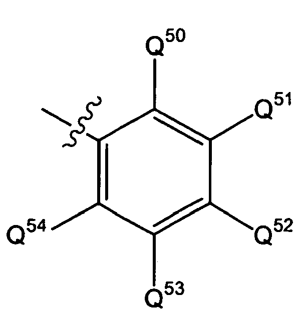 steht;
- Q¹⁰, Q¹¹, Q¹², Q¹³ und Q¹⁴: unabhängig voneinander H, F, Cl, Br, -NO₂, Methyl, Trifluormethyl, Ethyl, n-Propyl, 1-Methylethyl, O-CH₃, O-CF₃, -O-C₂H₅, CH₃ oder S-CF₃ bedeuten;
- Q³⁰, Q³¹, Q³², Q³³ und Q³⁴: unabhängig voneinander H, Cl oder O-CH₃ bedeuten;
- Q⁴⁰, Q⁴¹, Q⁴², Q⁴³ und Q⁴⁴: unabhängig voneinander H, Cl, CH₃ oder CF₃ bedeuten; und
- Q⁵⁰, Q⁵¹, Q⁵², Q⁵³ und Q⁵⁴: unabhängig voneinander H, F, Cl, Br, Methyl, Ethyl, n-Propyl, 1-Methylethyl, O-CH₃, O-CF₃ O-CH₂CH₃, O-CH₂CH₂CH₃, O-CH₂CH₂CH₂CH₃ bedeuten.

Zu den am meisten bevorzugten erfindungsgemäßen Verbindungen der allgemeinen Formel (I) gehören solche, worin
- R¹: H, Methyl, But-2-in oder Diethylaminoethyl bedeutet;
- R²: C(=O)-R²⁰ oder SO₂-R²¹ bedeutet;
- R³: Phenyl bedeutet;
- R⁴: 5-H oder 5-Cl bedeutet;
- R²⁰: n-Propyl, n-Butyl, n-Pentyl, 1-Methylbutyl, 2,2-Dimethylpropyl, 1-Propylbutyl, 1-Ethylpentyl, But-3-enyl, -(CH₂)₂-CO₂-Methyl, -(CH₂)₂-CO₂-Ethyl, -CH₂-O-CH₃, -CH₂-O-(CH₂)₂-O-CH₃, -CH₂-O-Phenyl, -CH₂-O-(4-Chlorphenyl), -CH(CH₃)-O-Phenyl, -(CH₂)₃-O-Phenyl, Dimethylaminomethyl, Cyclopropyl, Cyclobutyl, Cyclohexyl, 1-Adamantyl, -CH₂-Cyclopentyl, 4-Fluorphenyl, 4-Chlorphenyl, 2-Bromphenyl, 3-Bromphenyl, 2,3-Difluorphenyl, 3-Fluor-4-trifluormethylphenyl, 3-Fluor-6-trifluormethylphenyl, 3-Fluor-4-methylphenyl, 3,5-Difluorphenyl, 2-Chlor-4-nitrophenyl, 2-Chlor-5-trifluormethylphenyl, 4-Brom-3-methylphenyl, 2,3-Difluor-4-methylphenyl, 2,6-Difluor-3-methylphenyl, 2,3,4,5,6-Pentafluorphenyl, 4-Nitrophenyl, 4-Methyl-3-nitrophenyl, 3-Methylphenyl, 4-Methylphenyl, 2,3-Dimethylphenyl, 3-Trifluormethylphenyl, 2,5-Trifluormethylphenyl, 3,5-Trifluormethylphenyl, 4-Ethylphenyl, 4-n-Propylphenyl, 3-Methoxyphenyl, 3,5-Dimethoxyphenyl, 3-Trifluormethoxyphenyl, 4-Trifluormethoxyphenyl, 2-Ethoxyphenyl, 4-Ethoxyphenyl, 4-S-CF₃-Phenyl, Benzyl, 4-Chlorbenzyl, 3-Methoxybenzyl, 2,5-Dimethoxybenzyl, -CH(C₂H₅)-Phenyl, -CH=CH-(2-Chlorphenyl), -CH=CH-(3-Trifluormethylphenyl), Furan-2-yl, 5-tert-Butyl-2-methyl-furan-3-yl, 2,5-Dimethyl-furan-3-yl, 1-tert-Butyl-3-methyl-pyrazol-5-yl, 1-Phenyl-3-propyl-pyrazol-4-yl, Isoxazol-5-yl, 5-Methyl-4-(2-chlorphenyl)-isoxazol-3-yl, 5-Methyl-3-(2-chlor-6-fluorphenyl)-isoxazol-4-yl, Pyridin-2-yl, 2-Chlorpyridin-3-yl, 6-Chlorpyridin-3-yl, 2-(CH₃-S-)-pyridin-3-yl, 2-Methyl-6-trifluormethyl-pyridin-3-yl, 4,7,7-Trimethyloxabicyclo[2.2.1]heptan-3-on oder -CH₂-Imidazolidin-2,4-dion bedeutet; und
- R²¹: 3-Chlor-4-fluorphenyl, 2-Chlorphenyl, 4-Chlorphenyl, 3-Chlor-2-methylphenyl, 3-Bromphenyl, 2,3,5,6-Tetramethylphenyl, 4-n-Butylphenyl, 4-Methoxyphenyl, 2,5-Dimethoxyphenyl, 4-Trifluormethoxyphenyl, 4-(n-Butoxy)-phenyl, Thien-2-yl, 5-Chlor-thien-2-yl bedeutet oder für steht, 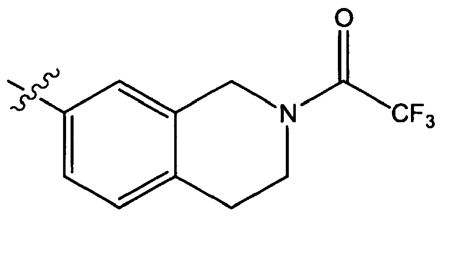 insbesondere solche substituierten 2-Pyrrolidin-2-yl-1 H-indol-Derivate, die aus der Gruppe ausgewählt sind, die umfaßt:
{2-[5-Chlor-1-(2-diethylamino-ethyl)-3-phenyl-1 H-indol-2-yl]-pyrrolidin-1-yl}-furan-2-yl-methanon;
4-{2-[5-Chlor-1-(2-diethylamino-ethyl)-3-phenyl-1 H-indol-2-yl]-pyrrolidin-1-yl}-4-oxo-buttersäureethylester;
{2-[5-Chlor-1-(2-diethylamino-ethyl)-3-phenyl-1H-indol-2-yl]-pyrrolidin-1-yl}-(4-fluor-phenyl)-methanon;
{2-[5-Chlor-1-(2-diethylamino-ethyl)-3-phenyl-1H-indol-2-yl]-pyrrolidin-1-yl}-cyclobutyl-methanon;
{2-[5-Chlor-1-(2-diethylamino-ethyl)-3-phenyl-1H-indol-2-y)]-pyrrolidin-1-yl}-(6-chlor-pyridin-3-yl)-methanon;
1-{2-[5-Chlor-1-(2-diethylamino-ethyl)-3-phenyl-1 H-indol-2-yl]-pyrrolidin-1-yl}-hexan-1-on;
{2-[5-Chlor-1-(2-diethylamino-ethyl)-3-phenyl-1H-indol-2-yl]-pyrrolidin-1-yl}-(3,5-dimethoxy-phenyl)-methanon;
{2-[5-Chlor-1-(2-diethylamino-ethyl)-3-phenyl-1H-indol-2-yl]-pyrrolidin-1-yl}-(4-ethoxy-phenyl)-methanon;
1-[2-(5-Chlor-1-methyl-3-phenyl-1 H-indol-2-yl)-pyrrolidin-1-yl]-2-(2-methoxy-ethoxy)-ethanon;
[2-(5-Chlor-1-methyl-3-phenyl-1H-indol-2-yl)-pyrrolidin-1-yl]-cyclopropyl-methanon; und
1-[2-(1-But-2-inyl-5-chlor-3-phenyl-1H-indol-2-yl)-pyrrolidin-1-yl]-2-methoxy-ethanon.

Die vorliegende Erfindung betrifft auch Verfahren zur Herstellung der erfindungsgemäßen Verbindungen der allgemeinen Formel (I).

So sind substituierte 2-Pyrrolidin-2-yl-1 H-indol-Derivate der Formel (I),
worin
- R¹: H, Alkyl, Cycloalkyl, Alkyl-Cycloalkyl, Alkyl-Aryl oder Alkyl-Heterocyclyl bedeutet;
- R²: Alkyl, Cycloalkyl, Alkyl-Cycloalkyl, Alkyl-Aryl, Alkyl-Heterocyclyl, C(=O)-R²⁰ oder SO₂-R²¹ bedeutet;
- R³: Alkyl, Aryl oder Heterocyclyl bedeutet;
- R⁴: H, F, Cl, Br, I, -CN, OR⁴⁰, Alkyl, Cycloalkyl oder NO₂ bedeutet;
- R²⁰: Alkyl, Cycloalkyl, Alkyl-Cycloalkyl, Aryl, Alkyl-Aryl, Heterocyclyl, Alkyl-Heterocyclyl, OR²⁰⁰ oder NHR²⁰¹ bedeutet;
- R²¹: Alkyl, Aryl, Alkyl-Aryl, Heterocyclyl oder Alkyl-Heterocyclyl bedeutet;
- R⁴⁰: H, Alkyl, Cycloalkyl, Alkyl-Cycloalkyl, Aryl oder Alkyl-Aryl bedeutet;
- R²⁰⁰: Alkyl, Cycloalkyl, Alkyl-Cycloalkyl, Aryl, Alkyl-Aryl, Heterocyclyl oder Alkyl-Heterocyclyl bedeutet; und
- R²⁰¹: Alkyl, Aryl, Alkyl-Aryl, Heterocyclyl oder Alkyl-Heterocyclyl bedeutet; dadurch herstellbar, daß in einem Verfahrensschritt (A) ein 2-Pyrrolidin-2-yl-1 H-indol-Derivat der allgemeinen Formel (II) 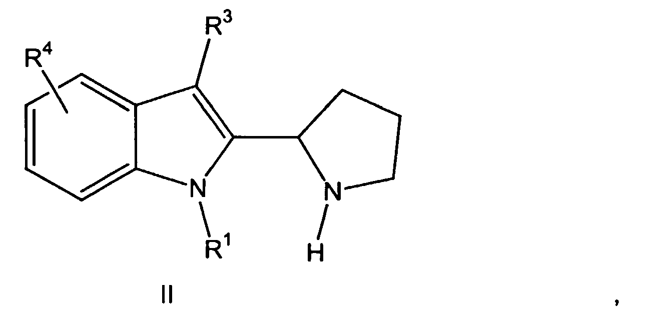 worin
R¹, R³ und R⁴ wie oben definiert sind, mit einer Verbindung der allgemeinen Formel (III)

R²-Hal III

worin
R² Alkyl, Cycloalkyl, Alkyl-Cycloalkyl, Alkyl-Aryl, Alkyl-Heterocyclyl, C(=O)-R²⁰ oder SO₂-R²¹ bedeutet; und
Hal für Chlor oder Brom steht;
umgesetzt wird. Im allgemeinen wird das Halogenid (III) im Überschuß, bevorzugt mit 1,05 bis 1,5 Äquivalenten (III) (bezogen auf (II)), mit der Verbindung der Formel (II) umgesetzt. Üblicherweise wird das Indol-Derivat (II) in einem geeigneten Lösungsmittel, bevorzugt einem halogenierten organischen Lösungsmittel, z.B. Dichlormethan, vorgelegt und das Halogenid (III) zugegeben. Die Reaktion wird bei 0 °C bis 150 °C, vorzugsweise bei 20 °C bis 40 °C. durchgeführt und dauert zwischen 10 min und 12 h. Die Umsetzung verläuft auch dann in guten Ausbeuten unter Substitution des Pyrrolidin-N-Wasserstoffs durch R², wenn auch R¹ in Formel (II) H bedeutet; der Pyrrolidin-Stickstoff erweist sich als basischer und somit wesentlich reaktiver als der Indol-Stickstoff, so daß eine vorherige Schützung des Indol-Stickstoffs mit einer geeigenten Schutzgruppe zwar möglich, aber nicht erforderlich ist Insbesondere wenn R² C(=O)-R²⁰ oder SO₂-R²¹ bedeutet, ist es vorteilhaft. als Verbindung (III) das entsprechende Säurehalogenid (Hal = Cl in R²-Hal (III)) einzusetzen, und zwar bevorzugt in einer Menge von ca. 1,05 Äquivalenten (bezogen auf das Indol (II)); die Umsetzung mit dem Indol (II) erfolgt dabei bevorzugt in Gegenwart von ca. 1,1 Äquivalenten Triethylamin und katalytischen Mengen (0,01 bis 0,1 Äquivalenten) DMAP (4-Dimethylaminopyridin) in Dichlormethan als Lösungsmittel.

Erfindungsgemäße Verbindungen der Formel (I), worin
- R¹ und R²: H bedeuten;
- R³: Alkyl, Aryl oder Heterocyclyl bedeutet;
- R⁴: H, F, Cl, Br, I, -CN, OR⁴⁰, Alkyl, Cycloalkyl oder NO₂ bedeutet; und
- R⁴⁰: H, Alkyl, Cycloalkyl, Alkyl-Cycloalkyl, Aryl oder Alkyl-Aryl bedeutet; werden in einem Verfahrensschritt (B) durch Umsetzung eines 2-Pyrrolidin-2-yl-1 H-indol-Derivats der allgemeinen Formel (IV) 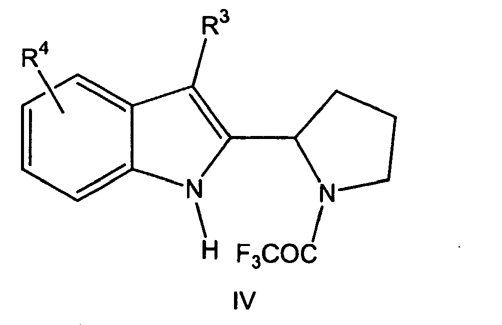 worin
- R³ und R⁴: wie oben definiert sind;
mit Kaliumcarbonat oder Natriumhydroxid erhalten. Vorzugsweise wird dabei die Base als wäßrige Lösung und im Überschuß eingesetzt.

Erfindungsgemäße Verbindungen der Formel (I), worin
- R¹: Alkyl, Cycloalkyl, Alkyl-Cycloalkyl, Alkyl-Aryl oder Alkyl-Heterocyclyl bedeutet (d.h. ungleich H ist);
- R²: H bedeutet;
- R³: Alkyl, Aryl oder Heterocyclyl bedeutet;
- R⁴: H, F, Cl, Br, I, -CN, OR⁴⁰, Alkyl, Cycloalkyl oder NO₂ bedeutet; und
- R⁴⁰: H, Alkyl, Cycloalkyl, Alkyl-Cycloalkyl, Aryl oder Alkyl-Aryl bedeutet; werden erfindungsgemäß in einem Verfahrensschritt (C) durch Reaktion eines 2-Pyrrolidin-2-yl-1 H-indol-Derivats der allgemeinen Formel (V) 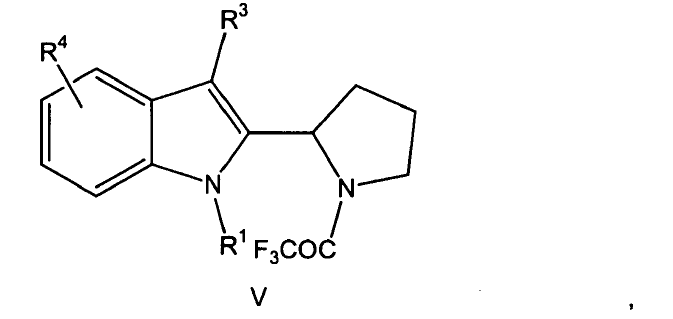 worin
R¹, R³ und R⁴ wie oben definiert sind;
mit Kaliumcarbonat oder Natriumhydroxid hergestellt. Vorzugsweise wird dabei die Base (d.h. K₂CO₃ oder NaOH) als wäßrige Lösung und im Überschuß eingesetzt.

2-Pyrrolidin-2-yl-1 H-indol-Derivate der allgemeinen Formel (V) mit
- R¹: Alkyl, Cycloalkyl, Alkyl-Cycloalkyl, Alkyl-Aryl oder Alkyl-Heterocyclyl;
- R³: Alkyl, Aryl oder Heterocyclyl;
- R⁴: H, F, Cl, Br, I, -CN, OR⁴⁰, Alkyl, Cycloalkyl oder NO₂; und
- R⁴⁰: H, Alkyl, Cycloalkyl, Alkyl-Cycloalkyl, Aryl oder Alkyl-Aryl;
werden in einem Verfahrensschritt (D) ausgehend von einem 2-Pyrrolidin-2-yl-1 H-indol-Derivat der allgemeinen Formel (IV) 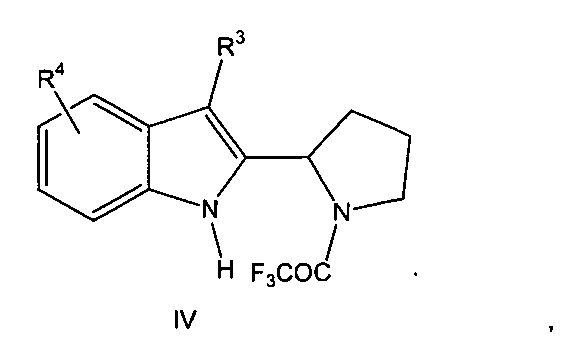 worin
- R³ und R⁴: wie oben definiert sind;
durch Umsetzung mit einer Verbindung der allgemeinen Formel (VI)

R¹-Hal VI

worin
R¹ wie oben definiert ist und Hal Cl, Br oder I bedeutet, erhalten. Dabei wird das Halogenid (VI) im allgemeinen im Überschuß eingesetzt. Zur Verbesserung von Geschwindigkeit bzw. Umsatz der Reaktion ist es bevorzugt, diese Umsetzung in Gegenwart eines geringfügigen Überschusses (z.B. 1,05 bis 1,2 Äquivalente) einer starken Base, z.B. Natriumhydrid, insbesondere in einem etherischen Lösungsmittel wie Tetrahydrofuran (THF) auszuführen.

Es ist ferner bevorzugt, die Verfahrensschritte (D) und (C) als zweistufige Reaktionssequenz ausgehend von einer wie oben definierten Verbindung der allgemeinen Formel (IV) über die korrespondierende Verbindung (V) zu der Verbindung der Formel (I), worin R¹ Alkyl, Cycloalkyl, Alkyl-Cycloalkyl, Alkyl-Aryl oder Alkyl-Heterocyclyl bedeutet; R² H bedeutet; R³ Alkyl, Aryl oder Heterocyclyl bedeutet; R⁴ H, F, Cl, Br, I, -CN, OR⁴⁰, Alkyl, Cycloalkyl oder NO₂ bedeutet; und R⁴⁰ H, Alkyl, Cycloalkyl, Alkyl-Cycloalkyl, Aryl oder Alkyl-Aryl bedeutet; auszuführen. Daran kann gegebenenfalls als weiterer Reaktionsschritt der Verfahrensschritt (A) angeschlossen werden, wobei dann Verbindungen der Formel (I) mit R² ungleich H (d.h. mit Alkyl, Cycloalkyl, Alkyl-Cycloalkyl, Alkyl-Aryl, Alkyl-Heterocyclyl, C(=O)-R²⁰ oder SO₂-R²¹) erhalten werden.

Ferner ist es zur Herstellung von Verbindungen der Formel (I) mit R¹ gleich H und R² Alkyl, Cycloalkyl, Alkyl-Cycloalkyl, Alkyl-Aryl, Alkyl-Heterocyclyl, C(=O)-R²⁰ oder SO₂-R²¹ bevorzugt, in einer zweistufigen Reaktionssequenz eine Verbindung der Formel (IV) in einem Verfahrensschritt (B) in eine Verbindung der Formel (I) mit R¹ und R² gleich H zu überführen, um diese anschließend in einem Verfahrensschritt (A) mit einem geeigneten Halogenid R²-Hal (III) zu der gewünschten Verbindung mit R² ungleich H umzusetzen.

Die Trifluoracetyl-geschützten erfindungsgemäßen Verbindungen der Formel (IV) sind in Anlehnung an literaturbekannte Verfahren (A. Fürstner, A. Hupperts, *J. Am. Chem.* Soc. (1995), *117*, 4468-4475; A. Fürstner et al., *Org. Synth.* (1999), *76*, 142-150) zugänglich: Die käuflich oder durch dem Fachmann bekannte Standardsynthesemethoden erhältlichen Anilin-Derivate der allgemeinen Formel (VIII) 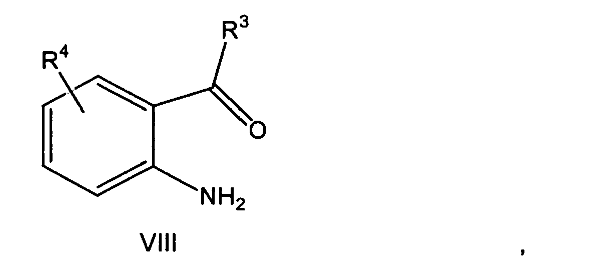 worin
- R³: Alkyl, Aryl oder Heterocyclyl bedeutet;
- R⁴: H, F, Cl, Br, l, -CN, OR⁴⁰, Alkyl, Cycloalkyl oder NO₂ bedeutet; und
- R⁴⁰: H, Alkyl, Cycloalkyl, Alkyl-Cycloalkyl, Aryl oder Alkyl-Aryl bedeutet,
werden in einem Verfahrensschritt (E) durch Umsetzung mit N-Trifluoracetylprolinalkylester (VII) 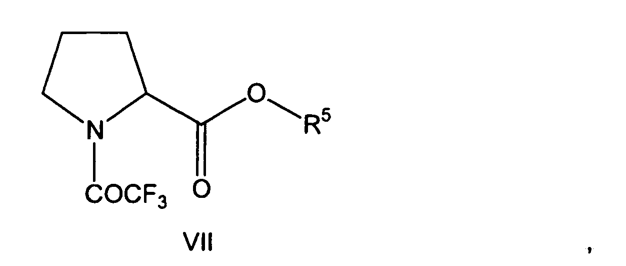 worin R⁵ Alkyl, insbesondere Methyl oder Ethyl, bedeutet,
in die entsprechende N-acylierte Verbindung der Formel (IX) 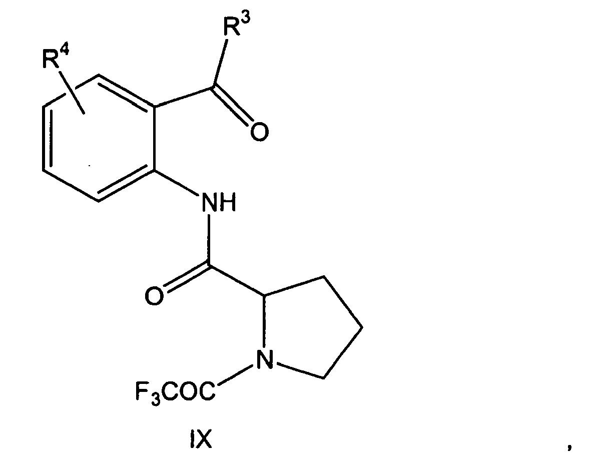 worin
R³ und R⁴ wie oben für Formel (VIII) definiert sind;
überführt. (Die Trifluoracetylprolinalkylester (VII) sind ohne weiteres aus den entsprechenden Prolinalkylestern und einem geeigneten Trifluoressigsäure-Derivat, z.B. Trifluoracetylchlorid, zugänglich.) Verbindung (IX) wird dann unter Reaktionsbedingungen, die denen von Fürstner et al. angegebenen Bedingungen (A. Fürstner, A. Hupperts, *J. Am. Chem.* Soc. (1995), *117,* 4468-4475; A. Fürstner et al., *Org. Synth.* (1999), 76, 142-150) entsprechen, in einem Verfahrensschritt (F) einer McMurry-Kupplung unterzogen. Eine geeignete Vorgehensweise sieht z.B. die Verwendung von ca. 2 Äquivalenten Titantrichlorid und ca. 4 Äquivalenten Zinkstaub - bezogen jeweils auf 1 Äquivalent Ketoamid (IX) - in Acetonitril vor. Es ist auch möglich, das Ketoamid (IX) mit 10 mol-% TiCl₃ und 4 Äquivalenten Zinkstaub mit CH₃CN zu suspendieren, anschließend 4 Äquivalente Trimethylsilylchlorid (TMSCI) hinzuzugeben und das Gemisch zum Rückfluß, z.B. für 15 min bis 2 h, zu erhitzen. Dies ergibt nach üblicher Aufarbeitung die gewünschte Verbindung der Formel (IV) in guten Ausbeuten. Setzt man im Verfahrensschritt (E) enantiomerenreines (R)-bzw. (S)-N-Trifluoracetylprolinalkylester (VII) ein, werden die Reaktionsprodukte der Verfahrensschritte (E) und (F) in hoher Stereoisomerenreinheit erhalten.

Bevorzugt sind die einzelnen erfindungsgemäßen Verfahrensschritte wie folgt zu mehrstufigen Synthesen zusammengestellt:

| | |
|---|---|
| Ausgehend von (IV): | 1. (B)→ 2. (A) oder |
| | 1. (D)→ 2. (C) und gegebenenfalls → 3. (A). |
| Ausgehend von (VIII): | 1. (E) → 2. (F) → 3. (B) → 4. (A) oder |
| | 1. (E) → 2. (F) → 3. (D) → 4. (C) und |
| | gegebenenfalls → 5. (A). |

Auch hier werden die entsprechenden Reaktionsprodukte in hoher Stereoisomerenreinheit erhalten, wenn Reaktionsschritt (E) mit enantiomerenreinem Trifluoracetylprolinalkylester (VII) ausgeführt wird.

Die erfindungsgemäßen Verbindungen der allgemeinen Struktur (I) (sowie der Formeln (II), (IV) und (V)) können sowohl in Substanz als auch als Salz isoliert werden. Die Substanzen der allgemeinen Struktur (I) werden üblicherweise nach Umsetzung gemäß dem oben dargelegten Verfahren und anschließender herkömmlicher Aufarbeitung erhalten. Die so gewonnenen Verbindungen können dann beispielsweise durch Versetzen mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder

Asparaginsäure, in das korrespondierende Salz überführt werden. Bei den gebildeten Salzen handelt es sich u.a. um Hydrochloride, Hydrobromide, Phosphate, Carbonate, Hydrogencarbonate, Formiate, Acetate, Oxalate, Succinate, Tartrate, Fumarate, Citrate und Glutaminate. Soweit es sich bei den Verbindungen der allgemeinen Formel (I) um Säuren, insbesondere Carbonsäuren, handelt, kann die Salzbildung durch Zugabe einer Base, z.B. Natriumhydroxid, NaHCO₃ oder Natriumcarbonat, herbeigeführt werden; für die (Carbon-)Säuren ist insbesondere die Bildung des Natriumsalzes bevorzugt. Die besonders bevorzugte Hydrochloridbildung kann insbesondere auch durch Versetzen der in einem geeigneten organischen Lösungsmittel gelösten Base (I) mit Trimethylsilylchlorid (TMSCI) herbeigeführt werden. Die Bildung von Natriumsalzen kann z.B. durch Titrieren der in einem geeigenten Lösungsmittel, beispielsweise Wasser-Methanol-Mischung, gelösten Verbindung (I) mit NatriumhydroxidLösung erfolgen.

Soweit die Verbindungen der allgemeinen Fomel (I) in dem erfindungsgemäßen Herstellungsverfahren als Racemate oder als Mischungen ihrer verschiedenen Stereoisomeren, insbesondere Enantiomeren und/oder Diastereomeren, erhalten werden, können diese Mischungen nach im Stand der Technik wohlbekannten Verfahren aufgetrennt werden. Geeignete Methoden sind u.a. chromatographische Trennverfahren, insbesondere Flüssigkeitschromatographie-Verfahren unter Normal- oder erhöhtem Druck, bevorzugt MPLC- und HPLC-Verfahren, sowie Verfahren der fraktionierten Kristallisation. Dabei können insbesondere einzelne Enantiomeren z.B. mittels HPLC an chiraler Phase oder mittels Kristallisation von mit chiralen Säuren, etwa (+)-Weinsäure, (-)-Weinsäure oder (+)-10-Camphersulfonsäure, oder - sofern es sich um Säuren handelt - mit chiralen Basen, etwa Brucin oder (-)-Ephedrin, gebildeten diastereomeren Salzen voneinander getrennt werden.

Die erfindungsgemäßen substituierten 2-Pyrrolidin-2-yl-1 H-indol-Derivate der Formel (I) haben sich als potente NOS-Inhibitoren erwiesen. Ein weiterer Gegenstand der vorliegenden Erfindung ist daher ein Arzneimittel, enthaltend mindestens eine Verbindung der wie oben definierten allgemeinen Formel (I), wobei die Verbindung (I) in Form des Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, in der in Formel (I) dargestellten Form oder in Form ihrer Säuren oder ihrer Basen oder in Form ihrer physiologisch verträglichen Salze, oder in Form ihrer Solvate, insbesondere der Hydrate, vorliegt. Bevorzugt liegt die Verbindung der Formel (I) in dem erfindungsgemäßen Arzneimittel als Hydrochlorid-Salz vor.

Ferner ist die Verwendung eines substituierten 2-Pyrrolidin-2-yl-1 H-indol-Derivats der wie oben definierten Formel (I) in Form des Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, in dargestellter Form oder in Form ihrer Säuren oder ihrer Basen oder in Form ihrer physiologisch verträglichen Salze, oder in Form ihrer Solvate, insbesondere der Hydrate, zur Herstellung eines Medikaments zur NO-Synthase-Inhibierung und insbesondere zur Herstellung eines Medikaments zur Prophylaxe und Therapie von Migräne Gegenstand der vorliegenden Erfindung.

Aufgrund ihrer NO-Synthase-inhibierenden Eigenschaften eignen sich die substituierten 2-Pyrrolidin-2-yl-1H-indol-Derivate der wie oben definierten Formel (I) auch zur Herstellung eines Medikaments zur Prophylaxe und/oder Therapie von septischem Schock, Multipler Sklerose, Morbus Parkinson, Morbus Alzheimer, Morbus Huntington, Entzündungen, Entzündungsschmerz, cerebraler Ischämie, Diabetes, Meningitis, Arteriosklerose und/oder für die Wundheilung.

Die erfindungsgemäßen Arzneimittel und Medikamente werden üblicherweise als pharmazeutische Zusammensetzungen hergestellt, die mindestens eine Verbindung der allgemeinen Formel (I), und zwar in Substanz und/oder als pharmazeutisch annehmbares Salz und/oder Solvat, und einen oder mehrere pharmazeutische Hilfsstoffe enthalten.

Die erfindungsgemäßen Arzneimittel, Medikamente und pharmazeutischen Zusammensetzungen können als flüssige, halbfeste oder feste Arzneiformen und in Form von z.B. Injektionslösungen, Tropfen, Säften, Sirupen, Sprays, Suspensionen, Granulaten, Tabletten, Pellets, Patches, Kapseln, Pflastern, Zäpfchen, Salben, Cremes, Lotionen, Gelen, Emulsionen oder Aerosolen vorliegen und verabreicht werden und enthalten neben mindestens einer erfindungsgemäßen Verbindung der allgemeinen Struktur (I) je nach galenischer Form pharmazeutische Hilfsstoffe, wie z.B. Trägermaterialien, Füllstoffe, Lösungsmittel, Verdünnungsmittel, oberflächenaktive Stoffe, Farbstoffe, Konservierungsstoffe, Sprengmittel, Gleitmittel, Schmiermittel, Aromen und/oder Bindemittel. Diese Hilfsstoffe können beispielsweise sein: Wasser, Ethanol, 2-Propanol, Glycerin, Ethylenglycol, Propylenglycol, Polyethylenglycol, Polypropylenglycol, Glucose, Fructose, Lactose, Saccharose, Dextrose, Melasse, Stärke, modifizierte Stärke, Gelatine, Sorbitol, Inositol, Mannitol, mikrokristalline Cellulose, Methylcellulose, Carboxymethylcellulose, Celluloseacetat, Schellack, Cetylalkohol, Polyvinylpyrrolidon, Paraffine, Wachse, pharmazeutisch annehmbare natürliche und synthetische Gummis, Akaziengummi, Alginate, Dextran, gesättigte und ungesättigte Fettsäuren, Stearinsäure, Magnesiumstearat, Zinkstearat, Glycerylstearat, Natriumlaurylsulfat, genießbare Öle, Sesamöl, Kokusnußöl, Erdnußöl, Sojabohnenöl, Lecithin, Natriumlactat, Polyoxyethylen- und -propylen-fettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid, Magnesiumoxid, Zinkoxid, Siliciumdioxid, Titanoxid, Titandioxid, Magnesiumsulfat, Zinksulfat, Calciumsulfat, Pottasche, Calciumphosphat, Dicalciumphosphat, Kaliumbromid, Kaliumiodid, Talkum, Kaolin, Pectin, Crospovidon, Agar und Bentonit.

Die Auswahl der Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich u.a. Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen der allgemeinen Struktur (I) in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Verbindungen der allgemeinen Struktur (I) verzögert freisetzen.

Die Herstellung der erfindungsgemäßen Arzneimittel und pharmazeutischen Zusammensetzungen erfolgt mit Hilfe von im Stand der Technik der pharmazeutischen Formulierung wohlbekannten Mitteln, Vorrichtungen, Methoden und Verfahren, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind.

So kann z.B. für eine feste Formulierung, wie eine Tablette, der Wirkstoff des Arzneimittels, d.h. eine Verbindung der allgemeinen Struktur (I) oder eines ihrer pharmazeutisch annehmbaren Salze, mit einem pharmazeutischen Träger, z.B. herkömmlichen Tabletteninhaltsstoffen, wie Maisstärke, Lactose, Saccharose, Sorbitol, Talkum, Magnesiumstearat, Dicalciumphosphat oder Gummi, und pharmazeutischen Verdünnungsmitteln, wie z.B. Wasser, gemischt werden, um eine feste Präformulierungs-Zusammensetzung zu bilden, die eine erfindungsgemäße Verbindung oder ein pharmazeutisch annehmbares Salz davon in homogener Verteilung enthält. Unter einer homogenen Verteilung wird hier verstanden, daß der Wirkstoff gleichmäßig über die gesamte Präformulierungs-Zusammensetzung verteilt ist, so daß diese ohne weiteres in gleich wirksame Einheitsdosis-Formen, wie Tabletten, Pillen oder Kapseln, unterteilt werden kann. Die feste Präformulierungs-Zusammensetzung wird anschließend in Einheitsdosis-Formen unterteilt. Die Tabletten oder Pillen des erfindungsgemäßen Arzneimittels bzw. der erfindungsgemäßen Zusammensetzungen können auch beschichtet oder auf andere Weise kompoundiert werden, um eine Dosisform mit verzögerter Freisetzung bereitzustellen. Geeignete Beschichtungsmittel sind u.a. polymere Säuren und Mischungen von polymeren Säuren mit Materialien wie z.B. Schellack, Cetylalkohol und/oder Celluloseacetat.

Ein weiterer Gegenstand der Erfindung betrifft ein Verfahren zur Behandlung von Krankheitszuständen in einem Säugetier und/oder Menschen, die durch Inhibierung der NO-Synthase positiv beeinflußt werden können, insbesondere von Migräne, von septischem Schock, Multipler Sklerose, Morbus Parkinson, Morbus Alzheimer, Morbus Huntington, Entzündungen, Entzündungsschmerz, cerebraler Ischämie, Diabetes, Meningitis, Arteriosklerose und/oder für die Wundheilung, wobei das Verfahren dadurch gekennzeichnet ist, daß eine therapeutisch wirksame Menge einer erfindungsgemäßen Verbindung der allgemeinen Formel (I) in dargestellter Form oder in Form ihrer Säuren oder ihrer Basen oder in Form der pharmazeutisch unbedenklichen, insbesondere physiologisch verträglichen Salze, oder in Form ihrer Solvate, insbesondere der Hydrate, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, verabreicht wird.

Die an den Patienten zu verabreichende Wirkstoffmenge variiert und ist abhängig vom Gewicht, dem Alter und der Krankheitsgeschichte des Patienten, sowie von der Applikationsart, der Indikation und dem Schweregrad der Erkrankung. Üblicherweise werden 0,1 bis 5000 mg/kg, insbesondere 1 bis 500 mg/kg, vorzugsweise 2 bis 250 mg/kg Körpergewicht wenigstens einer erfindungsgemäßen Verbindung der allgemeinen Formel (I) appliziert.

### Beispiele

### Herstellung erfindungsgemäßer substituierter 2-Pyrrolidin-2-yl-1H-indol-Derivate der Formel (I) (AAV 1)

1,1 Mol-Äquivalente des Anilinderivats (VIII) werden in Tetrahydrofuran (circa 2 ml pro mmol Amin) gelöst. Unter Eisbadkühlung werden dann 2,2 Mol-Äquivalente n-Butyllithium-Lösung (1,6 mol/l in Hexan) zugetropft und eine Stunde nachgerührt. Anschließend wird die Reaktionslösung mit Hilfe eines Trockeneisbades abgekühlt und eine Lösung des N-Trifluoracetylprolinethylesters (VII) (1 Mol-Äquivalent) in Tetrahydrofuran (circa 0,5 ml pro mmol Ester) zugetropft. Es wird eine Stunde unter Trockeneiskühlung nachgerührt und über Nacht aufgewärmt. Nach Zugabe von halbgesättigter Ammoniumchlorid-Lösung (circa 2,5 ml pro mmol Ester) wird mehrfach mit Ether, Ethylacetat bzw. Dichlormethan extrahiert. Die so erhaltenen Extrakte werden vereinigt, über Natriumsulfat getrocknet, filtriert und im Vakuum bei einem Druck von 500 bis 20 mbar vollständig eingeengt. Das entstehende Ketoamid (IX) wird mit 10 mol-% TiCl₃ und 4 Äquivalenten Zinkstaub mit Acetonitril suspendiert. Es werden 4 Äquivalente TMSCl zugegeben und 30 min zum Rückfluß erhitzt. Es wird auf Raumtemperatur abgekühlt, mit Essigsäureethylester verdünnt und über Kieselgel abfiltriert. Das Filtrat wird im Vakuum bei einem Druck von 500 bis 20 mbar vollständig eingeengt und über Kieselgel mit n-Hexan/Essigsäureethylester 9:1 aufgereinigt.

Das entstehende Indolderivat (IV) wird zum Zweck der Bildung der Verbindung (V) (mit R¹ ≠ H) mit 1,05 Äquivalenten NaH und 1,1 Äquivalenten Alkylchlorid, -bromid oder -iodid in THF umgesetzt.

Die COCF₃-Schutzgruppe wird mit Hilfe von wäßriger K₂CO₃- oder NaOH-Lösung abgespalten.

Substanzen der allgemeinen Formel (II) (mit R² = H) werden mit 1,05 Äquivalenten Carbonsäure-, Sulfonsäure- bzw. Alkyl- oder Cycloalkylchlorid in Gegenwart von 1,1 Äquivalenten Triethylamin und katalytischen Mengen DMAP in Dichlormethan in die korrespondierenden Verbindungen (I) (mit R² ≠ H) überführt.

Folgende Verbindungen (Tabelle 1) wurden nach der allgemeinen Arbeitsvorschrift beispielhaft hergestellt und mittels NMR und/oder MS identifiziert:

**Tabelle 1**

| **Beispiel- Nr.** | **Verbindung** |
|---|---|
| 1 | (2-Brom-phenyl)-[2-(3-phenyl-1H-indol-2-yl)-pyrrolidin-1-yl]-methanon |
| 2 | 1-But-2-inyl-5-chlor-3-phenyl-2-[1-(thiophen-2-sulfonyl)-pyrrolidin-2-yl]-1H-indol |
| 3 | 2-Phenyl-1-[2-(3-phenyl-1H-indol-2-yl)-pyrrolidin-1-yl]-butan-1-on |
| 4 | 2-(2,5-Dimethoxy-phenyl)-1-[2-(3-phenyl-1H-indol-2-yl)-pyrrolidin-1-yl]-ethanon |
| 5 | (4-Brom-3-methyl-phenyl)-[2-(5-chlor-3-phenyl-1H-indol-2-yl)-pyrrolidin-1-yl]-methanon |
| 6 | (3-Methoxy-phenyl)-[2-(3-phenyl-1H-indol-2-yl)-pyrrolidin-1-yl)-methanon |
| 7 | (4-Nitro-phenyl)-[2-(3-phenyl-1H-indol-2-yl)-pyrrolidin-1-yl]-methanon |
| 8 | (4-Chlor-phenyl)-[2-(3-phenyl-1H-indol-2-yl)-pyrrolidin-1-yl]-methanon |
| 9 | {2-[5-Chlor-1-(2-diethylamino-ethyl)-3-phenyl-1H-indol-2-yl]-pyrrolidin-1-yl}-furan-2-yl-methanon |
| 10 | [2-(5-Chlor-3-phenyl-1H-indol-2-yl)-pyrrolidin-1-yl]-(2,3-difluor-4-methyl-phenyl)-methanon |
| 11 | (2,6-Difluor-3-methyl-phenyl)-[2-(3-phenyl-1H-indol-2-yl)-pyrrolidin-1-yl]-methanon |
| 12 | 4-{2-[5-Chlor-1-(2-diethylamino-ethyl)-3-phenyl-1H-indol-2-yl]-pyrrolidin-1-yl)-4-oxo-buttersäureethylester |
| 13 | {2-[5-Chlor-1-(2-diethylamino-ethyl)-3-phenyl-1H-indol-2-yl]-pyrrolidin-1-yl}-(4-fluor-phenyl)-methanon |
| 14 | 2-Phenoxy-1-[2-(3-phenyl-1H-indol-2-yl)-pyrrolidin-1-yl]-propan-1-on |
| 15 | {2-[5-Chlor-1-(2-diethylamino-ethyl)-3-phenyl-1H-indol-2-yl]-pyrrolidin-1-yl}-cyclobutyl-methanon |
| 16 | [2-(1-But-2-inyl-3-phenyl-1H-indol-2-yl)-pyrrolidin-1-yl]-(2-chlor-5-trifluormethyl-phenyl)-methanon |
| 17 | {2-[5-Chlor-1-(2-diethylamino-ethyl)-3-phenyl-1H-indol-2-yl]-pyrrotidin-1-yl}-(6-chlor-pyridin-3-yl)-methanon |
| 18 | Pentafluorphenyl-[2-(3-phenyl-1H-indol-2-yl)-pyrrolidin-1-yl]-methanon |
| 19 | 1-[2-(3-Phenyl-1H-indol-2-yl)-pyrrolidin-1-yl]-butan-1-on |
| 20 | 1-{2-[5-Chlor-1-(2-diethylamino-ethyl)-3-phenyl-1H-indol-2-yl]-pyrrolidin-1-yl}-hexan-1-on |
| 21 | 5-Chlor-1-methyl-3-phenyl-2-[1-(thiophen-2-sulfonyl)-pyrrolidin-2-yl]-1H-indol |
| 22 | (2-Chlor-4-nitro-phenyl)-[2-(5-chlor-3-phenyl-1H-indol-2-yl)-pyrrolidin-1-yl]-methanon |
| 23 | (4-Brom-3-methyl-phenyl)-[2-(3-phenyl-1H-indol-2-yl)-pyrrolidin-1-yl]-methanon |
| 24 | 1-[2-(5-Chlor-3-phenyl-1H-indol-2-yl)-pyrrolidin-1-yl]-pent-4-en-1-on |
| 25 | 2-[1-(3-Brom-benzylsulfonyl)-pyrrolidin-2-yl]-3-phenyl-1H-indol |
| 26 | {2-[5-Chlor-1-(2-diethylamino-ethyl)-3-phenyl-1H-indol-2-yl]-pyrrolidin-1-yl}-(3,5-dimethoxy-phenyl)-methanon |
| 27 | 4-Oxo-4-[2-(3-phenyl-1H-indol-2-yl)-pyrrolidin-1-yl]-buttersäuremethylester |
| 28 | {2-[5-Chlor-1-(2-diethylamino-ethyl)-3-phenyl-1H-indol-2-yl]-pyrrolidin-1-yl}-(4-ethoxy-phenyl)-methanon |
| 29 | 5-Chlor-3-phenyl-2-[1-(2,3,5,6-tetramethyl-benzylsulfonyl)-pyrrolidin-2-yl]-1H-indol |
| 30 | (2,5-Bis-trifluormethyl-phenyl)-[2-(3-phenyl-1H-indol-2-yl)-pyrrolidin-1-yl]-methanon |
| 31 | [2-(1-But-2-inyl-5-chlor-3-phenyl-1H-indol-2-yl)-pyrrolidin-1-yl]-(3-methoxy-phenyl)-methanon |
| 32 | [2-(1-But-2-inyl-3-phenyl-1H-indol-2-yl)-pyrrolidin-1-yl]-(2-methylsulfanyl-pyridin-3-yl)-methanon |
| 33 | 1-[2-(5-Chlor-3-phenyl-1H-indol-2-yl)-pyrrolidin-1-yl]-2-phenoxy-ethanon |
| 34 | [3-(2-Chlor-6-fluor-phenyl)-5-methyl-isoxazol-4-yl]-[2-(5-chlor-3-phenyl-1H-indol-2-yl)-pyrrolidin-1-yl]-methanon |
| 35 | 1-[2-(5-Chlor-1-methyl-3-phenyl-1H-indol-2-yl)-pyrrolidin-1-yl]-2-(2-methoxy-ethoxy)-ethanon |
| 36 | 5-Chlor-3-phenyl-2-[1-(thiophen-2-sulfonyl)-pyrrolidin-2-yl]-1H-indol |
| 37 | [2-(3-Phenyl-1H-indol-2-yl)-pyrrolidin-1-yl]-(3-trifluormethoxy-phenyl)-methanon |
| 38 | 5-Chlor-2-[1-(3-chlor-2-methyl-benzylsulfonyl}-pyrrolidin-2-yl]-3-phenyl-1H-indol |
| 39 | 1-[2-(1-But-2-inyl-5-chlor-3-phenyl-1H-indol-2-yl)-pyrrolidin-1-yl]-pentan-1-on |
| 40 | 5-{2-Oxo-2-[2-(3-phenyl-1H-indol-2-yl)-pyrrolidin-1-yl]-ethyl}-imidazolidin-2,4-dion |
| 41 | [2-(5-Chlor-1-methyl-3-phenyl-1H-indol-2-yl)-pyrrolidin-1-yl]-(3-trifluormethoxy-phenyl)-methanon |
| 42 | (2-tert-Butyl-5-methyl-2H-pyrazol-3-yl)-[2-(5-chlor-3-phenyl-1H-indol-2-yl)-pyrrolidin-1-yl]-methanon |
| 43 | (2,3-Dimethyl-phenyl)-[2-(3-phenyl-1H-indol-2-yl)-pyrrolidin-1-yl]-methanon |
| 44 | [2-(5-Chlor-3-phenyl-1H-indol-2-yl)-pyrrolidin-1-yl]-(3-trifluormethyl-phenyl)-methanon |
| 45 | [2-(5-Chlor-3-phenyl-1H-indol-2-yl)-pyrrolidin-1-yl]-(4-trifluormethylsulfanyl-phenyl)-methanon |
| 46 | (5-tert-Butyl-2-methyl-furan-3-yl)-[2-(5-chlor-3-phenyl-1H-indol-2-yl)-pyrrolidin-1-yl]-methanon |
| 47 | [2-(5-Chlor-1-methyl-3-phenyl-1H-indol-2-yl)-pyrrolidin-1-yl]-(2-chlor-pyridin-3-yl)-methanon |
| 48 | [2-(5-Chlor-1-methyl-3-phenyl-1H-indol-2-yl)-pyrrolidin-1-yl]-cyclopropyl-methanon |
| 49 | 3-Phenyl-2-[1-(4-trifluormethoxy-benzylsulfonyl)-pyrrolidin-2-yl]-1H-indol |
| 50 | 2-Methyl-1-[2-(3-phenyl-1H-indol-2-yl)-pyrrolidin-1-yl]-pentan-1-on |
| 51 | 1-But-2-inyl-2-[1-(4-chlor-benzylsulfonyl)-pyrrolidin-2-yl]-3-phenyl-1 H-indol |
| 52 | 1-[2-(5-Chlor-1-methyl-3-phenyl-1H-indol-2-yl)-pyrrolidin-1-yl]-4-phenoxy-butan-1-on |
| 53 | [2-(5-Chlor-3-phenyl-1H-indol-2-yl)-pyrrolidin-1-yl]-(2,3-dimethyl-phenyl)-methanon |
| 54 | [2-(5-Chlor-3-phenyl-1H-indol-2-yl)-pyrrolidin-1-yl]-(3,5-dimethoxy-phenyl)-methanon |
| 55 | [2-(3-Phenyl-1H-indol-2-yl)-pyrrolidin-1-yl]-(4-trifluormethylsulfanyl-phenyl)-methanon |
| 56 | 1-But-2-inyl-5-chlor-2-[1-(2-chlor-benzylsulfonyl)-pyrrolidin-2-yl]-3-phenyl-1H-indol |
| 57 | [2-(1-But-2-inyl-5-chlor-3-phenyl-1H-indol-2-yl)-pyrrolidin-1-yl]-(2-chlor-4-nitro-phenyl)-methanon |
| 58 | [2-(3-Phenyl-1H-indol-2-yl)-pyrrolidin-1-yl]-m-tolyl-methanon |
| 59 | (3,5-Bis-trifluormethyl-phenyl)-[2-(1-but-2-inyl-3-phenyl-1H-indol-2-yl)-pyrrolidin-1-yl]-methanon |
| 60 | 1-But-2-inyl-5-chlor-2-[1-(4-methoxy-benzylsulfonyl)-pyrrolidin-2-yl]-3-phenyl-1H-indol |
| 61 | [2-(1-But-2-inyl-3-phenyl-1H-indol-2-yl)-pyrrolidin-1-yl]-(2-chlor-4-nitro-phenyl)-methanon |
| 62 | [2-(5-Chlor-1-methyl-3-phenyl-1H-indol-2-yl)-pyrrolidin-1-yl]-(2,3-dimethyl-phenyl)-methanon |
| 63 | 1-But-2-inyl-5-chtor-2-[1-(2,5-dimethoxy-benzylsulfonyl)-pyrrolidin-2-yl]-3-phenyl-1 H-indol |
| 64 | 1-[2-(5-Chlor-1-methyl-3-phenyl-1H-indol-2-yl)-pyrrolidin-1-yl]-2-methyl-pentan-1-on |
| 65 | 2-(4-Chlor-phenoxy)-1-[2-(5-chtor-3-pheny)-1H-indol-2-yl)-pyrrolidin-1-yl]-ethanon |
| 66 | (2-Methyl-6-trifluormethyl-pyridin-3-yl)-[2-(3-phenyl-1H-indol-2-yl)-pyrrolidin-1-yl]-methanon |
| 67 | 1-[2-(5-Chlor-1-methyl-3-phenyl-1H-indol-2-yl)-pyrrolidin-1-yl]-2-cyclopentyl-ethanon |
| 68 | 2-[1-(4-Butoxy-benzylsulfonylrpyrrolidin-2-yl]-1-but-2-inyl-3-phenyl-1H-indol |
| 69 | (4-Brom-3-methyl-phenyl)-[2-(5-chlor-1-methyl-3-phenyl-1H-indol-2-yl)-pyrrolidin-1-yl]-methanon |
| 70 | [2-(1-But-2-inyl-5-chlor-3-phenyl-1H-indol-2-yl)-pyrrolidin-1-yl]-(4-ethyl-phenyl)-methanon |
| 71 | [2-(5-Chlor-1-methyl-3-phenyl-1H-indol-2-yl)-pyrrolidin-1-yl]-(2,3-difluor-phenyl)-methanon |
| 72 | 1-[2-(1-But-2-inyl-3-phenyl-1 H-indol-2-yl)-pyrrolidin-1-yl]-3-(3-trifluormethyl-phenyl)-propenon |
| 73 | 1-[2-(1-But-2-inyl-5-chlor-3-phenyl-1H-indol-2-yl)-pyrrolidin-1-yl]-2-methoxy-ethanon |
| 74 | 1-[2-(1-But-2-inyl-5-chlor-3-phenyl-1H-indol-2-yl)-pyrrolid in-1-yl]-3,3-dimethyl-butan-1-on |
| 75 | [2-(1-But-2-inyl-5-chlor-3-phenyl-1H-indol-2-yl)-pyrrolidin-1-yl]-p-tolyl-methanon |
| 76 | [2-(3-Phenyl-1H-indol-2-yl)-pyrrolidin-1-yl]-(3-trifluormethyl-phenyl)-methanon |
| 77 | 3-Phenyl-2-[1-(thiophen-2-sulfonyl)-pyrrolidin-2-yl]-1H-indol |
| 78 | [2-(5-Chlor-1-methyl-3-phenyl-1H-indol-2-yl)-pyrrolidin-1-yl]-[3-(2-chlor-phenyl)-5-methyl-isoxazol-4-yl]-methanon |
| 79 | 1-[2-(1-But-2-inyl-5-chlor-3-phenyl-1 H-indol-2-yl)-pyrrolidin-1-carbonyl]-4,7,7-trimethyl-2-oxa-bicyclo[2.2.1]heptan-3-on |
| 80 | 1-[2-(5-Chlor-1-methyl-3-phenyl-1 H-indol-2-yl)-pyrrolidin-1-yl]-2-phenoxy-ethanon |
| 81 | (3-Fluor-4-trifluormethyl-phenyl)-[2-(3-phenyl-1H-indol-2-yl)-pyrrolidin-1-yl]-methanon |
| 82 | 1-But-2-inyl-2-[1-(2-chlor-benzylsulfonyl)-pyrrolidin-2-yl]-3-phenyl-1H-indol |
| 83 | [2-(1-But-2-inyl-3-phenyl-1H-indol-2-yl)-pyrrolidin-1-yl]-(4-trifluormethoxy-phenyl)-methanon |
| 84 | [2-(1-But-2-inyl-3-phenyl-1H-indol-2-yl)-pyrrolidin-1-yl]-(4-propyl-phenyl)-methanon |
| 85 | [2-(1-But-2-inyl-5-chlor-3-phenyl-1H-indol-2-yl)-pyrrolidin-1-yl]-(3-fluor-4-methyl-phenyl)-methanon |
| 86 | 1-[2-(1-But-2-inyl-5-chlor-3-phenyl-1H-indol-2-yl)-pyrrol idin-1-yl]-2-propyl-pentan-1-on |
| 87 | 1-[2-(1 -But-2-inyl-3-phenyl-1H-indol-2-yl)-pyrrolidin-1-yl]-2-phenoxy-propan-1-on |
| 88 | (3-Brom-phenyl)-[2-(1-but-2-inyl-5-chlor-3-phenyl-1H-indol-2-yl)-pyrrolidin-1 -yl]-methanon |
| 89 | 1-[2-(1-But-2-inyl-5-chlor-3-phenyl-1H-indol-2-yl)-pyrrolidin-1-yl]-2-(2,5-dimethoxy-phenyl)-ethanon |
| 90 | 1-[2-(1-But-2-inyl-5-chlor-3-phenyl-1H-indol-2-yl)-pyrrolidin-1-yl]-2-dimethylamino-ethanon |
| 91 | Naphthalen-1-yl-[2-(3-phenyl-1H-indol-2-yl)-pyrrolidin-1-yl]-methanon |
| 92 | [2-(5-Chlor-1-methyl-3-phenyl-1H-indol-2-yl)-pyrrolidin-1-yl]-(4-methyl-3-nitro-phenyl)-methanon |
| 93 | 1-[2-(1-But-2-inyl-5-chlor-3-phenyl-1H-indol-2-yl)-pyrrolidin-1-yl]-2-(4-chlor-phenoxy)-ethanon |
| 94 | 1-[2-(1-But-2-inyl-3-phenyl-1H-indol-2-yl)-pyrrolidin-1-yl]-butan-1-on |
| 95 | 1-[2-(1-But-2-inyl-3-phenyl-1H-indol-2-yl)-pyrrolidin-1-yl]-3-(2-chlor-phenyl)-propenon |
| 96 | (2,5-Bis-trifluormethyl-phenyl)-[2-(1-but-2-inyl-5-chlor-3-phenyl-1H-indol-2-yl)-pyrrolidin-1-yl]-methanon |
| 97 | 2-Ethyl-1-[2-(3-phenyl-1H-indol-2-yl)-pyrrolidin-1-yl]-hexan-1-on |
| 98 | [2-(1-But-2-inyl-3-phenyl-1H-indol-2-yl)-pyrrolidin-1-yl]-(3-trifluormethyl-phenyl)-methanon |
| 99 | 1-[2-(1-But-2-inyl-5-chlor-3-phenyl-1H-indol-2-yl)-pyrrolidin-1-yl]-2-(4-chlor-phenyl)-ethanon |
| 100 | [2-(1-But-2-inyl-3-phenyl-1H-indol-2-yl)-pyrrolidin-1-yl]-(5-fluor-2-trifluormethyl-phenyl)-methanon |
| 101 | 1-[2-(1-But-2-inyl-3-phenyl-1H-indol-2-yl)-pyrrolidin-1-yl]-2-phenyl-ethanon |
| 102 | 1-But-2-inyl-3-phenyl-2-[1-(4-propyl-benzylsulfonyl)-pyrrolidin-2-yl]-1H-indol |
| 103 | 1-But-2-inyl-2-[1-(2,5-dimethoxy-benzylsulfonyl)-pyrrolidin-2-yl]-3-phenyl-1H-indol |
| 104 | 1-[2-(1-But-2-inyl-3-phenyl-1H-indol-2-yl)-pyrrolidin-1-yl]-3,3-dimethyl-butan-1-on |
| 105 | [2-(1-But-2-inyl-3-phenyl-1H-indol-2-yl)-pyrrolidin-1-yl]-p-tolyl-methanon |
| 106 | 1-But-2-inyl-2-[1-(5-chlor-thiophen-2-sulfonyl)-pyrrolidin-2-yl]-3-phenyl-1H-indol |
| 107 | 1-[2-(1-But-2-inyl-3-phenyl-1H-indol-2-yl)-pyrrolidin-1-yl]-2-phenoxy-ethanon |
| 108 | [2-(1-But-2-inyl-3-phenyl-1H-indol-2-yl)-pyrrolidin-1-yl]-(4-methyl-3-nitro-phenyl)-methanon |
| 109 | [2-(1-But-2-inyl-5-chlor-3-phenyl-1H-indol-2-yl)-pyrrolidin-1-yl]-(2,5-dimethyl-furan-3-yl)-methanon |
| 110 | Adamantan-1-yl-[2-(3-phenyl-1 H-indol-2-yl)-pyrrolidin-1-yl]-methanon |
| 111 | [2-(1-But-2-inyl-3-phenyl-1H-indol-2-yl)-pyrrolidin-1-yl]-(3,5-difluor-phenyl)-methanon |
| 112 | [2-(1-But-2-inyl-3-phenyl-1H-indol-2-yl)-pyrrolidin-1-yl]-cyclohexyl-methanon |
| 113 | 1-[2-(1-But-2-inyl-3-phenyl-1H-indol-2-yl)-pyrrolidin-1-yl]-2-(3-methoxy-phenyl)-ethanon |
| 114 | (3-Fluor-4-methyl-phenyl)-[2-(3-phenyl-1-prop-2-inyl-1H-indol-2-yl)-pyrrolidin-1-yl]-methanon |
| 115 | 2-[1-(2-Chlor-benzylsulfonyl)-pyrrolidin-2-yl]-3-phenyl-1H-indol |
| 116 | 1-{7-[2-(1-But-2-inyl-3-phenyl-1H-indol-2-yl)-pyrrolidin-1-sulfonyl]-3,4-dihydro-1H-isoquinolin-2-yl}-2,2,2-trifluor-ethanon |
| 117 | 2-[1-(3-Chlor-4-fluor-benzylsulfonyl)-pyrrolidin-2-yl]-3-phenyl-1H-indol |
| 118 | 1-[2-(1-But-2-inyl-3-phenyl-1H-indol-2-yl)-pyrrolidin-1-yl]-2-phenyl-butan-1-on |
| 119 | [2-(1-But-2-inyl-3-phenyl-1H-indol-2-yl)-pyrrolidin-1-yl]-(1-phenyl-5-propyl-1H-pyrazol-4-yl)-methanon |
| 120 | 1-[2-(1-But-2-inyl-3-phenyl-1H-indol-2-yl)-pyrrolidin-1-yl]-2-(4-chlor-phenyl)-ethanon |
| 121 | [2-(1-But-2-inyl-5-chlor-3-phenyl-1H-indol-2-yl)-pyrrolidin-1-yl]-(6-chlor-pyridin-3-yl)-methanon |
| 122 | [2-(1-But-2-inyl-5-chlor-3-phenyl-1H-indol-2-yl)-pyrrolidin-1-yl]-(2-ethoxy-phenyl)-methanon |
| 123 | [2-(1-But-2-inyl-3-phenyl-1H-indol-2-yl)-pyrrolidin-1-yl]-pyridin-2-yl-methanon |
| 124 | [2-(1-But-2-inyl-5-chlor-3-phenyl-1H-indol-2-yl)-pyrrolidin-1-yl]-isoxazol-5-yl-methanon |

### Biochemische Testung

### NOS-Assay

### Allgemeines

Dieser Assay erlaubt die Bestimmung der prozentualen Hemmung von NO-Synthase durch einen Wirkstoff mittels Messung der NOS-Aktivität bei Einwirken des Wirkstoffs. Dabei wird NO-Synthase zusammen mit radioaktiv markiertem Arginin und dem Wirkstoff unter geeigneten Bedingungen gemischt. Nach Abbruch der NO-Bildungsreaktion zu einem vorgegebenen Zeitpunkt wird die Menge an nicht umgesetztem Arginin direkt oder indirekt bestimmt. Der Vergleich dieser Menge mit der in einem ohne Zusatz von Wirkstoff und unter sonst gleichen Bedingungen aus der Mischung von NOS und Arginin zurückbleibenden Menge an Arginin ergibt die %-Hemmung von NO-Synthase durch den getesteten Wirkstoff. Dieser Assay läßt sich wie folgt durchführen:
(a) Inkubation der NO-Synthase mit markiertem Arginin als Substrat in einem Reaktionsgefäß,
(b) Trennung des markierten Arginins von dem gegebenenfalls als Produkt der enzymatischen Reaktion entstandenen, markierten Citrullin zu einem Zeitpunkt, zu dem die Konzentration an Citrullin ansteigt,
(c) Messung der Menge an jeweils abgetrenntem Arginin.
Die Trennung erfolgt über eine Filterplatten-Membran.

Dieser NOS-Assay eignet sich insbesondere für ein "High Throughput Screening" (HTS) auf Mikrotiterplatten (MTP).

### HTS-NOS-Assay: Allgemeine Verfahrensweise

In diesem HTS-NOS-Assay wird radioaktives Arginin als Substrat benutzt. Das Assayvolumen kann je nach Art der Mikrotiterplatte (MTP) im Bereich zwischen 25 µl und 250 µl gewählt werden. In Abhängigkeit von der benutzten Enzymquelle werden Cofaktoren und Coenzyme zugefügt. Die Inkubation der Ansätze in dieser Mikrotiterplatte (Assay-MTP) gemäß Schritt (a) wird bei Raumtemperatur vorgenommen und beträgt je nach verwendeter Enzymaktivität (units) zwischen 5 und 60 Minuten. Zum Ende der Inkubation (Schritt (a)) wird die Platte in einen Zellharvester plaziert, der mit einer MTP bestückt ist, die eine Kationenaustauschermembran als Filterboden besitzt (Filter-MTP). Alle Ansätze der Assay-MTP werden in diese Filter-MTP überführt und über eine Kationenaustauscher-Filter-Platte, einen mit Phosphatgruppen beladenen Papierfilter, abgesaugt. Die Filter-MTP wird anschließend mit Puffer oder Wasser gewaschen. Mit Hilfe dieser Vorgehensweise wird das verbliebene Substrat Arginin auf dem Kationenaustauscher gebunden, während das enzymatisch gebildete radioaktive Citrullin quantitativ ausgewaschen wird. Nach Trocknen der Filter-MTP und Zugabe von Szintillationsflüssigkeit kann das gebundene Arginin am Szintillationszähler ausgezählt werden. Eine nicht gehemmte NOS-Reaktion spiegelt sich in einer geringen Radioaktivität wieder. Eine gehemmte Enzymreaktion bedeutet, daß das radioaktive Arginin nicht umgesetzt worden ist, d.h. auf dem Filter befindet sich eine hohe Radioaktivität.

### Verwendete Materialien

- Arginin, L-[2, 3, 4-³H]-monohydrochlorid; Best.-Nr. NET-1123, Fa. NEN
- CaCl₂ wasserfrei; Best.- Nr. 2388.1000; Fa. Merck KGaA
- 1.4-Dithiothreitol (DTT), Best.-Nr. 708984; Fa. ROCHE
- Na₂EDTA-Dihydrat; Best.-Nr. 03680; Fa. FLUKA
- HEPES, Best:-Nr. H-3375; Fa. SIGMA
- NADPH, Tetranatriumsalz; Best.-Nr. 1585363; Fa. ROCHE
- TRIS; BEST.-Nr. 93349; Fa. FLUKA

| | |
|---|---|
| Enzym-Präparationspuffer: | 50 mM Tris-HCl mit 1 mM EDTA: Der pH-Wert des Puffers wurde bei 4 °C auf 7,4 eingestellt. |
| Inkubationspuffer (-medium): CaCl₂ und 1 mM Dithiothreitol. | 50 mM HEPES mit 1 mM EDTA; 1,25 mM |
| Der pH-Wert des Puffers wurde bei 25 °C auf 7,4 eingestellt. Waschmedium: | H₂O |

### Enzympräparation

Als Ausgangsgewebe wurden Ratten-Cerebelli benutzt. Die Tiere wurden betäubt und getötet, das Gehirngewebe, das Cerebellum, wurde herauspräpariert, pro Rattenkleinhirn wurde 1 ml Enzympräparationspuffer (4 °C) hinzugegeben, und es wurde mit einem Polytron-Homogenisierer für 1 min bei 6000 U/min aufgeschlossen. Danach erfolgte Zentrifugation bei 4 °C für 15 min bei 20 000 g und anschließend Abdekantieren des Überstand und portioniertes Einfrieren bei - 80 °C (Verwerfen des Niederschlags).

### Testerqebnisse

Erfindungsgemäße Verbindungen wurden im oben beschriebenen NOS-Assay auf nNOS-Inhibition getestet. Die Ergebnisse sind in Tabelle 2 wiedergegeben.

**Tabelle 2**

| **Beispiel-** | **Verbindung** | **nNOS-** |
|---|---|---|
| **Nr.** | | **Inhibition (10 µm) [%]** |
| 9 | {2-[5-Chlor-1-(2-diethylamino-ethyl)-3-phenyl-1H-indol-2-yl]-pyrrolidin-1-yl}-furan-2-yl-methanon | 45 |
| 12 | 4-{2-[5-Chlor-1-(2-diethylamino-ethyl)-3-phenyl-1H-indol-2-yl]-pyrrolidin-1-yl}-4-oxo-buttersäureethylester | 34 |
| 13 | {2-[5-Chlor-1-(2-diethylamino-ethyl)-3-phenyl-1H-indol-2-yl]-pyrrolidin-1-yl}-(4-fluor-phenyl)-methanon | 47 |
| 15 | {2-[5-Chlor-1-(2-diethylamino-ethylr3-phenyl-1H-indol-2-yl]-pyrrolidin-1-yl}-cyclobutyl-methanon | 38 |
| 17 | {2-[5-Chlor-1-(2-diethylamino-ethyl)-3-phenyl-1H-indol-2-yl]-pyrrolidin-1-yl}-(6-chlor-pyridin-3-yl)-methanon | 36 |
| 20 | 1-{2-[5-Chlor-1-(2-diethylamino-ethyl)-3-phenyl-1H-indol-2-yl]-pyrrolidin-1-yl}-hexan-1-on | 35 |
| 26 | {2-[5-Chlor-1-(2-diethylamino-ethyl)-3-phenyl-1H-indol-2-yl]-pyrrolidin-1-yl}-(3,5-dimethoxy-phenyl)-methanon | 51 |
| 28 | {2-[5-Chlor-1-(2-diethylamino-ethyl)-3-phenyl-1H-indol-2-yl]-pyrrolidin-1-yl}-(4-ethoxy-phenyl)-methanon | 41 |
| 35 | 1-[2-(5-Chlor-1-methyl-3-phenyl-1H-indol-2-yl)-pyrrolidin-1-yl]-2-(2-methoxy-ethoxyrethanon | 41 |
| 48 | [2-(5-Chlor-1-methyl-3-phenyl-1H-indol-2-yl)-pyrrolidin-1-yl]-cyclopropyl-methanon | 35 |
| 73 | 1-[2-(1-But-2-inyl-5-chlor-3-phenyl-1H-indol-2-yl)-pyrrolidin-1-yl]-2-methoxy-ethanon | 54 |

Als Vergleichsbeispiel wurde der bekannte NOS-Inhibitor 7-Nitroindazol in diesem NOS-Assay mit einer Hemmung (10 µM) von 50% getestet.

### Pharmazeutische Formulierung eines erfindungsgemäßen Arzneimittels

1 g 1-[2-(1-But-2-inyl-5-chlor-3-phenyl-1H-indol-2-yl)-pyrrolidin-1-yl]-2-methoxy-ethanon wird in 1 l Wasser für Injektionszwecke bei Raumtemperatur gelöst und anschließend durch Zugabe von Natriumchlorid auf isotone Bedingungen eingestellt.

## Patentansprüche

1. Substituierte 2-Pyrrolidin-2-yl-1 H-indol-Derivate der allgemeinen Formel (I) 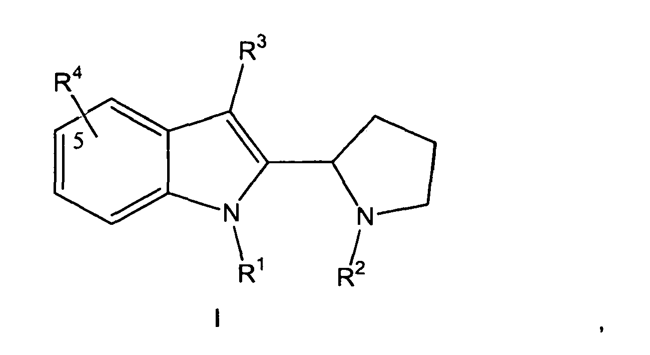 in Form ihrer Racemate, ihrer reinen Stereoisomeren, oder in Form von Mischungen der Stereoisomeren, in einem beliebigen Mischungsverhältnis; in dargestellter Form oder in Form ihrer Säuren oder ihrer Basen oder in Form ihrer Salze, oder in Form ihrer Solvate,
wobei
R¹ H, Alkyl, Cycloalkyl, Alkyl-Cycloalkyl, Alkyl-Aryl oder Alkyl-Heterocyclyl bedeutet;
R² H, Alkyl, Cycloalkyl, Alkyl-Cycloalkyl, Alkyl-Aryl, Alkyl-Heterocyclyl, C(=O)-R²⁰ oder SO₂-R²¹ bedeutet;
R³ Alkyl, Aryl oder Heterocyclyl bedeutet;
R⁴ H, F, Cl, Br, l, -CN, OR⁴⁰, Alkyl, Cycloalkyl oder NO₂ bedeutet;
R²⁰ Alkyl, Cycloalkyl, Alkyl-Cycloalkyl, Aryl, Alkyl-Aryl, Heterocyclyl, Alkyl-Heterocyclyl, OR²⁰⁰ oder NHR²⁰¹ bedeutet;
R²¹ Alkyl, Aryl, Alkyl-Aryl, Heterocyclyl oder Alkyl-Heterocyclyl bedeutet;
R⁴⁰ H, Alkyl, Cycloalkyl, Alkyl-Cycloalkyl, Aryl oder Alkyl-Aryl bedeutet;
R²⁰⁰ Alkyl, Cycloalkyl, Alkyl-Cycloalkyl, Aryl, Alkyl-Aryl, Heterocyclyl oder Alkyl-Heterocyclyl bedeutet; und
R²⁰¹ Alkyl, Aryl, Alkyl-Aryl, Heterocyclyl oder Alkyl-Heterocyclyl bedeutet;
wobei
"Cycloalkyl" einen alicyclischen gesättigten oder ungesättigten Kohlenwasserstoff-Rest bedeutet, wobei der Rest unsubstituiert oder einfach substituiert oder mehrfach gleich oder verschieden substituiert und ggf. benzokondensiert sein kann und auch bi-, tri- oder polycyclische Alicyclen umfaßt,
"Alkyl" acyclische gesättigte oder ungesättigte Kohlenwasserstoffreste ("Alkanyle", "Alkenyle" und "Alkinyle") umfasst, die verzweigt oder geradkettig sowie unsubstituiert oder einfach substituiert oder mehrfach gleich oder verschieden substituiert sein können;
und "Heterocyclyl" für einen monocyclischen oder polycyclischen organischen Rest steht, in dem mindestens ein Cyclus 1 Heteroatom oder 2, 3, 4 oder 5 gleiche oder verschiedene Heteroatome enthält, das/die aus der Gruppe, die N, O und S enthält, ausgewählt ist/sind, wobei der Rest gesättigt oder ungesättigt ist und unsubstituiert oder einfach substituiert oder mehrfach gleich oder verschieden substituiert ist,
unter der Maßgabe, daß
2-(3-Phenyl-1H-indol-2-yl)-1-(trifluoracetyl)-pyrrolidin;
α-Methyl-2-(1-methyl-2-pyrrolidinyl)-indol-3-essigsäuremethylester;
3-(1-Cyanoethyl)-2-(1-methyl-2-pyrrolidinyl)-indol; und
2-(1-Methyl-2-pyrrolidinyl)-3-vinylindol
ausgenommen sind.

2. Substituierte 2-Pyrrolidin-2-yl-1H-indol-Derivate nach Anspruch 1, **dadurch gekennzeichnet, daß**
R¹ H, Alkyl, Cycloalkyl oder Alkyl-Cycloalkyl bedeutet;
R² C(=O)-R²⁰ oder SO₂-R²¹ bedeutet;
R³ Aryl bedeutet;
R⁴ H, F, Cl, Br, l, -CN, OR⁴⁰, Alkyl, Cycloalkyl oder NO₂, jeweils in 5-Position des Indolrings, bedeutet;
R²⁰ Alkyl, Cycloalkyl, Alkyl-Cycloalkyl, Aryl, Alkyl-Aryl, Heterocyclyl oder Alkyl-Heterocyclyl bedeutet;
R²¹ Alkyl, Aryl, Alkyl-Aryl, Heterocyclyl oder Alkyl-Heterocyclyl bedeutet; und
R⁴⁰ H, Alkyl, Cycloalkyl, Alkyl-Cycloalkyl, Aryl oder Alkyl-Aryl bedeutet.

3. Substituierte 2-Pyrrolidin-2-yl-1H-indol-Derivate nach Anspruch 1,
**dadurch gekennzeichnet, daß**
R¹ H oder Alkyl bedeutet;
R² C(=O)-R²⁰ oder SO₂-R²¹ bedeutet;
R³ Phenyl bedeutet;
R⁴ H, F, Cl, Br oder I, jeweils in 5-Position des Indolrings, bedeutet;
R²⁰ C₁₋₈-Alkanyl, C₂₋₈-Alkenyl, C₁₋₄-Alkanyl-CO₂-C₁₋₄-Alkyl, C₁₋₄-Alkanyl-O-R²⁰², C₁₋₄-Alkanyl-NR²⁰³R²⁰⁴, C₃₋₈-Cycloalkyl, 1-Adamantyl, 2-Adamantyl, -(C₁₋₃-Alkyl)-C₃₋₈-Cycloalkyl, Aryl¹, Aryl², C₁₋₄-Alkanyl-Aryl³, C₂₋₄-Alkenyl-Aryl⁴; oder unsubstituiertes oder substituiertes Furanyl, Pyrazolyl, Isoxazolyl oder Pyridinyl; oder 4,7,7-Trimethyloxabicyclo[2.2.1]heptan-3-on, -CH₂-lmidazolidin-2,4-dion bedeutet;
R²¹ Aryl⁵, unsubstituiertes oder substituiertes Thienyl bedeutet oder für 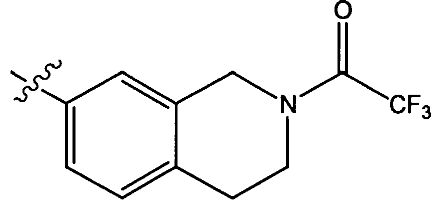 steht;
R²⁰² H, C₁₋₄-Alkanyl, C₁₋₄-Alkanyl-OH, C₁₋₄-Alkanyl-O-C₁₋₄-Alkanyl oder Aryl bedeutet;
R²⁰³ und R²⁰⁴ unabhängig voneinander C₁₋₆-Alkyl bedeuten oder mit dem N-Atom ein 5-, 6- oder 7-gliedriges gesättigtes Heterocyclyl bilden;
Aryl¹ für 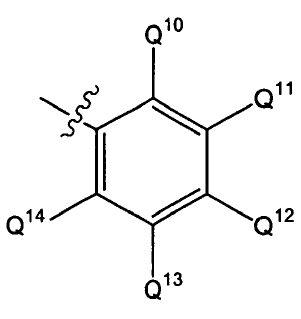 steht;
Aryl² für 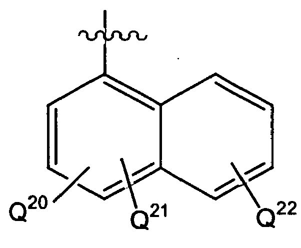 steht;
Aryl³ für 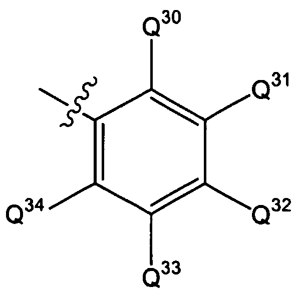 steht;
Aryl⁴ für 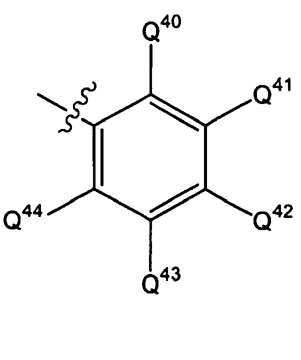 steht;
Aryl⁵ für 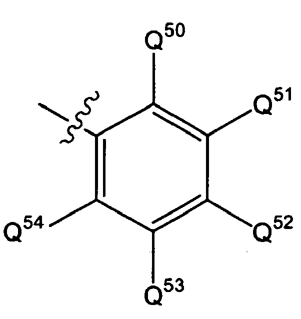 steht;
Q¹⁰, Q¹¹, Q¹², Q¹³, Q¹⁴, Q²⁰, Q²¹, Q²², Q³⁰, Q³¹, Q³², Q³³, Q³⁴, Q⁴⁰, Q⁴¹, Q⁴², Q⁴³, Q⁴⁴, Q⁵⁰, Q⁵¹, Q⁵², Q⁵³ und Q⁵⁴
unabhängig voneinander H, F, Cl, Br, I, -CN, -NO₂, C₁₋₄-Alkyl, OH, O-C₁₋₄-Alkyl, SH oder S-C₁₋₄-Alkyl bedeuten.

4. Substituierte 2-Pyrrolidin-2-yl-1H-indol-Derivate nach Anspruch 3,
**dadurch gekennzeichnet, daß**
R¹ H, Methyl, But-2-in oder Diethylaminoethyl bedeutet;
R² C(=O)-R²⁰ oder SO₂-R²¹ bedeutet;
R³ Phenyl bedeutet;
R⁴ 5-H oder 5-Cl bedeutet;
R²⁰ Methyl, Ethyl, n-Propyl, n-Butyl, 1-Methylpropyl, 2-Methylpropyl, tert-Butyl, n-Pentyl, 1-Methylbutyl, 2,2-Dimethylpropyl, 1-Propylbutyl, 1-Ethylpentyl, Prop-1-enyl, Prop-2-enyl, But-1-enyl, But-2-enyl, But-3-enyl, -(CH₂)₂-CO₂-Methyl, -(CH₂)₂-CO₂-Ethyl, -CH₂-O-CH₃, -CH₂-O-(CH₂)₂-O-CH₃, -CH₂-O-Phenyl, -CH₂-O-(4-Chlorphenyl), -CH(CH₃)-O-Phenyl, -(CH₂)₃-O-Phenyl, Dimethylaminomethyl, Cyclopropyl, Cyclobutyl, Cyclopentyl, Cyclohexyl, 1-Adamantyl, -CH₂-Cyclopropyl, -CH₂-Cyclopentyl, Aryl¹, 1-Naphthyl, CH₂-Aryl³, -CH(C₂H₅)-Aryl³, -CH=CH-Aryl⁴, Furan-2-yl, Furan-3-yl, 5-tert-Butyl-2-methyl-furan-3-yl, 2,5-Dimethyl-furan-3-yl, Pyrazol-4-yl, Pyrazol-5-yl, 1-tert-Butyl-3-methyl-pyrazol-5-yl, 1-Phenyl-3-propyl-pyrazol-4-yl, Isoxazol-3-yl, Isoxazol-4-yl, Isoxazol-5-yl, 5-Methyl-4-(2-chlorphenyl)-isoxazol-3-yl, 5-Methyl-3-(2-chlor-6-fluorphenyl)-isoxazol-4-yl, Pyridin-2-yl, Pyridin-3-yl, Pyridin-4-yl, 2-Chlorpyridin-3-yl, 6-Chlorpyridin-3-yl, 2-(CH₃-S-)-pyridin-3-yl, 2-Methyl-6-trifluormethyl-pyridin-3-yl, 4,7,7-Trimethyloxabicyclo[2.2.1]heptan-3-on oder -CH₂-Imidazolidin-2,4-dion bedeutet;
R²¹ Aryl⁵, Thien-2-yl, Thien-3-yl oder 5-Chlor-thien-2-yl bedeutet oder für 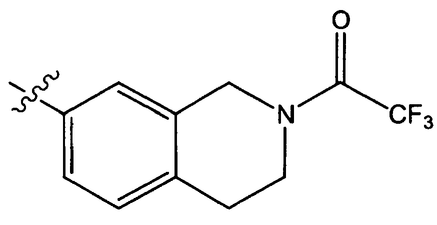 steht;
Aryl¹ für 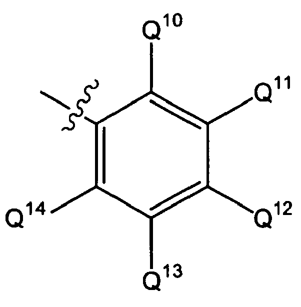 steht;
Aryl³ für 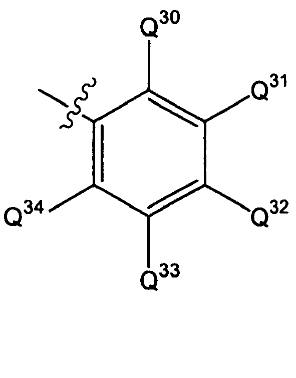 steht;
Aryl⁴ für 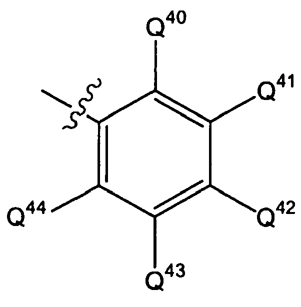 steht;
Aryl⁵ für 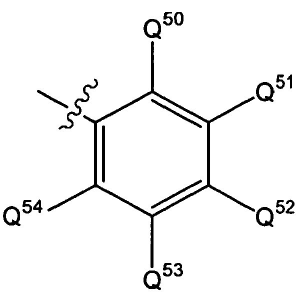 steht;
Q¹⁰, Q¹¹, Q¹², Q¹³ und Q¹⁴ unabhängig voneinander H, F, Cl, Br, -NO₂, Methyl, Trifluormethyl, Ethyl, n-Propyl, 1-Methylethyl, O-CH₃, O-CF₃, -O-C₂H₅, S- CH₃ oder S-CF₃ bedeuten;
Q³⁰, Q³¹, Q³², Q³³ und Q³⁴ unabhängig voneinander H, Cl oder O-CH₃ bedeuten;
Q⁴⁰, Q⁴¹, Q⁴², Q⁴³ und Q⁴⁴ unabhängig voneinander H, Cl, CH₃ oder CF₃ bedeuten;
Q⁵⁰, Q⁵¹, Q⁵², Q⁵³ und Q⁵⁴ unabhängig voneinander H, F, Cl, Br, Methyl, Ethyl, n-Propyl, 1-Methylethyl, O-CH₃, O-CF₃ O-CH₂CH₃, O-CH₂CH₂CH₃, O-CH₂CH₂CH₂CH₃ bedeuten.

5. Substituierte 2-Pyrrolidin-2-yl-1 H-indol-Derivate nach Anspruch 4,
**dadurch gekennzeichnet, daß**
R¹ H, Methyl, But-2-in oder Diethylaminoethyl bedeutet;
R² C(=O)-R²⁰ oder SO₂-R²¹ bedeutet;
R³ Phenyl bedeutet;
R⁴ 5-H oder 5-Cl bedeutet;
R²⁰ n-Propyl, n-Butyl, n-Pentyl, 1-Methylbutyl, 2,2-Dimethylpropyl, 1-Propylbutyl, 1-Ethylpentyl, But-3-enyl, -(CH₂)₂-CO₂-Methyl, -(CH₂)₂-CO₂-Ethyl, -CH₂-O-CH₃, -CH₂-O-(CH₂)₂-O-CH₃, -CH₂-O-Phenyl, -CH₂-O-(4-Chlorphenyl), -CH(CH₃)-O-Phenyl, -(CH₂)₃-O-Phenyl, Dimethylaminomethyl, Cyclopropyl, Cyclobutyl, Cyclohexyl, 1-Adamantyl, -CH₂-Cyclopentyl, 4-Fluorphenyl, 4-Chlorphenyl, 2-Bromphenyl, 3-Bromphenyl, 2,3-Difluorphenyl, 3-Fluor-4-trifluormethylphenyl, 3-Fluor-6-trifluormethylphenyl, 3-Fluor-4-methylphenyl, 3,5-Difluorphenyl, 2-Chlor-4-nitrophenyl, 2-Chlor-5-trifluormethylphenyl, 4-Brom-3-methylphenyl, 2,3-Difluor-4-methylphenyl, 2,6-Difluor-3-methylphenyl, 2,3,4,5,6-Pentafluorphenyl, 4-Nitrophenyl, 4-Methyl-3-nitrophenyl, 3-Methylphenyl, 4-Methylphenyl, 2,3-Dimethylphenyl, 3-Trifluormethylphenyl, 2,5-Trifluormethylphenyl, 3,5-Trifluormethylphenyl, 4-Ethylphenyl, 4-n-Propylphenyl, 3-Methoxyphenyl, 3,5-Dimethoxyphenyl, 3-Trifluormethoxyphenyl, 4-Trifluormethoxyphenyl, 2-Ethoxyphenyl, 4-Ethoxyphenyl, 4-S-CF₃-Phenyl, Benzyl, 4-Chlorbenzyl, 3-Methoxybenzyl, 2,5-Dimethoxybenzyl,-CH(C₂H₅)-Phenyl, -CH=CH-(2-Chlorphenyl), -CH=CH-(3-Trifluormethylphenyl), Furan-2-yl, 5-tert-Butyl-2-methyl-furan-3-yl, 2,5-Dimethyl-furan-3-yl, 1-tert-Butyl-3-methyl-pyrazol-5-yl, 1-Phenyl-3-propyl-pyrazol-4-yl, Isoxazol-5-yl, 5-Methyl-4-(2-chlorphenyl)-isoxazol-3-yl, 5-Methyl-3-(2-chlor-6-fluorphenyl)-isoxazol-4-yl, Pyridin-2-yl, 2-Chlorpyridin-3-yl, 6-Chlorpyridin-3-yl, 2-(CH₃-S-)-pyridin-3-yl, 2-Methyl-6-trifluormethyl-pyridin-3-yl, 4,7,7-Trimethyloxa-bicydo[2.2.1]heptan-3-on oder -CH₂-Imidazolidin-2,4-dion bedeutet; und
R²¹ 3-Chlor-4-fluorphenyl, 2-Chlorphenyl, 4-Chlorphenyl, 3-Chlor-2-methylphenyl, 3-Bromphenyl, 2,3,5,6-Tetramethylphenyl, 4-n-Butylphenyl, 4-Methoxyphenyl, 2,5-Dimethoxyphenyl, 4-Trifluormethoxyphenyl, 4-(n-Butoxy)-phenyl, Thien-2-yl, 5-Chlor-thien-2-yl bedeutet oder für 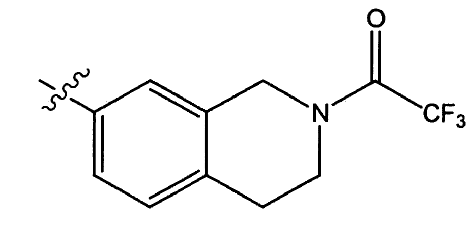 steht.

6. Substituierte 2-Pyrrolidin-2-yl-1H-indol-Derivate nach Anspruch 1, ausgewählt aus der Gruppe, die umfaßt:
{2-[5-Chlor-1-(2-diethylamino-ethyl)-3-phenyl-1H-indol-2-yl]-pyrrolidin-1-yl}-furan-2-yl-methanon;
4-{2-[5-Chlor-1-(2-diethylamino-ethyl)-3-phenyl-1H-indol-2-yl]-pyrrolidin-1-yl}-4-oxo-buttersäureethylester;
{2-[5-Chlor-1-(2-diethylamino-ethyl)-3-phenyl-1H-indol-2-yl]-pyrrolidin-1-yl}-(4-fluor-phenyl)-methanon;
{2-[5-Chlor-1-(2-diethylamino-ethyl)-3-phenyl-1H-indol-2-yl]-pyrrolidin-1-yl}-cyclobutyl-methanon;
{2-[5-Chlor-1-(2-diethylamino-ethyl)-3-phenyl-1H-indol-2-yl]-pyrrolidin-1-yl}-(6-chlor-pyridin-3-yl)-methanon;
1-{2-[5-Chlor-1-(2-diethylamino-ethyl)-3-phenyl-1H-indol-2-yl]-pyrrolidin-1-yl}-hexan-1-on;
{2-[5-Chlor-1-(2-diethylamino-ethyl)-3-phenyl-1H-indol-2-yl]-pyrrolidin-1-yl}-(3,5-dimethoxy-phenyl)-methanon;
{2-[5-Chlor-1-(2-diethylamino-ethyl)-3-phenyl-1H-indol-2-yl]-pyrrolidin-1-yl}-(4-ethoxy-phenyl)-methanon;
1-[2-(5-Chlor-1-methyl-3-phenyl-1H-indol-2-yl)-pyrrolidin-1-yl]-2-(2-methoxy-ethoxy)-ethanon;
[2-(5-Chlor-1-methyl-3-phenyl-1H-indol-2-yl)-pyrrolidin-1-yl]-cyclopropyl-methanon; und
1-[2-(1-But-2-inyl-5-chlor-3-phenyl-1H-indol-2-yl)-pyrrolidin-1-yl]-2-methoxy-ethanon.

7. Verfahren zur Herstellung eines substituierten 2-Pyrrolidin-2-yl-1H-indol-Derivats der allgemeinen Formel (I) 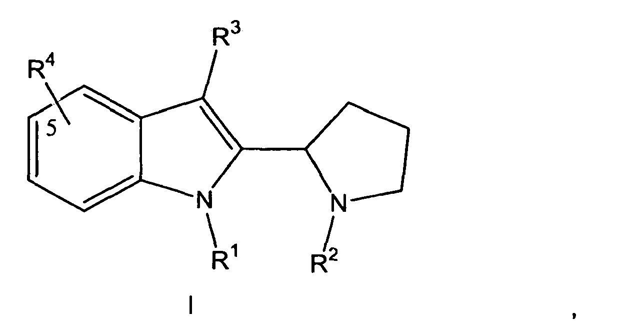 worin
R¹ H, Alkyl, Cycloalkyl, Alkyl-Cycloalkyl, Alkyl-Aryl oder Alkyl-Heterocyclyl bedeutet;
R² Alkyl, Cycloalkyl, Alkyl-Cycloalkyl, Alkyl-Aryl, Alkyl-Heterocyclyl, C(=O)-R²⁰ oder SO₂-R²¹ bedeutet;
R³ Alkyl, Aryl oder Heterocyclyl bedeutet;
R⁴ H, F, Cl, Br, I, -CN, OR⁴⁰, Alkyl, Cycloalkyl oder NO₂ bedeutet;
R²⁰ Alkyl, Cycloalkyl, Alkyl-Cycloalkyl, Aryl, Alkyl-Aryl, Heterocyclyl, Alkyl-Heterocyclyl, OR²⁰⁰ oder NHR²⁰¹ bedeutet;
R²¹ Alkyl, Aryl, Alkyl-Aryl, Heterocyclyl oder Alkyl-Heterocyclyl bedeutet;
R⁴⁰ H, Alkyl, Cycloalkyl, Alkyl-Cycloalkyl, Aryl oder Alkyl-Aryl bedeutet;
R²⁰⁰ Alkyl, Cycloalkyl, Alkyl-Cycloalkyl, Aryl, Alkyl-Aryl, Heterocyclyl oder Alkyl-Heterocyclyl bedeutet; und
R²⁰¹ Alkyl, Aryl, Alkyl-Aryl, Heterocyclyl oder Alkyl-Heterocyclyl bedeutet;
wobei
"Cycloalkyl" einen alicyclischen gesättigten oder ungesättigten Kohlenwasserstoff-Rest bedeutet, wobei der Rest unsubstituiert oder einfach substituiert oder mehrfach gleich oder verschieden substituiert und ggf. benzokondensiert sein kann und auch bi-, tri- oder polycyclische Alicyclen umfaßt,
"Alkyl" acyclische gesättigte oder ungesättigte Kohlenwasserstoffreste ("Alkanyle", "Alkenyle" und "Alkinyle") umfasst, die verzweigt oder geradkettig sowie unsubstituiert oder einfach substituiert oder mehrfach gleich oder verschieden substituiert sein können;
und "Heterocyclyl" für einen monocyclischen oder polycyclischen organischen Rest steht, in dem mindestens ein Cyclus 1 Heteroatom oder 2, 3, 4 oder 5 gleiche oder verschiedene Heteroatome enthält, das/die aus der Gruppe, die N, O und S enthält, ausgewählt ist/sind, wobei der Rest gesättigt oder ungesättigt ist und unsubstituiert oder einfach substituiert oder mehrfach gleich oder verschieden substituiert ist,
**dadurch gekennzeichnet, daß**
in einem Verfahrensschritt (A) ein 2-Pyrrolidin-2-yl-1H-indol-Derivat der allgemeinen Formel (II) 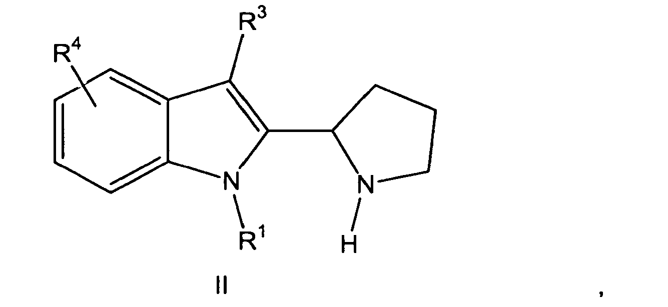 worin
R¹, R³ und R⁴ wie oben in diesem Anspruch definiert sind; mit einer Verbindung der allgemeinen Formel (III)
R²-Hal III
worin
R² wie oben in diesem Anspruch definiert ist; und
Hal Cl oder Br bedeutet;
umgesetzt wird.

8. Verfahren zur Herstellung eines substituierten 2-Pyrrolidin-2-yl-1H-indol-Derivats der allgemeinen Formel (I) 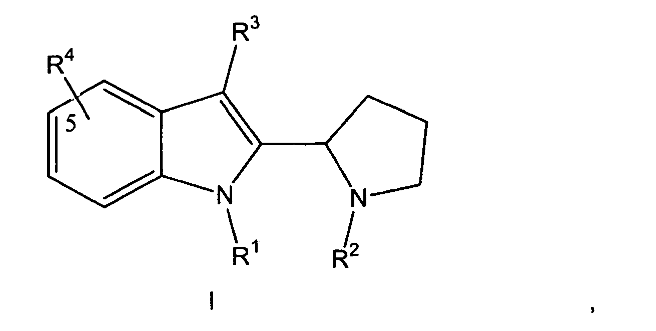 worin
R¹ und R² H bedeuten;
R³ Alkyl, Aryl oder Heterocyclyl bedeutet;
R⁴ H, F, Cl, Br, I, -CN, OR⁴⁰, Alkyl, Cycloalkyl oder NO₂ bedeutet; und
R⁴⁰ H, Alkyl, Cycloalkyl, Alkyl-Cycloalkyl, Aryl oder Alkyl-Aryl bedeutet;
wobei
"Cycloalkyl" einen alicyclischen gesättigten oder ungesättigten Kohlenwasserstoff-Rest bedeutet, wobei der Rest unsubstituiert oder einfach substituiert oder mehrfach gleich oder verschieden substituiert und ggf. benzokondensiert sein kann und auch bi-, tri- oder polycyclische Alicyclen umfaßt,
"Alkyl" acyclische gesättigte oder ungesättigte Kohlenwasserstoffreste ("Alkanyle", "Alkenyle" und "Alkinyle") umfasst, die verzweigt oder geradkettig sowie unsubstituiert oder einfach substituiert oder mehrfach gleich oder verschieden substituiert sein können;
und "Heterocyclyl" für einen monocyclischen oder polycyclischen organischen Rest steht, in dem mindestens ein Cyclus 1 Heteroatom oder 2, 3, 4 oder 5 gleiche oder verschiedene Heteroatome enthält, das/die aus der Gruppe, die N, O und S enthält, ausgewählt ist/sind, wobei der Rest gesättigt oder ungesättigt ist und unsubstituiert oder einfach substituiert oder mehrfach gleich oder verschieden substituiert ist,
**dadurch gekennzeichnet, daß** in einem Verfahrensschritt (B) ein 2-Pyrrolidin-2-yl-1 H-indol-Derivat der allgemeinen Formel (IV) 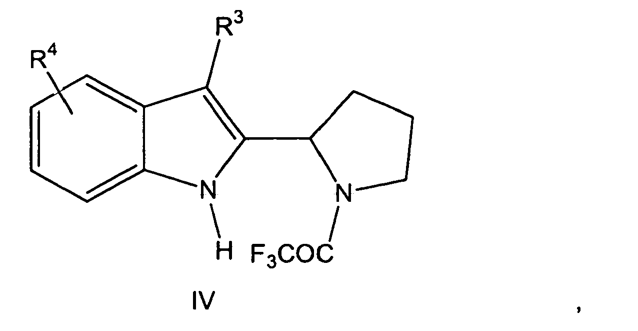 worin
R³ und R⁴ wie oben in diesem Anspruch definiert sind;
mit Kaliumcarbonat oder Natriumhydroxid umgesetzt wird.

9. Verfahren zur Herstellung eines substituierten 2-Pyrrolidin-2-yl-1 H-indol-Derivats der allgemeinen Formel (I) 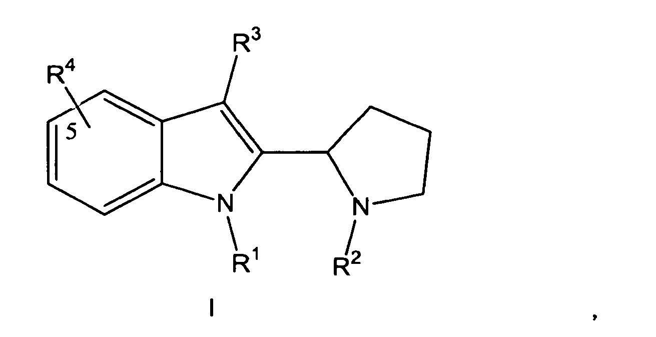 worin
R¹ Alkyl, Cycloalkyl, Alkyl-Cycloalkyl, Alkyl-Aryl oder Alkyl-Heterocyclyl bedeutet;
R² H bedeutet;
R³ Alkyl, Aryl oder Heterocyclyl bedeutet;
R⁴ H, F, Cl, Br, 1, -CN, OR⁴⁰, Alkyl, Cycloalkyl oder NO₂ bedeutet; und
R⁴⁰ H, Alkyl, Cycloalkyl, Alkyl-Cycloalkyl, Aryl oder Alkyl-Aryl bedeutet;
wobei
"Cycloalkyl" einen alicyclischen gesättigten oder ungesättigten Kohlenwasserstoff-Rest bedeutet, wobei der Rest unsubstituiert oder einfach substituiert oder mehrfach gleich oder verschieden substituiert und ggf. benzokondensiert sein kann und auch bi-, tri- oder polycyclische Alicyclen umfaßt,
"Alkyl" acyclische gesättigte oder ungesättigte Kohlenwasserstoffreste ("Alkanyle", "Alkenyle" und "Alkinyle") umfasst, die verzweigt oder geradkettig sowie unsubstituiert oder einfach substituiert oder mehrfach gleich oder verschieden substituiert sein können;
und "Heterocyclyl" für einen monocyclischen oder polycyclischen organischen Rest steht, in dem mindestens ein Cyclus 1 Heteroatom oder 2, 3, 4 oder 5 gleiche oder verschiedene Heteroatome enthält, das/die aus der Gruppe, die N, O und S enthält, ausgewählt ist/sind, wobei der Rest gesättigt oder ungesättigt ist und unsubstituiert oder einfach substituiert oder mehrfach gleich oder verschieden substituiert ist,
**dadurch gekennzeichnet, daß**
in einem Verfahrensschritt (C) ein 2-Pyrrolidin-2-yl-1 H-indol-Derivat der allgemeinen Formel (V) 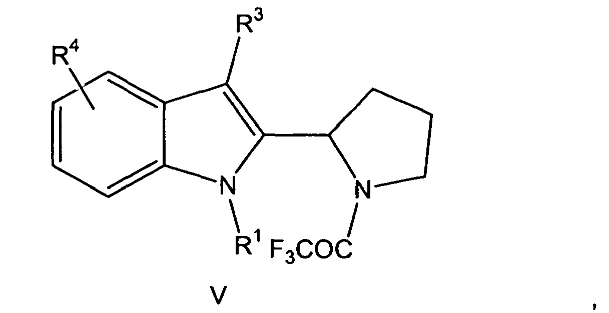 worin
R¹, R³ und R⁴ wie oben in diesem Anspruch definiert sind;
mit Kaliumcarbonat oder Natriumhydroxid umgesetzt wird.

10. Verfahren zur Herstellung eines 2-Pyrrolidin-2-yl-1 H-indol-Derivats der allgemeinen Formel (V) 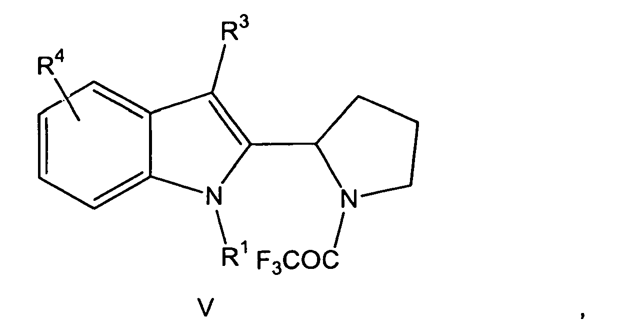 worin
R¹ Alkyl, Cycloalkyl, Alkyl-Cycloalkyl, Alkyl-Aryl oder Alkyl-Heterocyclyl bedeutet;
R³ Alkyl, Aryl oder Heterocyclyl bedeutet;
R⁴ H, F, Cl, Br, I, -CN, OR⁴⁰, Alkyl, Cycloalkyl oder NO₂ bedeutet;
R⁴⁰ H, Alkyl, Cycloalkyl, Alkyl-Cycloalkyl, Aryl oder Alkyl-Aryl bedeutet;
wobei
"Cycloalkyl" einen alicyclischen gesättigten oder ungesättigten Kohlenwasserstoff-Rest bedeutet, wobei der Rest unsubstituiert oder einfach substituiert oder mehrfach gleich oder verschieden substituiert und ggf. benzokondensiert sein kann und auch bi-, tri- oder polycyclische Alicyclen umfaßt,
"Alkyl" acyclische gesättigte oder ungesättigte Kohlenwasserstoffreste ("Alkanyle", "Alkenyle" und "Alkinyle") umfasst, die verzweigt oder geradkettig sowie unsubstituiert oder einfach substituiert oder mehrfach gleich oder verschieden substituiert sein können;
und "Heterocyclyl" für einen monocyclischen oder polycyclischen organischen Rest steht, in dem mindestens ein Cyclus 1 Heteroatom oder 2, 3, 4 oder 5 gleiche oder verschiedene Heteroatome enthält, das/die aus der Gruppe, die N, O und S enthält, ausgewählt ist/sind, wobei der Rest gesättigt oder ungesättigt ist und unsubstituiert oder einfach substituiert oder mehrfach gleich oder verschieden substituiert ist,
**dadurch gekennzeichnet, daß** in einem Verfahrensschritt (D) ein 2-Pyrrolidin-2-yl-1 H-indol-Derivat der allgemeinen Formel (IV) 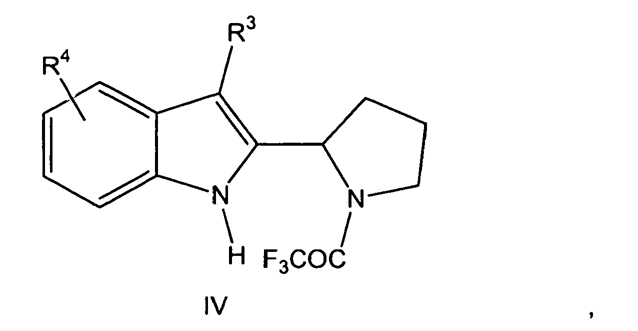 worin
R³ und R⁴ wie oben in diesem Anspruch definiert sind;
mit einer Verbindung der allgemeinen Formel (VI)
R¹-Hal VI ,
worin
R¹ wie oben in diesem Anspruch definiert ist; und
Hal Cl, Br oder I bedeutet;
umgesetzt wird.

11. Verfahren nach Anspruch 9, **dadurch gekennzeichnet, daß** vor dem Verfahrensschritt (C) der Verfahrensschritt (D) gemäß Anspruch 10 ausgeführt wird.

12. Verfahren nach Anspruch 8 oder 11, **dadurch gekennzeichnet, daß** vor dem Verfahrensschritt (B) bzw. dem Verfahrenschritt (D) die folgenden Verfahrenschritte ausgeführt werden:
(E) Umsetzung von N-Trifluoracetylprolinalkylester (VII) 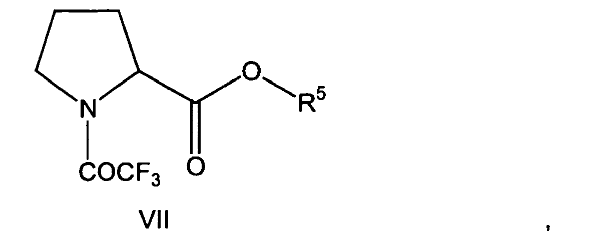 worin
R⁵ Alkyl bedeutet;
mit einem Anilin-Derivat der allgemeinen Formel (VIII) 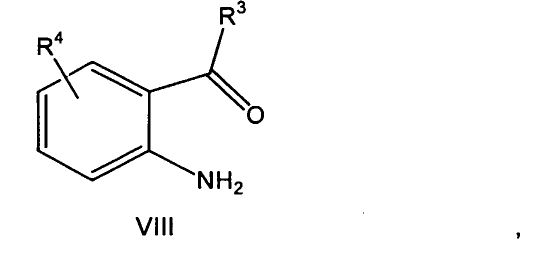 worin
R³ Alkyl, Aryl oder Heterocyclyl bedeutet;
R⁴ H, F, Cl, Br, I, -CN, OR⁴⁰, Alkyl, Cycloalkyl oder NO₂ bedeutet; und
R⁴⁰ H, Alkyl, Cycloalkyl, Alkyl-Cycloalkyl, Aryl oder Alkyl-Aryl bedeutet;
und
(F) anschließende Überführung des Produkts (IX) aus Verfahrensschritt (E) 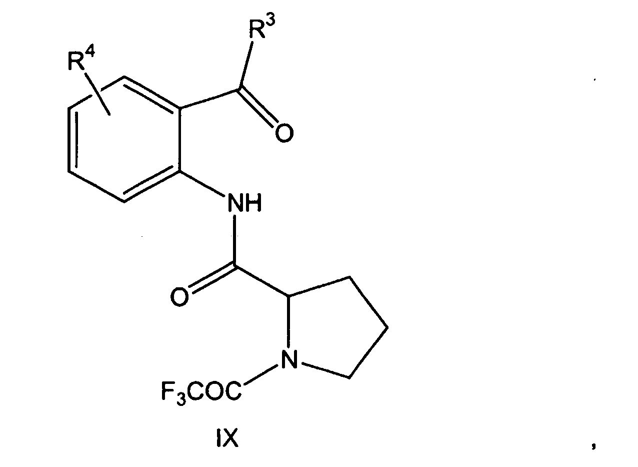
worin
R³ und R⁴ wie oben in diesem Anspruch definiert sind;
mittels McMurry-Kupplung in ein 2-Pyrrolidin-2-yl-1H-indol-Derivat der allgemeinen Formel (IV) 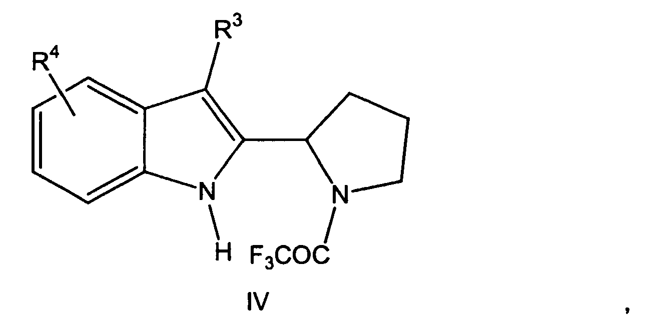 worin
R³ und R⁴ wie oben in diesem Anspruch definiert sind.

13. Arzneimittel, enthaltend mindestens eine Verbindung der allgemeinen Formel (I) 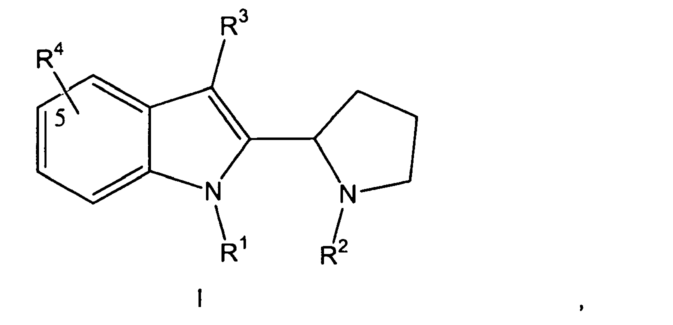 in Form ihrer Racemate, ihrer reinen Stereoisomeren, oder in Form von Mischungen der Stereoisomeren, in einem beliebigen Mischungsverhältnis; in dargestellter Form oder in Form ihrer Säuren oder ihrer Basen oder in Form ihrer Salze, oder in Form ihrer Solvate,
wobei
R¹ H, Alkyl, Cycloalkyl, Alkyl-Cycloalkyl, Alkyl-Aryl oder Alkyl-Heterocyclyl bedeutet;
R² H, Alkyl, Cycloalkyl, Alkyl-Cycloalkyl, Alkyl-Aryl, Alkyl-Heterocyclyl, C(=O)-R²⁰ oder SO₂-R²¹ bedeutet;
R³ Alkyl, Aryl oder Heterocyclyl bedeutet;
R⁴ H, F, Cl, Br, l, -CN, OR⁴⁰, Alkyl, Cycloalkyl oder NO₂ bedeutet;
R²⁰ Alkyl, Cycloalkyl, Alkyl-Cycloalkyl, Aryl, Alkyl-Aryl, Heterocyclyl, Alkyl-Heterocyclyl, OR²⁰⁰ oder NHR²⁰¹ bedeutet;
R²¹ Alkyl, Aryl, Alkyl-Aryl, Heterocyclyl oder Alkyl-Heterocyclyl bedeutet;
R⁴⁰ H, Alkyl, Cycloalkyl, Alkyl-Cycloalkyl, Aryl oder Alkyl-Aryl bedeutet;
R²⁰⁰ Alkyl, Cycloalkyl, Alkyl-Cycloalkyl, Aryl, Alkyl-Aryl, Heterocyclyl oder Alkyl-Heterocyclyl bedeutet; und
R²⁰¹ Alkyl, Aryl, Alkyl-Aryl, Heterocyclyl oder Alkyl-Heterocyclyl bedeutet
wobei
"Cycloalkyl" einen alicyclischen gesättigten oder ungesättigten Kohlenwasserstoff-Rest bedeutet, wobei der Rest unsubstituiert oder einfach substituiert oder mehrfach gleich oder verschieden substituiert und ggf. benzokondensiert sein kann und auch bi-, tri- oder polycyclische Alicyclen umfaßt,
"Alkyl" acyclische gesättigte oder ungesättigte Kohlenwasserstoffreste ("Alkanyle", "Alkenyle" und "Alkinyle") umfasst, die verzweigt oder geradkettig sowie unsubstituiert oder einfach substituiert oder mehrfach gleich oder verschieden substituiert sein können;
und "Heterocyclyl" für einen monocyclischen oder polycyclischen organischen Rest steht, in dem mindestens ein Cyclus 1 Heteroatom oder 2, 3, 4 oder 5 gleiche oder verschiedene Heteroatome enthält, das/die aus der Gruppe, die N, O und S enthält, ausgewählt ist/sind, wobei der Rest gesättigt oder ungesättigt ist und unsubstituiert oder einfach substituiert oder mehrfach gleich oder verschieden substituiert ist.

14. Verwendung eines substituierten 2-Pyrrolidin-2-yl-1 H-indol-Derivats der allgemeinen Formel (I) 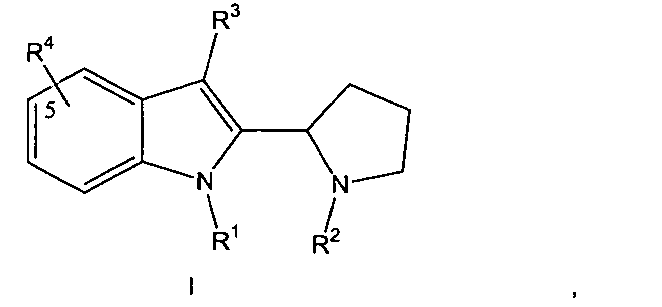 in Form ihrer Racemate, ihrer reinen Stereoisomeren, oder in Form von Mischungen der Stereoisomeren, in einem beliebigen Mischungsverhältnis; in dargestellter Form oder in Form ihrer Säuren oder ihrer Basen oder in Form ihrer Salze, oder in Form ihrer Solvate,
wobei
R¹ H, Alkyl, Cycloalkyl, Alkyl-Cycloalkyl, Alkyl-Aryl oder Alkyl-Heterocyclyl bedeutet;
R² H, Alkyl, Cycloalkyl, Alkyl-Cycloalkyl, Alkyl-Aryl, Alkyl-Heterocyclyl, C(=O)-R²⁰ oder SO₂-R²¹ bedeutet;
R³ Alkyl, Aryl oder Heterocyclyl bedeutet;
R⁴ H, F, Cl, Br, l, -CN, OR⁴⁰, Alkyl, Cycloalkyl oder NO₂ bedeutet;
R²⁰ Alkyl, Cycloalkyl, Alkyl-Cycloalkyl, Aryl, Alkyl-Aryl, Heterocyclyl, Alkyl-Heterocyclyl, OR²⁰⁰ oder NHR²⁰¹ bedeutet;
R²¹ Alkyl, Aryl, Alkyl-Aryl, Heterocyclyl oder Alkyl-Heterocyclyl bedeutet;
R⁴⁰ H, Alkyl, Cycloalkyl, Alkyl-Cycloalkyl, Aryl oder Alkyl-Aryl bedeutet;
R²⁰⁰ Alkyl, Cycloalkyl, Alkyl-Cycloalkyl, Aryl, Alkyl-Aryl, Heterocyclyl oder Alkyl-Heterocyclyl bedeutet; und
R²⁰¹ Alkyl, Aryl, Alkyl-Aryl, Heterocyclyl oder Alkyl-Heterocyclyl bedeutet;
wobei
"Cycloalkyl" einen alicyclischen gesättigten oder ungesättigten Kohlenwasserstoff-Rest bedeutet, wobei der Rest unsubstituiert oder einfach substituiert oder mehrfach gleich oder verschieden substituiert und ggf. benzokondensiert sein kann und auch bi-, tri- oder polycyclische Alicyclen umfaßt,
"Alkyl" acyclische gesättigte oder ungesättigte Kohlenwasserstoffreste ("Alkanyle", "Alkenyle" und "Alkinyle") umfasst, die verzweigt oder geradkettig sowie unsubstituiert oder einfach substituiert oder mehrfach gleich oder verschieden substituiert sein können;
und "Heterocyclyl" für einen monocyclischen oder polycyclischen organischen Rest steht, in dem mindestens ein Cyclus 1 Heteroatom oder 2, 3, 4 oder 5 gleiche oder verschiedene Heteroatome enthält, das/die aus der Gruppe, die N, O und S enthält, ausgewählt ist/sind, wobei der Rest gesättigt oder ungesättigt ist und unsubstituiert oder einfach substituiert oder mehrfach gleich oder verschieden substituiert ist;
zur Herstellung eines Medikaments zur NO-Synthase-Inhibierung.

15. Verwendung eines substituierten 2-Pyrrolidin-2-yl-1H-indol-Derivats der allgemeinen Formel (I) 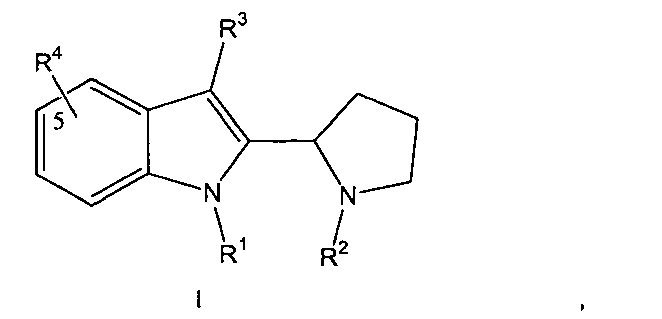 in Form ihrer Racemate, ihrer reinen Stereoisomeren, oder in Form von Mischungen der Stereoisomeren, in einem beliebigen Mischungsverhältnis; in dargestellter Form oder in Form ihrer Säuren oder ihrer Basen oder in Form ihrer Salze, oder in Form ihrer Solvate,
wobei
R¹ H, Alkyl, Cycloalkyl, Alkyl-Cycloalkyl, Alkyl-Aryl oder Alkyl-Heterocyclyl bedeutet;
R² H, Alkyl, Cycloalkyl, Alkyl-Cycloalkyl, Alkyl-Aryl, Alkyl-Heterocyclyl, C(=O)-R²⁰ oder SO₂-R²¹ bedeutet;
R³ Alkyl, Aryl oder Heterocyclyl bedeutet;
R⁴ H, F, Cl, Br, I, -CN, OR⁴⁰, Alkyl, Cycloalkyl oder NO₂ bedeutet;
R²⁰ Alkyl, Cycloalkyl, Alkyl-Cycloalkyl, Aryl, Alkyl-Aryl, Heterocyclyl, Alkyl-Heterocyclyl, OR²⁰⁰ oder NHR²⁰¹ bedeutet;
R²¹ Alkyl, Aryl, Alkyl-Aryl, Heterocyclyl oder Alkyl-Heterocyclyl bedeutet;
R⁴⁰ H, Alkyl, Cycloalkyl, Alkyl-Cycloalkyl, Aryl oder Alkyl-Aryl bedeutet;
R²⁰⁰ Alkyl, Cycloalkyl, Alkyl-Cycloalkyl, Aryl, Alkyl-Aryl, Heterocyclyl oder Alkyl-Heterocyclyl bedeutet; und
R²⁰¹ Alkyl, Aryl, Alkyl-Aryl, Heterocyclyl oder Alkyl-Heterocyclyl bedeutet
wobei
"Cycloalkyl" einen alicyclischen gesättigten oder ungesättigten Kohlenwasserstoff-Rest bedeutet, wobei der Rest unsubstituiert oder einfach substituiert oder mehrfach gleich oder verschieden substituiert und ggf. benzokondensiert sein kann und auch bi-, tri- oder polycyclische Alicyclen umfaßt,
"Alkyl" acyclische gesättigte oder ungesättigte Kohlenwasserstoffreste ("Alkanyle", "Alkenyle" und "Alkinyle") umfasst, die verzweigt oder geradkettig sowie unsubstituiert oder einfach substituiert oder mehrfach gleich oder verschieden substituiert sein können;
und "Heterocyclyl" für einen monocyclischen oder polycyclischen organischen Rest steht, in dem mindestens ein Cyclus 1 Heteroatom oder 2, 3, 4 oder 5 gleiche oder verschiedene Heteroatome enthält, das/die aus der Gruppe, die N, O und S enthält, ausgewählt ist/sind, wobei der Rest gesättigt oder ungesättigt ist und unsubstituiert oder einfach substituiert oder mehrfach gleich oder verschieden substituiert ist;
zur Herstellung eines Medikaments zur Prophylaxe und Behandlung von Migräne.

16. Verwendung eines substituierten 2-Pyrrolidin-2-yl-1 H-indol-Derivats der allgemeinen Formel (I) 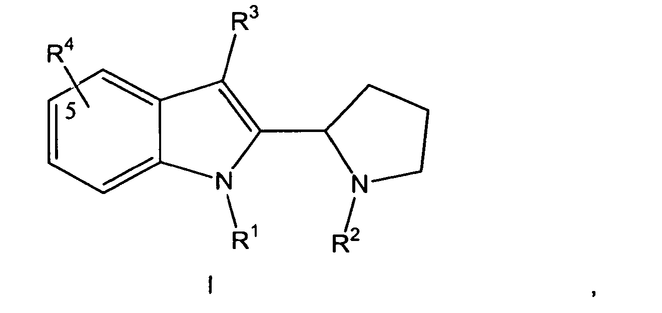 in Form ihrer Racemate, ihrer reinen Stereoisomeren, oder in Form von Mischungen der Stereoisomeren, in einem beliebigen Mischungsverhältnis; in dargestellter Form oder in Form ihrer Säuren oder ihrer Basen oder in Form ihrer Salze, oder in Form ihrer Solvate,
wobei
R¹ H, Alkyl, Cycloalkyl, Alkyl-Cycloalkyl, Alkyl-Aryl oder Alkyl-Heterocyclyl bedeutet;
R² H, Alkyl, Cycloalkyl, Alkyl-Cycloalkyl, Alkyl-Aryl, Alkyl-Heterocyclyl, C(=O)-R²⁰ oder SO₂-R²¹ bedeutet;
R³ Alkyl, Aryl oder Heterocyclyl bedeutet;
R⁴ H, F, Cl, Br, I, -CN, OR⁴⁰, Alkyl, Cycloalkyl oder NO₂ bedeutet;
R²⁰ Alkyl, Cycloalkyl, Alkyl-Cycloalkyl, Aryl, Alkyl-Aryl, Heterocyclyl, Alkyl-Heterocyclyl, OR²⁰⁰ oder NHR²⁰¹ bedeutet;
R²¹ Alkyl, Aryl, Alkyl-Aryl, Heterocyclyl oder Alkyl-Heterocyclyl bedeutet;
R⁴⁰ H, Alkyl, Cycloalkyl, Alkyl-Cycloalkyl, Aryl oder Alkyl-Aryl bedeutet;
R²⁰⁰ Alkyl, Cycloalkyl, Alkyl-Cycloalkyl, Aryl, Alkyl-Aryl, Heterocyclyl oder Alkyl-Heterocyclyl bedeutet; und
R²⁰¹ Alkyl, Aryl, Alkyl-Aryl, Heterocyclyl oder Alkyl-Heterocyclyl bedeutet;
wobei
"Cycloalkyl" einen alicyclischen gesättigten oder ungesättigten Kohlenwasserstoff-Rest bedeutet, wobei der Rest unsubstituiert oder einfach substituiert oder mehrfach gleich oder verschieden substituiert und ggf. benzokondensiert sein kann und auch bi-, tri- oder polycyclische Alicyclen umfaßt,
"Alkyl" acyclische gesättigte oder ungesättigte Kohlenwasserstoffreste ("Alkanyle", "Alkenyle" und "Alkinyle") umfasst, die verzweigt oder geradkettig sowie unsubstituiert oder einfach substituiert oder mehrfach gleich oder verschieden substituiert sein können;
und "Heterocyclyl" für einen monocyclischen oder polycyclischen organischen Rest steht, in dem mindestens ein Cyclus 1 Heteroatom oder 2, 3, 4 oder 5 gleiche oder verschiedene Heteroatome enthält, das/die aus der Gruppe, die N, O und S enthält, ausgewählt ist/sind, wobei der Rest gesättigt oder ungesättigt ist und unsubstituiert oder einfach substituiert oder mehrfach gleich oder verschieden substituiert ist;
zur Herstellung eines Medikaments zur Behandlung von septischem Schock, Multipler Sklerose, Morbus Parkinson, Morbus Alzheimer, Morbus Huntington, Entzündungen, Entzündungsschmerz, cerebraler Ischämie, Diabetes, Meningitis, Arteriosklerose und/oder für die Wundheilung.

## Claims

1. Substituted 2-pyrrolidin-2-yl-1H-indole derivatives of the general formula (I) 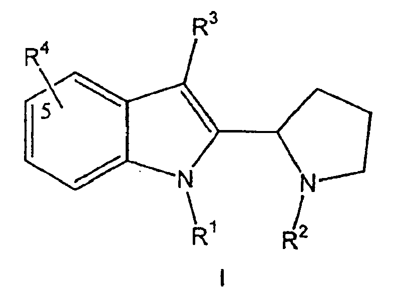 in the form of their racemates, their pure stereoisomers, or in the form of mixtures of the stereoisomers, in any desired mixture ratio; in the form shown or in the form of their acids or their bases or in the form of their salts, or in the form of their solvates,
wherein
R¹ denotes H, alkyl, cycloalkyl, alkyl-cycloalkyl, alkyl-aryl or alkyl-heterocyclyl;
R² denotes H, alkyl, cycloalkyl, alkyl-cycloalkyl, alkyl-aryl, alkyl-heterocyclyl, C(=O)-R²⁰ or SO₂-R²¹;
R³ denotes alkyl, aryl or heterocyclyl;
R⁴ denotes H, F, Cl, Br, I, -CN, OR⁴⁰, alkyl, cycloalkyl or NO₂;
R²⁰ denotes alkyl, cycloalkyl, alkyl-cycloalkyl, aryl, alkyl-aryl, heterocyclyl, alkyl-heterocyclyl, OR²⁰⁰ or NHR²⁰¹;
R²¹ denotes alkyl, aryl, alkyl-aryl, heterocyclyl or alkyl-heterocyclyl;
R⁴⁰ denotes H, alkyl, cycloalkyl, alkyl-cycloalkyl, aryl or alkyl-aryl;
R²⁰⁰ denotes alkyl, cycloalkyl, alkyl-cycloalkyl, aryl, alkyl-aryl, heterocyclyl or alkyl-heterocyclyl; and
R²⁰¹ denotes alkyl, aryl, alkyl-aryl, heterocyclyl or alkyl-heterocyclyl;
wherein
"cycloalkyl" denotes an alicyclic saturated or unsaturated hydrocarbon radical, wherein the radical can be unsubstituted or monosubstituted or polysubstituted by identical or different substituents and optionally benzo-fused and also includes bi-, tri- or polycyclic alicyclic radicals,
"alkyl" includes acyclic saturated or unsaturated hydrocarbon radicals ("alkanyls", "alkenyls" and "alkynyls") which can be branched or straight-chain and unsubstituted or monosubstituted or polysubstituted by identical or different substituents;
and "heterocyclyl" represents a monocyclic or polycyclic organic radical in which at least one ring contains 1 heteroatom or 2, 3, 4 or 5 identical or different heteroatoms which is/are chosen from the group which contains N, O and S, wherein the radical is saturated or unsaturated and unsubstituted or monosubstituted or polysubstituted by identical or different substituents,
with the proviso that
2-(3-phenyl-1H-indol-2-yl)-1-(trifluoroacetyl)-pyrrolidine;
α-methyl-2-(1-methyl-2-pyrrolidinyl)-indole-3-acetic acid methyl ester;
3-(1-cyanoethyl)-2-(1-methyl-2-pyrrolidinyl)-indole; and
2-(1-methyl-2-pyrrolidinyl)-3-vinylindole are excluded.

2. Substituted 2-pyrrolidin-2-yl-1H-indole derivatives according to claim 1, **characterized in that**
R¹ denotes H, alkyl, cycloalkyl or alkyl-cycloalkyl;
R² denotes C(=O)-R²⁰ or SO₂-R²¹;
R³ denotes aryl;
R⁴ denotes H, F, Cl, Br, I, -CN, OR⁴⁰, alkyl, cycloalkyl or NO₂, in each case in the 5-position of the indole ring;
R²⁰ denotes alkyl, cycloalkyl, alkyl-cycloalkyl, aryl, alkyl-aryl, heterocyclyl or alkyl-heterocyclyl;
R²¹ denotes alkyl, aryl, alkyl-aryl, heterocyclyl or alkyl-heterocyclyl; and
R⁴⁰ denotes H, alkyl, cycloalkyl, alkyl-cycloalkyl, aryl or alkyl-aryl.

3. Substituted 2-pyrrolidin-2-yl-1H-indole derivatives according to claim 1, **characterized in that**
R¹ denotes H or alkyl;
R² denotes C (=O) -R²⁰ or SO₂-R²¹;
R³ denotes phenyl;
R⁴ denotes H, F, Cl, Br or I, in each case in the 5-position of the indole ring;
R²⁰ denotes C₁₋₈-alkanyl, C₂₋₈-alkenyl, C₁₋₄-alkanyl-CO₂-C₁₋₄-alkyl, C₁₋₄-alkanyl-O-R²⁰², C₁₋₄-alkanyl-NR²⁰³R²⁰⁴, C₃₋₈-cycloalkyl, 1-adamantyl, 2-adamantyl, - (C₁₋₃-alkyl) -C₃₋₈-cycloalkyl, aryl¹, aryl², C₁₋₄-alkanyl-aryl³, C₂₋₄-alkenyl-aryl⁴; or unsubstituted or substituted furanyl, pyrazolyl, isoxazolyl or pyridinyl; or 4,7,7-trimethyloxabicyclo[2.2.1]heptan-3-one, -CH₂-imidazolidine-2,4-dione;
R²¹ denotes aryl⁵, unsubstituted or substituted thienyl or represents 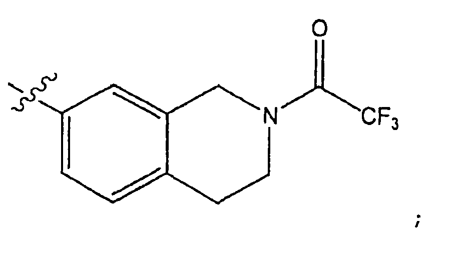
R²⁰² denotes H, C₁₋₄-alkanyl C₁₋₄-alkanyl-OH, C₁₋₄-alkanoyl-O-C₁₋₄-alkanyl or aryl;
R²⁰³ and R²⁰⁴ independently of one another denotes C₁₋₆-alkyl or, with the N atom, form a 5-, 6- or 7-membered saturated heterocyclyl;
aryl¹ represents 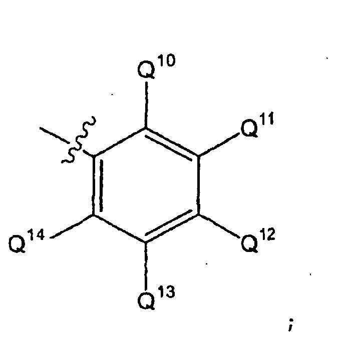
aryl² represents 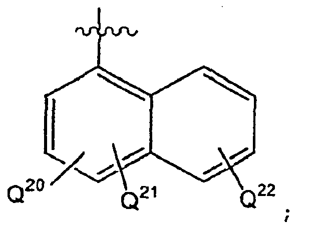
aryl³ represents 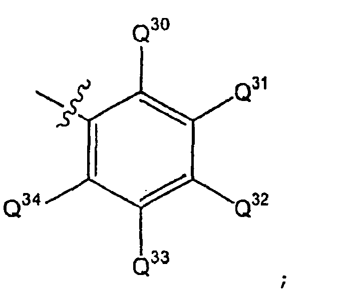
aryl⁴ represents 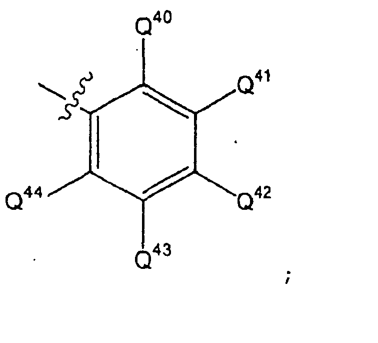
aryl⁵ represents 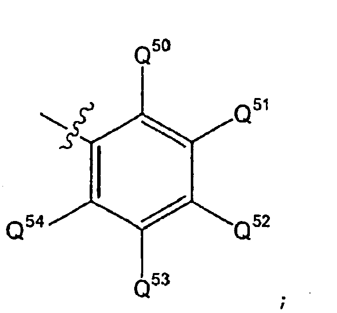 Q¹⁰*,* Q¹¹*,* Q¹²*,* Q¹³*,* Q¹⁴*,* Q²⁰*,* Q²¹*,* Q²²*,* Q³⁰*,* Q³¹*,* Q³²*,* Q³³, Q³⁴, Q⁴⁰, Q⁴¹, Q⁴², Q⁴³, Q⁴⁴, Q⁵⁰, Q⁵¹, Q⁵², Q⁵³ and Q⁵⁴ independently of one another denote H, F, Cl, Br, I, -CN, -NO₂, C₁₋₄-alkyl, OH, O-C₁₋₄-alkyl, SH or S-C₁₋₄-alkyl.

4. Substituted 2-pyrrolidin-2-yl-1H-indole derivatives according to claim 3, **characterized in that**
R¹ denotes H, methyl, but-2-yne or diethylaminoethyl;
R² denotes C(=O)-R²⁰ or SO₂-R²¹;
R³ denotes phenyl ;
R⁴ denotes 5-H or 5-Cl;
R²⁰ denotes methyl, ethyl, n-propyl, n-butyl, 1-methylpropyl, 2-methylpropyl, tert-butyl, n-pentyl, 1-methylbutyl, 2,2-dimethylpropyl, 1-propylbutyl, 1-ethylpentyl, prop-1-enyl, prop-2-enyl, but-1-enyl, but-2-enyl, but-3-enyl, -(CH₂)₂-CO₂-methyl, - (CH₂)₂-CO₂-ethyl, -CH₂-O-CH₃, -CH₂-O-(CH₂)₂-O-CH₃, -CH₂-O-phenyl, -CH₂-O-(4-chlorophenyl), -CH(CH₃)-O-phenyl, - -(CH₂)₃-O-phenyl), dimethylaminomethyl, cyclopropyl, cyclobutyl, cyclopentyl, cyclohexyl, 1-adamantyl, -CH₂-cyclopropyl, -CH₂-cyclopentyl, aryl¹, 1-naphthyl, CH₂-aryl³, -CH(C₂H₅)-aryl³, -CH=CH-aryl⁴, furan-2-yl, furan-3-yl, 5-tert-butyl-2-methyl-furan-3-yl, 2,5-dimethyl-furan-3-yl, pyrazol-4-yl, pyrazol-5-yl, 1-tert-butyl-3-methyl-pyrazol-5-yl, 1-phenyl-3-propyl-pyrazol-4-yl, isoxazol-3-yl, isoxazol-4-yl, isoxazol-5-yl, 5-methyl-4-(2-chlorophenyl)-isoxazol-3-yl, 5-methyl-3-(2-chloro-6-fluorophenyl)-isoxazol-4-yl, pyridin-2-yl, pyridin-3-yl, pyridin-4-yl, 2-chloropyridin-3-yl, 6-chloropyridin-3-yl, 2-(CH₃-S-)-pyridin-3-yl, 2-methyl-6-trifluoromethyl-pyridin-3-yl, 4,7,7-trimethyloxabicyclo[2.2.1]heptan-3-one or-CH₂-imidazolidine-2,4-dione;
R²¹ denotes aryl⁵, thien-2-yl, thien-3-yl or 5-chloro-thien-2-yl or represents 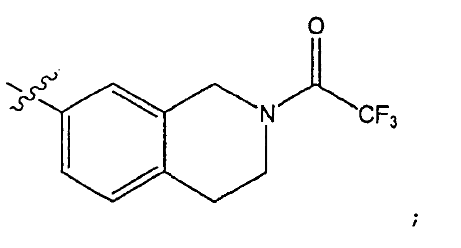
aryl¹ represents 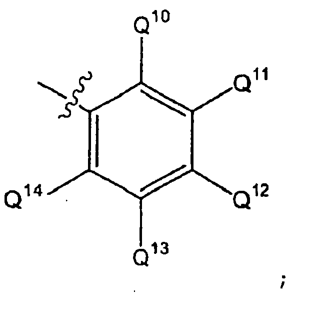
aryl³ represents 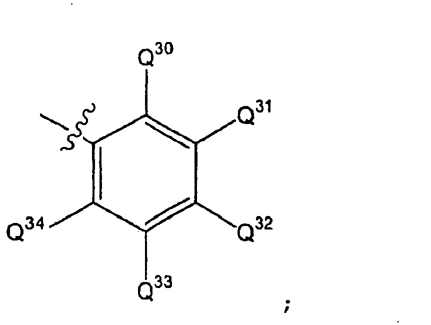
aryl⁴ represents 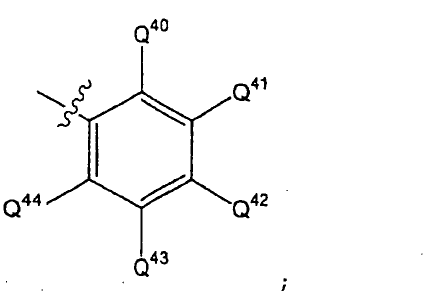
aryl⁵ represents 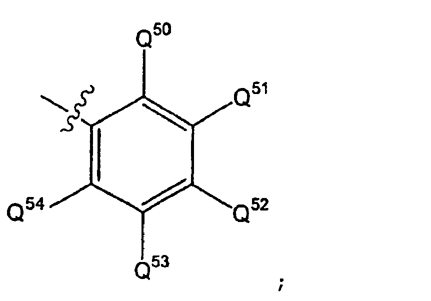
Q¹⁰, Q¹¹, Q¹², Q¹³ and Q¹⁴ independently of one another denote H, F, Cl, Br, -NO₂, methyl, trifluoromethyl, ethyl, n-propyl, 1-methylethyl, O-CH₃, O-CF₃, -O-C₂H₅, S- CH₃ or S-CF₃;
Q³⁰, Q³¹, Q³², Q³³ and Q³⁴ independently of one another denote H, Cl or O-CH₃;
Q⁴⁰, Q⁴¹, Q⁴², Q⁴³ and Q⁴⁴ independently of one another denote H, Cl, CH₃ or CF₃;
Q⁵⁰, Q⁵¹, Q⁵², Q⁵³ and Q⁵⁴ independently of one another denote H, F, Cl, Br, methyl, ethyl, n-propyl, 1-methylethyl, O-CH₃, O-CF₃ O-CH₂CH₃, O-CH₂CH₂CH₃, 0-CH₂CH₂CH₂HC₃.

5. Substituted 2-pyrrolidin-2-yl-1H-indole derivatives according to claim 4, **characterized in that**
R¹ denotes H, methyl, but-2-yne or diethylaminoethyl;
R² denotes C(=O) -R²⁰ or SO₂-R²¹;
R³ denotes phenyl;
R⁴ denotes 5-H or 5-Cl;
R²⁰ denotes n-propyl, n-butyl, n-pentyl, 1-methylbutyl, 2,2-dimethylpropyl, 1-propylbutyl, 1-ethylpentyl, but-3-enyl, -(CH₂)₂-CO₂-methyl, -(CH₂)₂-CO₂-ethyl, -CH₂-O-CH₃, -CH₂-O- (CH₂)₂-O-CH₃, -CH₂-O-phenyl, -CH₂-O-(4-chlorophenyl), -CH(CH₃)-O-phenyl, -(CH₂)₃-O-phenyl, dimethylaminomethyl, cyclopropyl, cyclobutyl, cyclohexyl, 1-adamantyl, -CH₂-cyclopentyl, 4-fluorophenyl, 4-chlorophenyl, 2-bromophenyl, 3-bromophenyl, 2,3-difluorophenyl, 3-fluoro-4-trifluoromethylphenyl, 3-fluoro-6-trifluoromethylphenyl, 3-fluoro-4-methylphenyl, 3,5-difluorophenyl, 2-chloro-4-nitrophenyl, 2-chloro-5-trifluoromethylphenyl, 4-bromo-3-methylphenyl, 2,3-difluoro-4-methylphenyl, 2,6-difluoro-3-methylphenyl, 2,3,4,5,6-pentafluorophenyl, 4-nitrophenyl, 4-methyl-3-nitrophenyl, 3-methylphenyl, 4-methylphenyl, 2,3-dimethylphenyl, 3-trifluoromethylphenyl, 2,5-trifluoromethylphenyl, 3,5-trifluoromethylphenyl, 4-ethylphenyl, 4-n-propylphenyl, 3-methoxyphenyl, 3,5-dimethoxyphenyl, 3-trifluoromethoxyphenyl, 4-trifluoromethoxyphenyl, 2-ethoxyphenyl, 4-ethoxyphenyl, 4-S-CF₃-phenyl, benzyl, 4-chlorobenzyl, 3-methoxybenzyl, 2,5-dimethoxybenzyl, -CH (C₂H₅)-phenyl, -CH=CH-(2-chlorophenyl), -CH=CH-(3-trifluoromethylphenyl), furan-2-yl, 5-tert-butyl-2-methyl-furan-3-yl, 2,5-dimethyl-furan-3-yl, 1-tert-butyl-3-methyl-pyrazol-5-yl, 1-phenyl-3-propyl-pyrazol-4-yl, isoxazol-5-yl, 5-methyl-4-(2-chlorophenyl)-isoxazol-3-yl, 5-methyl-3-(2-chloro-6-fluorophenyl)-isoxazol-4-yl, pyridin-2-yl, 2-chloropyridin-3-yl, 6-chloropyridin-3-yl, 2-(CH₃-S-)-pyridin-3-yl, 2-methyl-6-trifluoromethyl-pyridin-3-yl, 4,7,7-trimethyloxabicyclo[2.2.1]heptan-3-one or -CH₂-imidazolidine-2,4-dione; and
R²¹ denotes 3-chloro-4-fluorophenyl, 2-chlorophenyl, 4-chlorophenyl, 3-chloro-2-methylphenyl, 3-bromophenyl, 2,3,5,6-tetramethylphenyl, 4-n-butylphenyl, 4-methoxyphenyl, 2,5-dimethoxyphenyl, 4-trifluoromethoxyphenyl, 4-(n-butoxy)-phenyl, thien-2-yl, 5-chlorothien-2-yl or represents

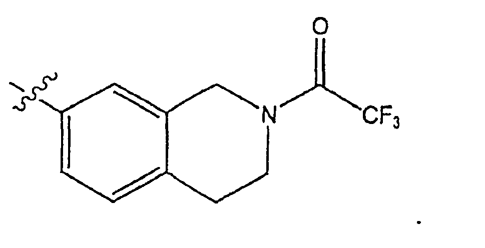

6. Substituted 2-pyrrolidin-2-yl-1H-indole derivative according to claim 1, chosen from the group which includes:
{2-[5-chloro-1-(2-diethylamino-ethyl)-3-phenyl-1H-indol-2-yl]-pyrrolidin-1-yl}-furan-2-yl-methanone;
4-{2-[5-chloro-1-(2-diethylamino-ethyl)-3-phenyl-1H-indol-2-yl]-pyrrolidin-1-yl}-4-oxo-butyric acid ethyl ester;
{2-[5-chloro-1-(2-diethylamino-ethyl)-3-phenyl-1H-indol-2-yl]-pyrrolidin-1-yl}-(4-fluoro-phenyl)-methanone;
{2-[5-chloro-1-(2-diethylamino-ethyl)-3-phenyl-1H-indol-2-yl]-pyrrolidin-l-yl}-cyclobutyl-methanone;
{2-[5-chloro-1-(2-diethylamino-ethyl)-3-phenyl-1H-indol-2-yl]-pyrrolidin-1-yl}-(6-chloro-pyridin-3-yl)-methanone;
1-{2-[5-chloro-1-(2-diethylamino-ethyl)-3-phenyl-1H-indol-2-yl]-pyrrolidin-1-yl}-hexan-1-one ;
{2-[5-chloro-1-(2-diethylamino-ethyl)-3-phenyl-1H-indol-2-yl]-pyrrolidin-1-yl}-(3,5-dimethoxy-phenyl)-methanone;
{2-[5-chloro-1-(2-diethylamino-ethyl)-3-phenyl-1H-indol-2-yl]-pyrrolidin-1-yl}-(4-ethoxy-phenyl)-methanone;
1-[2-(5-chloro-1-methyl-3-phenyl-1H-indol-2-yl)-pyrrolidin-1-yl]-2-(2-methoxy-ethoxy)-ethanone;
[2-(5-chloro-1-methyl-3-phenyl-1H-indol-2-yl)-pyrrolidin-1-yl]-cyclopropyl-methanone; and
1-[2-(1-but-2-ynyl-5-chloro-3-phenyl-1H-indol-2-yl)-pyrrolidin-1-yl]-2-methoxy-ethanone.

7. Process for the preparation of a substituted 2-pyrrolidin-2-yl-1H-indole derivative of the general formula (I) 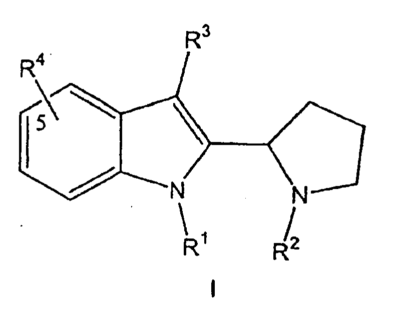 wherein
R¹ denotes H, alkyl, cycloalkyl, alkyl-cycloalkyl, alkyl-aryl or alkyl-heterocyclyl;
R² denotes alkyl, cycloalkyl, alkyl-cycloalkyl, alkyl-aryl, alkyl-heterocyclyl, C(=O)-R²⁰ or SO₂-R²¹;
R³ denotes alkyl, aryl or heterocyclyl;
R⁴ denotes H, F, Cl, Br, I, -CN, OR⁴⁰, alkyl, cycloalkyl or NO₂;
R²⁰ denotes alkyl, cycloalkyl, alkyl-cycloalkyl, aryl, alkyl-aryl, heterocyclyl, alkyl-heterocyclyl, OR²⁰⁰ or NHR²⁰¹;
R²¹ denotes alkyl, aryl, alkyl-aryl, heterocyclyl or alkyl-heterocyclyl;
R⁴⁰ denotes H, alkyl, cycloalkyl, alkyl-cycloalkyl, aryl or alkyl-aryl;
R²⁰⁰ denotes alkyl, cycloalkyl, alkyl-cycloalkyl, aryl, alkyl-aryl, heterocyclyl or alkyl-heterocyclyl; and
R²⁰¹ denotes alkyl, aryl, alkyl-aryl, heterocyclyl or alkyl-heterocyclyl;
wherein
"cycloalkyl" denotes an alicyclic saturated or unsaturated hydrocarbon radical, wherein the radical can be unsubstituted or monosubstituted or polysubstituted by identical or different substituents and optionally benzo-fused and also includes bi-, tri- or polycyclic alicyclic radicals,
"alkyl" includes acyclic saturated or unsaturated hydrocarbon radicals ("alkanyls", "alkenyls" and "alkynyls") which can be branched or straight-chain and unsubstituted or monosubstituted or polysubstituted by identical or different substituents;
and "heterocyclyl" represents a monocyclic or polycyclic organic radical in which at least one ring contains 1 heteroatom or 2, 3, 4 or 5 identical or different heteroatoms which is/are chosen from the group which contains N, O and S, wherein the radical is saturated or unsaturated and unsubstituted or monosubstituted or polysubstituted by identical or different substituents,
**characterized in that**
in a process step (A) a 2-pyrrolidin-2-yl-1H-indole derivative of the general formula (II) 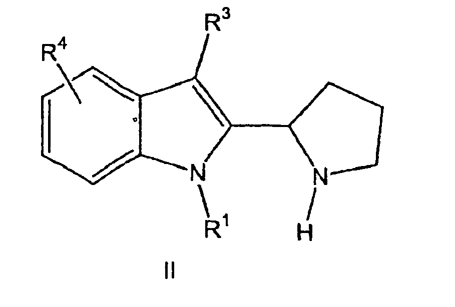 wherein
R¹, R³ and R³ are as defined above in this claim; is reacted with a compound of the general formula (III)
R²-Hal III
wherein
R² is as defined above in this claim; and
Hal denotes Cl or Br.

8. Process for the preparation of a substituted 2-pyrrolidin-2-yl-1H-indole derivative of the general formula (I) 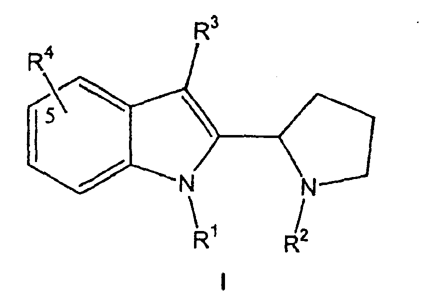 wherein
R¹ and R² denote H;
R³ denotes alkyl, aryl or heterocyclyl;
R⁴ denotes H, F, Cl, Br, 1, -CN, OR⁴⁰, alkyl, cycloalkyl or NO₂; and
R⁴⁰ denotes H, alkyl, cycloalkyl, alkyl-cycloalkyl, aryl or alkyl-aryl;
wherein
"cycloalkyl" denotes an alicyclic saturated or unsaturated hydrocarbon radical, wherein the radical can be unsubstituted or monosubstituted or polysubstituted by identical or different substituents and optionally benzo-fused and also includes bi-, tri- or polycyclic alicyclic radicals,
"alkyl" includes acyclic saturated or unsaturated hydrocarbon radicals ("alkanyls", "alkenyls" and "alkynyls") which can be branched or straight-chain and unsubstituted or monosubstituted or polysubstituted by identical or different substituents;
and "heterocyclyl" represents a monocyclic or polycyclic organic radical in which at least one ring contains 1 heteroatom or 2, 3, 4 or 5 identical or different heteroatoms which is/are chosen from the group which contains N, O and S, wherein the radical is saturated or unsaturated and unsubstituted or monosubstituted or polysubstituted by identical or different substituents,
**characterized in that**
in a process step (B) a 2-pyrrolidin-2-yl-1H-indole derivative of the general formula (IV) 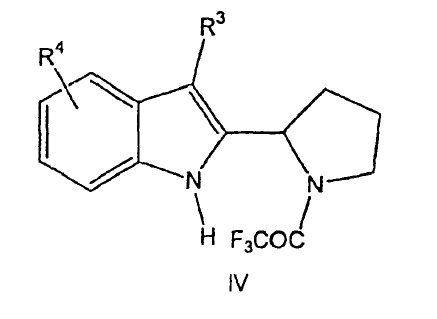 wherein
R³ and R⁴ are as defined above in this claim; is reacted with potassium carbonate or sodium hydroxide.

9. Process for the preparation of a substituted 2-pyrrolidin-2-yl-1H-indole derivative of the general formula (I) 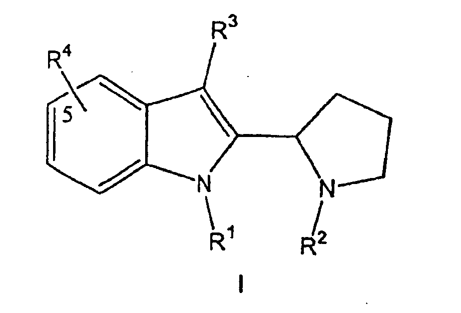 wherein
R¹ denotes alkyl, cycloalkyl, alkyl-cycloalkyl, alkyl-aryl or alkyl-heterocyclyl;
R² denotes H;
R³ denotes alkyl, aryl or heterocyclyl;
R⁴ denotes H, F, Cl, Br, I, -CN, OR⁴⁰, alkyl, cycloalkyl or NO₂; and
R⁴⁰ denotes H, alkyl, cycloalkyl, alkyl-cycloalkyl, aryl or alkyl-aryl;
wherein
"cycloalkyl" denotes an alicyclic saturated or unsaturated hydrocarbon radical, wherein the radical can be unsubstituted or monosubstituted or polysubstituted by identical or different substituents and optionally benzo-fused and also includes bi-, tri- or polycyclic alicyclic radicals,
"alkyl" includes acyclic saturated or unsaturated hydrocarbon radicals ("alkanyls", "alkenyls" and "alkynyls") which can be branched or straight-chain and unsubstituted or monosubstituted or polysubstituted by identical or different substituents;
and "heterocyclyl" represents a monocyclic or polycyclic organic radical in which at least one ring contains 1 heteroatom or 2, 3, 4 or 5 identical or different heteroatoms which is/are chosen from the group which contains N, O and S, wherein the radical is saturated or unsaturated and unsubstituted or monosubstituted or polysubstituted by identical or different substituents,
**characterized in that**
in a process step (C) a 2-pyrrolidin-2-yl-1H-indole derivative of the general formula (V) 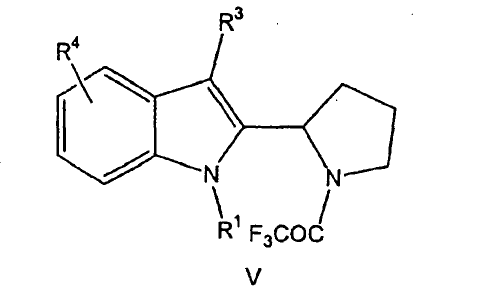 wherein
R¹, R³ and R⁴ are as defined above in this claim;
is reacted with potassium carbonate or sodium hydroxide.

10. Process for the preparation of a 2-pyrrolidin-2-yl-1H-indole derivative of the general formula (V) 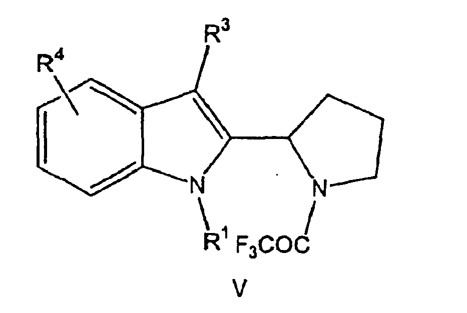 wherein
R¹ denotes alkyl, cycloalkyl, alkyl-cycloalkyl, alkyl-aryl or alkyl-heterocyclyl;
R³ denotes alkyl, aryl or heterocyclyl;
R⁴ denotes H, F, Cl, Br, I, -CN, OR⁴⁰, alkyl, cycloalkyl or NO₂;
R⁴⁰ denotes H, alkyl, cycloalkyl, alkyl-cycloalkyl, aryl or alkyl-aryl;
wherein
"cycloalkyl" denotes an alicyclic saturated or unsaturated hydrocarbon radical, wherein the radical can be unsubstituted or monosubstituted or polysubstituted by identical or different substituents and optionally benzo-fused and also includes bi-, tri- or polycyclic alicyclic radicals,
"alkyl" includes acyclic saturated or unsaturated hydrocarbon radicals ("alkanyls", "alkenyls" and "alkynyls") which can be branched or straight-chain and unsubstituted or monosubstituted or polysubstituted by identical or different substituents;
and "heterocyclyl" represents a monocyclic or polycyclic organic radical in which at least one ring contains 1 heteroatom or 2, 3, 4 or 5 identical or different heteroatoms which is/are chosen from the group which contains N, O and S, wherein the radical is saturated or unsaturated and unsubstituted or monosubstituted or polysubstituted by identical or different substituents,
**characterized in that**
in a process step (D) a 2-pyrrolidin-2-yl-1H-indole derivative of the general formula (IV) 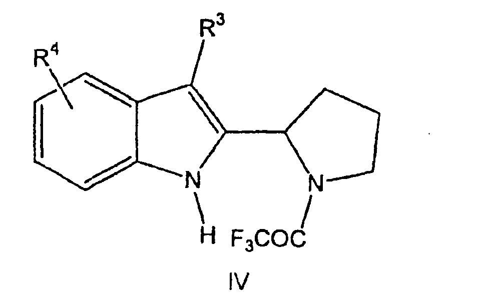 wherein
R³ and R⁴ are as defined above in this claim;
is reacted with a compound of the general formula (VI)
R¹-Hal VI
wherein
R¹ is as defined above in this claim; and
Hal denotes Cl, Br or I.

11. Process according to claim 9, **characterized in that** the process step (D) according to claim 10 is carried out before the process step (C).

12. Process according to claim 8 or 11, **characterized in that** the following process steps are carried out before the process step (B) or the process step (D):
(E) Reaction of N-trifluoroacetylproline alkyl esters (VII) 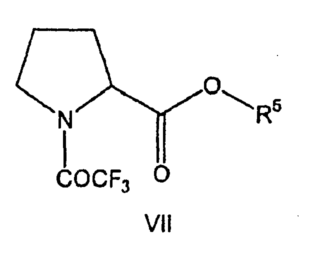 wherein
R⁵ denotes alkyl;
with an aniline derivative of the general formula (VIII) 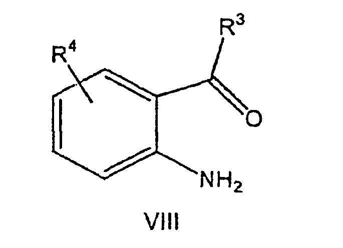 wherein
R³ denotes alkyl, aryl or heterocyclyl;
R⁴ denotes H, F, Cl, Br, I, -CN, OR⁴⁰, alkyl, cycloalkyl or NO₂; and
R⁴ denotes H, alkyl, cycloalkyl, alkyl-cycloalkyl, aryl or alkyl-aryl;
and
(F) subsequent conversion of the product (IX) from process step (E) 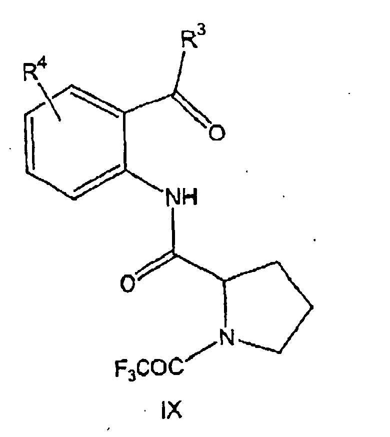 wherein
R³ and R⁴ are as defined above in this claim;
by means of McMurry coupling into a 2-pyrrolidin-2-yl-1H-indole derivative of the general formula (IV) 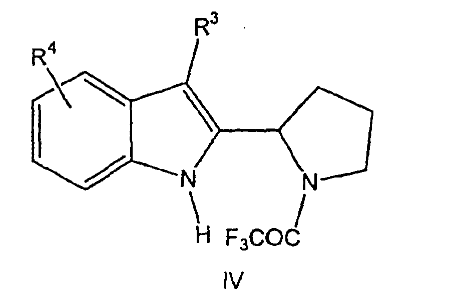 wherein
R³ and R⁴ are as defined in this claim.

13. Medicament comprising at least one compound of the general formula (I) 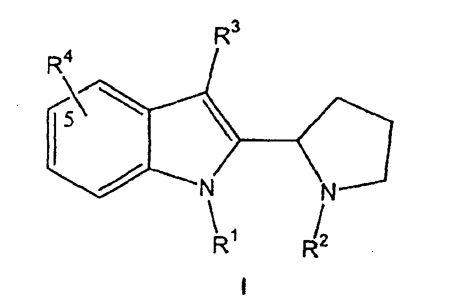 in the form of its racemate, its pure stereoisomers, or in the form of mixtures of the stereoisomers, in any desired mixture ratio; in the form shown or in the form of its acids or its bases or in the form of its salts, or in the form of its solvates,
wherein
R¹ denotes H, alkyl, cycloalkyl, alkyl-cycloalkyl, alkyl-aryl or alkyl-heterocyclyl;
R² denotes H, alkyl, cycloalkyl, alkyl-cycloalkyl, alkyl-aryl, alkyl-heterocyclyl, C(=O)-R²⁰ or SO₂-R²¹ ;
R³ denotes alkyl, aryl or heterocyclyl;
R⁴ denotes H, F, Cl, Br, I, -CN, OR⁴⁰, alkyl, cycloalkyl or NO₂;
R²⁰ denotes alkyl, cycloalkyl, alkyl-cycloalkyl, aryl, alkyl-aryl, heterocyclyl, alkyl-heterocyclyl, OR²⁰⁰ or NHR²⁰¹;
R²¹ denotes alkyl, aryl, alkyl-aryl, heterocyclyl or alkyl-heterocyclyl;
R⁴⁰ denotes H, alkyl, cycloalkyl, alkyl-cycloalkyl, aryl or alkyl-aryl;
R²⁰⁰ denotes alkyl, cycloalkyl, alkyl-cycloalkyl, aryl, alkyl-aryl, heterocyclyl or alkyl-heterocyclyl; and
R²⁰¹ denotes alkyl, aryl, alkyl-aryl, heterocyclyl or alkyl-heterocyclyl
wherein
"cycloalkyl" denotes an alicyclic saturated or unsaturated hydrocarbon radical, wherein the radical can be unsubstituted or monosubstituted or polysubstituted by identical or different substituents and optionally benzo-fused and also includes bi-, tri- or polycyclic alicyclic radicals,
"alkyl" includes acyclic saturated or unsaturated hydrocarbon radicals ("alkanyls", "alkenyls" and "alkynyls") which can be branched or straight-chain and unsubstituted or monosubstituted or polysubstituted by identical or different substituents;
and "heterocyclyl" represents a monocyclic or polycyclic organic radical in which at least one ring contains 1 heteroatom or 2, 3, 4 or 5 identical or different heteroatoms which is/are chosen from the group which contains N, O and S,
wherein the radical is saturated or unsaturated and unsubstituted or monosubstituted or polysubstituted by identical or different substituents.

14. Use of a substituted 2-pyrrolidin-2-yl-1H-indole derivative of the general formula (I) 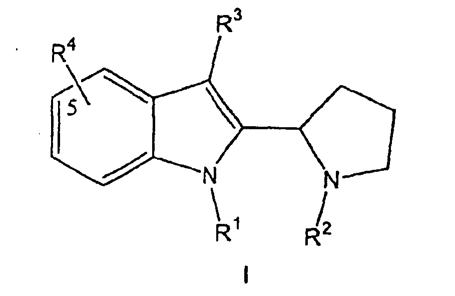 in the form of its racemate, its pure stereoisomers, or in the form of mixtures of the stereoisomers, in any desired mixture ratio; in the form shown or in the form of its acids or its bases or in the form of its salts, or in the form of its solvates,
wherein
R¹ denotes H, alkyl, cycloalkyl, alkyl-cycloalkyl, alkyl-aryl or alkyl-heterocyclyl;
R² denotes H, alkyl, cycloalkyl, alkyl-cycloalkyl, alkyl-aryl, alkyl-heterocyclyl, C(=O) -R²⁰ or SO₂-R²¹;
R³ denotes alkyl, aryl or heterocyclyl;
R⁴ denotes H, F, Cl, Br, I, -CN, OR⁴⁰, alkyl, cycloalkyl or NO₂;
R²⁰ denotes alkyl, cycloalkyl, alkyl-cycloalkyl, aryl, alkyl-aryl, heterocyclyl, alkyl-heterocyclyl, OR²⁰⁰ or NHR²⁰¹;
R²¹ denotes alkyl, aryl, alkyl-aryl, heterocyclyl or alkyl-heterocyclyl;
R⁴⁰ denotes H, alkyl, cycloalkyl, alkyl-cycloalkyl, aryl or alkyl-aryl;
R²⁰⁰ denotes alkyl, cycloalkyl, alkyl-cycloalkyl, aryl, alkyl-aryl, heterocyclyl or alkyl-heterocyclyl; and
R²⁰¹ denotes alkyl, aryl, alkyl-aryl, heterocyclyl or alkyl-heterocyclyl
wherein
"cycloalkyl" denotes an alicyclic saturated or unsaturated hydrocarbon radical, wherein the radical can be unsubstituted or monosubstituted or polysubstituted by identical or different substituents and optionally benzo-fused and also includes bi-, tri- or polycyclic alicyclic radicals,
"alkyl" includes acyclic saturated or unsaturated hydrocarbon radicals ("alkanyls", "alkenyls" and "alkynyls") which can be branched or straight-chain and unsubstituted or monosubstituted or polysubstituted by identical or different substituents;
and "heterocyclyl" represents a monocyclic or polycyclic organic radical in which at least one ring contains 1 heteroatom or 2, 3, 4 or 5 identical or different heteroatoms which is/are chosen from the group which contains N, O and S, wherein the radical is saturated or unsaturated and unsubstituted or monosubstituted or polysubstituted by identical or different substituents,
for the preparation of a medicament for inhibition of NO synthase.

15. Use of a substituted 2-pyrrolidin-2-yl-1H-indole derivative of the general formula (I) 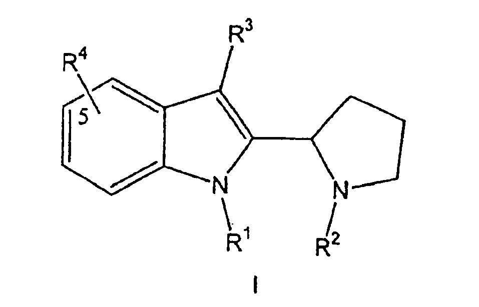 in the form of its racemates, its pure stereoisomers, or in the form of mixtures of the stereoisomers, in any desired mixture ratio; in the form shown or in the form of its acids or its bases or in the form of its salts, or in the form of its solvates,
wherein
R¹ denotes H, alkyl, cycloalkyl, alkyl-cycloalkyl, alkyl-aryl or alkyl-heterocyclyl;
R² denotes H, alkyl, cycloalkyl, alkyl-cycloalkyl, alkyl-aryl, alkyl-heterocyclyl, C(=O)-R²⁰ or SO₂-R²¹;
R³ denotes alkyl, aryl or heterocyclyl;
R⁴ denotes H, F, Cl, Br, I, -CN, OR⁴⁰, alkyl, cycloalkyl or NO₂;
R²⁰ denotes alkyl, cycloalkyl, alkyl-cycloalkyl, aryl, alkyl-aryl, heterocyclyl, alkyl-heterocyclyl, OR²⁰⁰ or NHR²⁰¹;
R²¹ denotes alkyl, aryl, alkyl-aryl, heterocyclyl or alkyl-heterocyclyl;
R⁴⁰ denotes H, alkyl, cycloalkyl, alkyl-cycloalkyl, aryl or alkyl-aryl;
R²⁰⁰ denotes alkyl, cycloalkyl, alkyl-cycloalkyl, aryl, alkyl-aryl, heterocyclyl or alkyl-heterocyclyl; and
R²⁰¹ denotes alkyl, aryl, alkyl-aryl, heterocyclyl or alkyl-heterocyclyl
wherein
"cycloalkyl" denotes an alicyclic saturated or unsaturated hydrocarbon radical, wherein the radical can be unsubstituted or monosubstituted or polysubstituted by identical or different substituents and optionally benzo-fused and also includes bi-, tri- or polycyclic alicyclic radicals,
"alkyl" includes acyclic saturated or unsaturated hydrocarbon radicals ("alkanyls", "alkenyls" and "alkynyls") which can be branched or straight-chain and unsubstituted or monosubstituted or polysubstituted by identical or different substituents;
and "heterocyclyl" represents a monocyclic or polycyclic organic radical in which at least one ring contains 1 heteroatom or 2, 3, 4 or 5 identical or different heteroatoms which is/are chosen from the group which contains N, O and S, wherein the radical is saturated or unsaturated and unsubstituted or monosubstituted or polysubstituted by identical or different substituents, for the preparation of a medicament for prophylaxis and treatment of migraine.

16. Use of a substituted 2-pyrrolidin-2-yl-1H-indole derivative of the general formula (I) 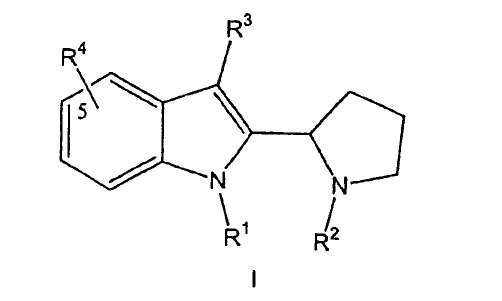 in the form of its racemates, its pure stereoisomers, or in the form of mixtures of the stereoisomers, in any desired mixture ratio; in the form shown or in the form of its acids or its bases or in the form of its salts, or in the form of its solvates,
wherein
R¹ denotes H, alkyl, cycloalkyl, alkyl-cycloalkyl, alkyl-aryl or alkyl-heterocyclyl;
R² denotes H, alkyl, cycloalkyl, alkyl-cycloalkyl, alkyl-aryl, alkyl-heterocyclyl, C(=O)-R²⁰ or SO₂-R²¹;
R³ denotes alkyl, aryl or heterocyclyl;
R⁴ denotes H, F, Cl, Br, I, -CN, OR⁴⁰, alkyl, cycloalkyl or NO₂;
R²⁰ denotes alkyl, cycloalkyl, alkyl-cycloalkyl, aryl, alkyl-aryl, heterocyclyl, alkyl-heterocyclyl, OR²⁰⁰ or NHR²⁰¹;
R²¹ denotes alkyl, aryl, alkyl-aryl, heterocyclyl or alkyl-heterocyclyl;
R⁴⁰ denotes H, alkyl, cycloalkyl, alkyl-cycloalkyl, aryl or alkyl-aryl;
R²⁰⁰ denotes alkyl, cycloalkyl, alkyl-cycloalkyl, aryl, alkyl-aryl, heterocyclyl or alkyl-heterocyclyl; and
R²⁰¹ denotes alkyl, aryl, alkyl-aryl, heterocyclyl or alkyl-heterocyclyl;
wherein
"cycloalkyl" denotes an alicyclic saturated or unsaturated hydrocarbon radical, wherein the radical can be unsubstituted or monosubstituted or polysubstituted by identical or different substituents and optionally benzo-fused and also includes bi-, tri- or polycyclic alicyclic radicals,
"alkyl" includes acyclic saturated or unsaturated hydrocarbon radicals ("alkanyls", "alkenyls" and "alkynyls") which can be branched or straight-chain and unsubstituted or monosubstituted or polysubstituted by identical or different substituents; and "heterocyclyl" represents a monocyclic or polycyclic organic radical in which at least one ring contains 1 heteroatom or 2, 3, 4 or 5 identical or different heteroatoms which is/are chosen from the group which contains N, O and S, wherein the radical is saturated or unsaturated and unsubstituted or monosubstituted or polysubstituted by identical or different substituents, for the preparation of a medicament for treatment of septic shock, multiple sclerosis, Parkinson's disease, Alzheimer's disease, Huntington's disease, inflammations, inflammation pain, cerebral ischaemia, diabetes, meningitis, arteriosclerosis and/or for wound healing.

## Revendications

1. Dérivés substitués de 2-pyrrolidine-2-yl-1H-indole de formule générale (I) 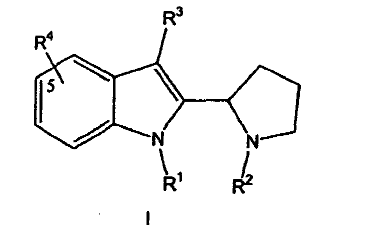 sous forme de leurs racémates, de leurs stéréoisomères purs ou sous forme de mélanges des stéréoisomères, dans un rapport de mélange quelconque ; sous la forme représentée ou sous forme de leurs acides ou de leurs bases ou sous forme de leurs sels, ou sous forme de leurs produits de solvatation,
formule dans laquelle
R¹ représente H, un reste alkyle, cycloalkyle, alkyl-cycloalkyle, alkyl-aryle ou alkyl-hétérocyclyle ;
R² représente H, un reste alkyle, cycloalkyle, alkyl-cycloalkyle, alkyl-aryle, alkyl-hétérocyclyle, C (=O) -R²⁰ ou SO₂-R²¹ ;
R³ représente un reste alkyle, aryle ou hétérocyclyle ;
R⁴ représente H, F, Cl, Br, I, -CN, OR⁴⁰, un reste alkyle, cycloalkyle ou NO₂ ;
R²⁰ représente un reste alkyle, cycloalkyle, alkyl-cycloalkyle, aryle, alkyl-aryle, hétérocyclyle, alkyl-hétérocyclyle, OR²⁰⁰ ou NHR²⁰¹;
R²¹ représente un reste alkyle, aryle, alkyl-aryle, hétérocyclyle ou alkyl-hétérocyclyle ;
R⁴⁰ représente H, un reste alkyle, cycloalkyle, alkyl-cycloalkyle, aryle ou alkyl-aryle ;
R²⁰⁰ représente un reste alkyle, cycloalkyle, alkyl-cycloalkyle, aryle, alkyl-aryle, hétérocyclyle ou alkyl-hétérocyclyle ; et
R²⁰¹ représente un reste alkyle, aryle, alkyl-aryle, hétérocyclyle ou alkyl-hétérocyclyle ;
où
le terme "cycloalkyle" désigne un reste hydrocarboné alicyclique saturé ou non saturé, le reste pouvant être non substitué ou substitué une fois ou plusieurs fois identiques ou différentes et, éventuellement, condensé au benzène et comprenant aussi des cycles aliphatiques bicycliques, tricycliques ou polycycliques,
"alkyle" désignant des restes hydrocarbonés acycliques saturés ou non saturés ("alcanyle", "alcényle" et "alcynyle") qui peuvent être ramifiés ou linéaires ainsi que non substitués ou substitués une fois ou substitués plusieurs fois identiques ou différentes ;
et "hétérocyclyle" désignant un reste organique monocyclique ou polycyclique dans lequel au moins un cycle contient un hétéroatome ou 2, 3, 4 ou 5 hétéroatomes identiques ou différents, qui est ou qui sont choisis dans le groupe comprenant N, O et S, le reste étant saturé ou non saturé et non substitué ou substitué une fois, ou substitué plusieurs fois identiques ou différentes, sous réserve d'exclusion de
la 2-(3-phényl-1H-indole-2-yl)-1-(trifluoracétyl)-pyrrolidine ;
l'ester méthylique d'acide α-méthyl-2-(1-méthyl-2-pyrrolidinyl)-indole-3-acétique ;
le 3-(1-cyanéthyl)-2-(1-méthyl-2-pyrrolidinyl)-indole ; et
le 2-(1-méthyl-2-pyrrolidinyl)-3-vinylindole.

2. Dérivés substitués de 2-pyrrolidine-2-yl-1H-indole suivant la revendication 1, **caractérisés en ce que**
R¹ représente H, un reste alkyle, cycloalkyle ou alkyl-cycloalkyle ;
R² représente un groupe C (=O) -R²⁰ ou SO₂-R²¹;
R³ représente un reste aryle ;
R⁴ représente H, F, Cl, Br, I, un reste -CN, OR⁴⁰, alkyle, cycloalkyle ou NO₂ ; chacun en position 5 du noyau d'indole ;
R²⁰ représente un reste alkyle, cycloalkyle, alkyl-cycloalkyle, aryle, alkyl-aryle, hétérocyclyle ou alkyl-hétérocyclyle ;
R²¹ représente un reste alkyle, aryle, alkyl-aryle, hétérocyclyle ou alkyl-hétérocyclyle ; et
R⁴⁰ représente H, un reste alkyle, cycloalkyle, alkyl-cycloalkyle, aryle ou alkyl-aryle.

3. Dérivés substitués de 2-pyrrolidine-2-yl-1H-indole suivant la revendication 1, **caractérisés en ce que**
R¹ représente H ou un reste alkyle ;
R² représente un groupe C (=O) -R²⁰ ou SO₂-R²¹ ;
R³ est un reste phényle ;
R⁴ représente H, F, Cl, Br ou I, chacun en position 5 du noyau d'indole ;
R²⁰ représente un reste alcanyle en C₁ à C₈, alcényle en C₂ à C₈, (alcanyle en C₁ à C₄)-CO₂-(alkyle en C₁ à C₄), (alcanyle en C₁ à C₄)-O-R²⁰², (alcanyle en C₁ à C₄) - NR²⁰³R²⁰⁴, cycloalkyle en C₃ à C₈, 1-adamantyle, 2-adamantyle, -(alkyle en C₁ à C₃)-(cycloalkyle en C₃ à C₈), aryle¹, aryle², (alcanyle en C₁ à C₄) -aryle³, (alcényle en C₂ à C₄) -aryle⁴ ; ou un reste, non substitué ou substitué, furannyle, pyrazolyle, isoxazolyle ou pyridinyle ; ou un groupe 4,7,7-triméthyloxabicyclo[2.2.1]-heptane-3-one, -CH₂-imidazolidine-2,4-dione ;
R²¹ représente un reste aryle⁵, thiényle non substitué ou substitué ou un groupe 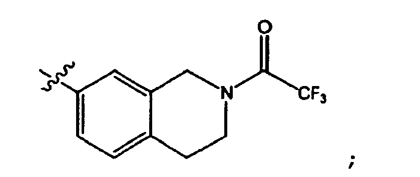
R²⁰² représente H, un reste alcanyle en C₁ à C₄, (alcanyle en C₁ à C₄) -OH, (alcanyle en C₁ à C₄)-O-(alcanyle en C₁ à C₄) ou aryle ;
R²⁰³ et R²⁰⁴ représentent, indépendamment l'un de l'autre, un reste alkyle en C₁ à C₆, ou forment avec l'atome N un hétérocyclyle saturé pentagonal, hexagonal ou heptagonal ;
aryle¹ représente 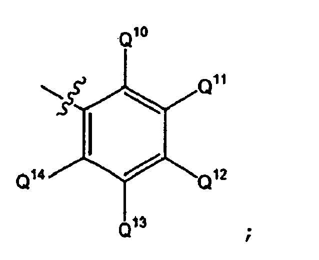
aryle² représente 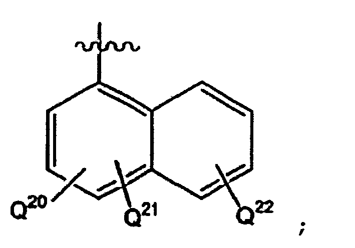
aryle³ représente 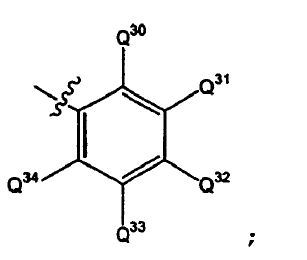
aryle⁴ représente 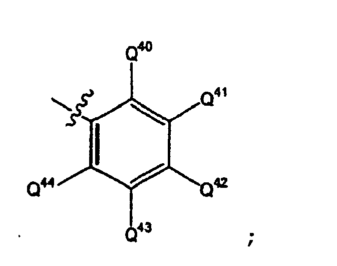
aryle⁵ représente 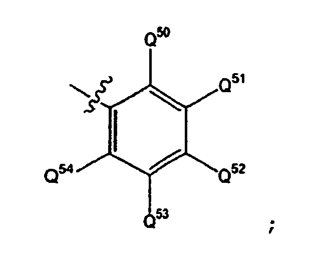
Q¹⁰, Q¹¹, Q¹², Q¹³, Q¹⁴, Q²⁰, Q²¹, Q²², Q³⁰, Q³¹, Q³², Q³³, Q³⁴, Q⁴⁰, Q⁴¹, Q⁴², Q⁴³, Q⁴⁴, Q⁵⁰, Q⁵¹, Q⁵², Q⁵³ et Q⁵⁴ représentent, indépendamment les uns des autres, H, F, Cl, Br, I, un reste -CN, -NO₂, alkyle en C₁ à C₄, OH, O-alkyle en C₁ à C₄, SH ou S-alkyle en C₁ à C₄.

4. Dérivés substitués de 2-pyrrolidine-2-yl-1H-indole suivant la revendication 3, **caractérisés en ce que**
R¹ représente H, un reste méthyle, but-2-yne ou diéthylaminoéthyle ;
R² est un groupe C (=O)-R²⁰ ou SO₂-R²¹;
R³ est un reste phényle ;
R⁴ représente 5-H ou 5-Cl ;
R²⁰ représente un reste méthyle, éthyle, n-propyle, n-butyle, 1-méthylpropyle, 2-méthylpropyle, tertiobutyle, n-pentyle, 1-méthylbutyle, 2,2-diméthylpropyle, 1-propylbutyle, 1-éthylpentyle, prop-1-ényle, prop-2-ényle, but-1-ényle, but-2-ényle, but-3-ényle, -(CH₂)₂-CO₂-méthyle, -(CH₂)₂-CO₂-éthyle, -CH₂-O-CH₃, -CH₂-O-(CH₂)₂-O-CH₃, -CH₂-O-phényle, -CH₂-O-(4-chlorophényle), -CH(CH₃)-O-phényle, -(CH₂)₃-O-phényle, diméthylaminométhyle, cyclopropyle, cyclobutyle, cyclopentyle, cyclohexyle, 1-adamantyle, -CH₂-cyclopropyle, -CH₂-cyclopentyle, aryle¹, 1-naphtyle, CH₂-aryle³, -CH(C₂H₅)-aryle³, -CH=H-aryle⁴, furanne-2-yle, furanne-3-yle, 5-tertiobutyl-2-méthyl-furanne-3-yle, 2,5-diméthyl-furanne-3-yle, pyrazole-4-yle, pyrazole-5-yle, 1-tertiobutyl-3-méthyl-pyrazole-5-yle, 1-phényl-3-propyl-pyrazole-4-yle, isoxazole-3-yle, isoxazole-4-yle, isoxazole-5-yle, 5-méthyl-4-(2-chlorophényl)-isoxazole-3-yle, 5-méthyl-3-(2-chloro-6-fluorophényl)-isoxazole-4-yle, pyridine-2-yle, pyridine-3-yle, pyridine-4-yle, 2-chloropyridine-3-yle, 6-chloropyridine-3-yle, 2-(CH₃-S-)-pyridine-3-yle, 2-méthyl-6-trifluorométhyl-pyridine-3-yle, 4,7,7-triméthyloxabicyclo-[2.2.1]heptane-3-one ou -CH₂-imidazolidine-2,4-dione ;
R²¹ représente un reste aryle⁵, thién-2-yle, thién-3-yle ou 5-chloro-thién-2-yle ou un groupe 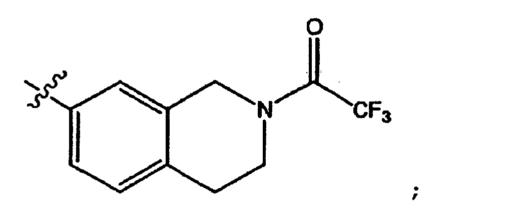
aryle¹ représente 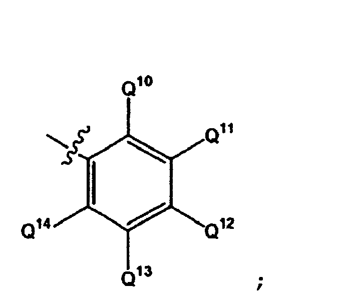
aryle³ représente 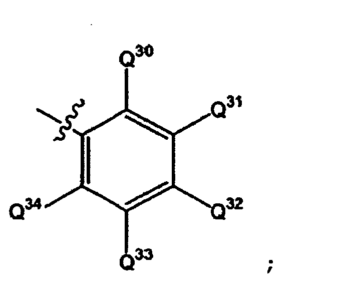
aryle⁴ représente 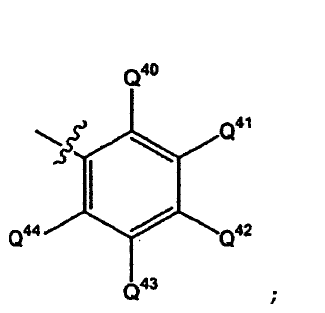
aryle⁵ représente 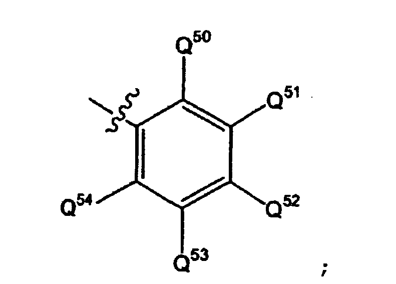
Q¹⁰, Q¹¹, Q¹², Q¹³ et Q¹⁴ représentent indépendamment les uns des autres H, F, Cl, Br, -NO₂, un reste méthyle, trifluorométhyle, éthyle, n-propyle, 1-méthyléthyle, O-CH₃, O-CF₃, -O-C₂H₅, S-CH₃ ou S-CF₃;
Q³⁰ Q³¹, Q³², Q³³ et Q³⁴ représentent indépendamment les uns des autres H, Cl ou un reste O-CH₃ ;
Q⁴⁰, Q⁴¹, Q⁴², Q⁴³ et Q⁴⁴ représentent indépendamment les uns des autres H, Cl, CH₃ ou CF₃;
Q⁵⁰ Q⁵¹, Q⁵², Q⁵³ et Q⁵⁴ représentent indépendamment les uns des autres H, F, Cl, Br, un reste méthyle, éthyle, n-propyle, 1-méthyléthyle, O-CH₃, O-CF₃, O-CH₂CH₃, O-CH₂CH₂CH₃, O-CH₂CH₂CH₂CH₃.

5. Dérivés substitués de 2-pyrrolidine-2-yl-1H-indole suivant la revendication 4, **caractérisés en ce que**
R¹ représente H, un reste méthyle, but-2-yne ou diéthylaminoéthyle ;
R² est un groupe C(=O)-R²⁰ ou SO₂-R²¹ ;
R³ est un reste phényle ;
R⁴ représente 5-H ou 5-Cl ;
R²⁰ représente un reste n-propyle, n-butyle, n-pentyle, 1-méthylbutyle, 2,2-diméthylpropyle, 1-propylbutyle, 1-éthylpentyle, but-3-ényle, -(CH₂)₂-CO₂-méthyle, -(CH₂)₂-CO₂-éthyle, -CH₂-O-CH₃, -CH₂-O- (CH₂)₂-O-CH₃, -CH₂-O-phényle, -CH₂-O- (4-chlorophényle), -CH (CH₃) -O-phényle, -(CH₂)₃-O-phényle, diméthylaminométhyle, cyclopropyle, cyclobutyle, cyclohexyle, 1-adamantyle, -CH₂-cyclopentyle, 4-fluorophényle, 4-chlorophényle, 2-bromophényle, 3-bromophényle, 2,3-difluorophényle, 3-fluoro-4-trifluorométhylphényle, 3-fluoro-6-trifluorométhylphényle, 3-fluoro-4-méthylphényle, 3,5-difluorophényle, 2-chloro-4-nitrophényle, 2-chloro-5-trifluorométhylphényle, 4-bromo-3-méthylphényle, 2,3-difluoro-4-méthylphényle, 2,6-difluoro-3-méthylphényle, 2,3,4,5,6-pentafluorophényle, 4-nitrophényle, 4-méthyl-3-nitrophényle, 3-méthylphényle, 4-méthylphényle, 2,3-diméthylphényle, 3-trifluorométhylphényle, 2, 5-trifluorométhylphényle, 3, 5-trifluorométhylphényle, 4-éthylphényle, 4-n-propylphényle, 3-méthoxyphényle, 3,5-diméthoxyphényle, 3-trifluorométhoxyphényle, 4-trifluorométhoxyphényle, 2-éthoxyphényle, 4-éthoxyphényle, 4-S-CF₃-phényle, benzyle, 4-chlorobenzyle, 3-méthoxybenzyle, 2,5-diméthoxybenzyle, -CH (C₂H₅) -phényle, -CH=CH-(2-chlorophényle), -CH=CH- (3-trifluorométhylphényle), furanne-2-yle, 5-tertiobutyl-2-méthyl-furanne-3-yle, 2,5-diméthyl-furanne-3-yle, 1-tertiobutyl-3-méthyl-pyrazole-5-yle, 1-phényl-3-propyl-pyrazole-4-yle, isoxazole-5-yle, 5-méthyl-4-(2-chlorophényl)-isoxazole-3-yle, 5-méthyl-3-(2-chloro-6-fluorophényl)-isoxazole-4-yle, pyridine-2-yle, 2-chloropyridine-3-yle, 6-chloropyridine-3-yle, 2-(CH₃-S-)-pyridine-3-yle, 2-méthyl-6-trifluorométhyl-pyridine-3-yle, 4,7,7-triméthyloxabicyclo[2.2.1]heptane-3-one ou -CH₂-imidazolidine-2,4-dione ; et
R²¹ représente un reste 3-chloro-4-fluorophényle, 2-chlorophényle, 4-chlorophényle, 3-chloro-2-méthylphényle, 3-bromophényle, 2,3,5,6-tétraméthylphényle, 4-n-butylphényle, 4-méthoxyphényle, 2,5-diméthoxyphényle, 4-trifluoro-méthoxyphényle, 4-(n-butoxy)-phényle, thién-2-yle, 5-chloro-thién-2-yle ou un groupe

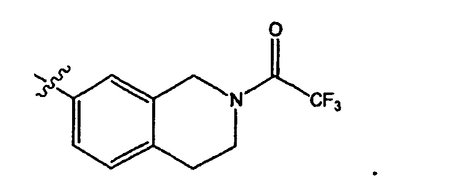

6. Dérivé substitué de 2-pyrrolidine-2-yl-1H-indole suivant la revendication 1, choisi dans le groupe qui comprend les dérivés suivants :
{2- [5-chloro-1- (2-diéthylamino-éthyl) -3-phényl-1H-indole-2-yl]-pyrrolidine-1-yl}-furanne-2-yl-méthanone ;
ester éthylique d'acide 4-{2-[5-chloro-1-(2-diéthylamino-éthyl)-3-phényl-1H-indole-2-yl]-pyrrolidine-1-yl}-4-oxo-butyrique ;
{2-[5-chloro-1-(2-diéthylamino-éthyl)-3-phényl-1H-indole-2-yl]-pyrrolidine-1-yl}-(4-fluoro-phényl)-méthanone ;
{2-[5-chloro-1-(2-diéthylamino-éthyl)-3-phényl-1H-indole-2-yl]-pyrrolidine-1-yl}-cyclobutyl-méthanone ;
{2-[5-chloro-1-(2-diéthylamino-éthyl)-3-phényl-1H-indole-2-yl]-pyrrolidine-1-yl}-(6-chloro-pyridine-3-yl)-méthanone;
1-{2-[5-chloro-1-(2-diéthylamino-éthyl)-3-phényl-1H-indole-2-yl]-pyrrolidine-1-yl}-hexane-1-one ;
{2- [5-chloro-1-(2-diéthylamino-éthyl)-3-phényl-1H-indole-2-yl]-pyrrolidine-1-yl}-(3,5-diméthoxy-phényl)-méthanone ;
{2-[5-chloro-1-(2-diéthylamino-éthyl)-3-phényl-1H-indole-2-yl]-pyrrolidine-1-yl}-(4-éthoxy-phényl)-méthanone;
1-[2-(5-chloro-1-méthyl-3-phényl-1H-indole-2-yl)-pyrrolidine-1-yl]-2-(2-méthoxy-éthoxy)-éthanone ;
[2-(5-chloro-1-méthyl-3-phényl-1H-indole-2-yl) -pyrrolidine-1-yl]-cyclopropyl-méthanone ; et
1-[2-(1-but-2-ynyl-5-chloro-3-phényl-1H-indole-2-yl)-pyrrolidine-1-yl]-2-méthoxy-éthanone.

7. Procédé de production d'un dérivé substitué de 2-pyrrolidine-2-yl-1H-indole de formule générale (I) 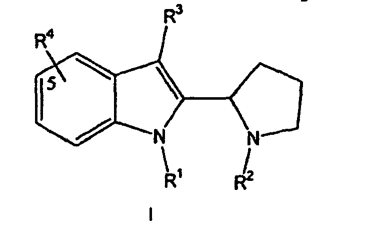 dans laquelle
R¹ représente H, un reste alkyle, cycloalkyle, alkyl-cycloalkyle, alkyl-aryle ou alkyl-hétérocyclyle ;
R² représente un reste alkyle, cycloalkyle, alkyl-cycloalkyle, alkyl-aryle, alkyl-hétérocyclyle, C(=O)-R²⁰ ou SO₂-R²¹ ;
R³ représente un reste alkyle, aryle ou hétérocyclyle ;
R⁴ représente H, F, Cl, Br, I, -CN, OR⁴⁰, un reste alkyle, cycloalkyle ou NO₂ ;
R²⁰ représente un reste alkyle, cycloalkyle, alkyl-cycloalkyle, aryle, alkyl-aryle, hétérocyclyle, alkyl-hétérocyclyle, OR²⁰⁰ ou NHR²⁰¹;
R²¹ représente un reste alkyle, aryle, alkyl-aryle, hétérocyclyle ou alkyl-hétérocyclyle ;
R⁴⁰ représente H, un reste alkyle, cycloalkyle, alkyl-cycloalkyle, aryle ou alkyl-aryle ;
R²⁰⁰ représente un reste alkyle, cycloalkyle, alkyl-cycloalkyle, aryle, alkyl-aryle, hétérocyclyle ou alkyl-hétérocyclyle ; et
R²⁰¹ représente un reste alkyle, aryle, alkyl-aryle, hétérocyclyle ou alkyl-hétérocyclyle ;
où
le terme "cycloalkyle" désigne un reste hydrocarboné alicyclique saturé ou non saturé, le reste pouvant être non substitué ou substitué une fois ou plusieurs fois identiques ou différentes et, éventuellement, condensé au benzène et comprenant aussi des cycles aliphatiques bicycliques, tricycliques ou polycycliques,
"alkyle" désignant des restes hydrocarbonés acycliques saturés ou non saturés ("alcanyle", "alcényle" et "alcynyle") qui peuvent être ramifiés ou linéaires ainsi que non substitués ou substitués une fois ou substitués plusieurs fois identiques ou différentes ;
et "hétérocyclyle" désignant un reste organique monocyclique ou polycyclique dans lequel au moins un cycle contient un hétéroatome ou 2, 3, 4 ou 5 hétéroatomes identiques ou différents, qui est ou qui sont choisis dans le groupe comprenant N, O et S, le reste étant saturé ou non saturé et non substitué ou substitué une fois, ou substitué plusieurs fois identiques ou différentes,
**caractérisé en ce que**
dans une étape (A) du procédé, on fait réagir un dérivé de 2-pyrrolidine-2-yl-1H-indole de formule générale (II) 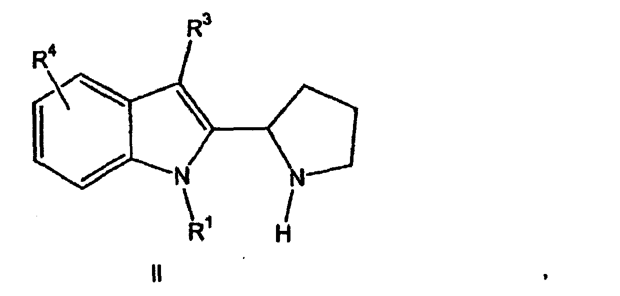 dans laquelle
R¹, R³ et R⁴ sont tels que définis ci-dessus dans cette revendication ;
avec un composé de formule générale (III)
R²-Hal III
dans laquelle
R² est tel que défini ci-dessus dans cette revendication ;
et
Hal représente Cl ou Br.

8. Procédé de production d'un dérivé substitué de 2-pyrrolidine-2-yl-1H-indole de formule générale (I) 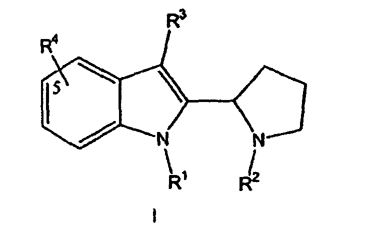 dans laquelle
R¹ et R² représentent H ;
R³ est un reste alkyle, aryle ou hétérocyclyle ;
R⁴ représente H, F, Cl, Br, I, un reste -CN, OR⁴⁰, alkyle, cycloalkyle ou NO₂; et
R⁴⁰ représente H, un reste alkyle, cycloalkyle, alkyl-cycloalkyle, aryle ou alkyl-aryle ;
où
le terme "cycloalkyle" désigne un reste hydrocarboné alicyclique saturé ou non saturé, le reste pouvant être non substitué ou substitué une fois ou plusieurs fois identiques ou différentes et, éventuellement, condensé au benzène et comprenant aussi des cycles aliphatiques bicycliques, tricycliques ou polycycliques,
"alkyle" désignant des restes hydrocarbonés acycliques saturés ou non saturés ("alcanyle", "alcényle" et "alcynyle") qui peuvent être ramifiés ou linéaires ainsi que non substitués ou substitués une fois ou substitués plusieurs fois identiques ou différentes ;
et "hétérocyclyle" désignant un reste organique monocyclique ou polycyclique dans lequel au moins un cycle contient un hétéroatome ou 2, 3, 4 ou 5 hétéroatomes identiques ou différents, qui est ou qui sont choisis dans le groupe comprenant N, O et S, le reste étant saturé ou non saturé et non substitué ou substitué une fois, ou substitué plusieurs fois identiques ou différentes,
**caractérisé en ce que**
dans une étape (B) du procédé, on fait réagir un dérivé de 2-pyrrolidine-2-yl-1H-indole de formule générale (IV) 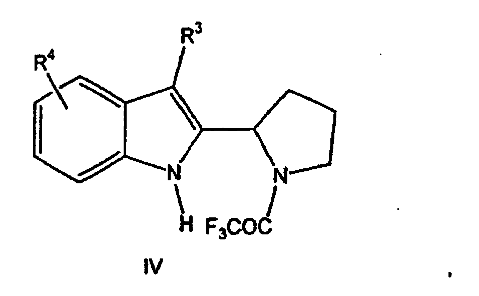 dans laquelle
R³ et R⁴ sont définis comme ci-dessus dans cette revendication ;
avec le carbonate de potassium ou l'hydroxyde de sodium.

9. Procédé de production d'un dérivé substitué de 2-pyrrolidine-2-yl-1H-indole de formule générale (I) 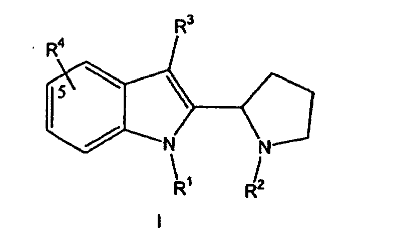 dans laquelle
R¹ désigne un reste alkyle, cycloalkyle, alkyl-cycloalkyle, alkyl-aryle ou alkyl-hétérocyclyle ;
R² représente H ;
R³ représente un reste alkyle, aryle ou hétérocyclyle ;
R⁴ représente H, F, Cl, Br, I, un reste -CN, OR⁴⁰, alkyle, cycloalkyle ou NO₂ ; et
R⁴⁰ représente H, un reste alkyle, cycloalkyle, alkyl-cycloalkyle, aryle ou alkyl-aryle ;
où
le terme "cycloalkyle" désigne un reste hydrocarboné alicyclique saturé ou non saturé, le reste pouvant être non substitué ou substitué une fois ou plusieurs fois identiques ou différentes et, éventuellement, condensé au benzène et comprenant aussi des cycles aliphatiques bicycliques, tricycliques ou polycycliques,
"alkyle" désignant des restes hydrocarbonés acycliques saturés ou non saturés ("alcanyle", "alcényle" et "alcynyle") qui peuvent être ramifiés ou linéaires ainsi que non substitués ou substitués une fois ou substitués plusieurs fois identiques ou différentes ;
et "hétérocyclyle" désignant un reste organique monocyclique ou polycyclique dans lequel au moins un cycle contient un hétéroatome ou 2, 3, 4 ou 5 hétéroatomes identiques ou différents, qui est ou qui sont choisis dans le groupe comprenant N, O et S, le reste étant saturé ou non saturé et non substitué ou substitué une fois, ou substitué plusieurs fois identiques ou différentes,
**caractérisé en ce que** dans une étape (C) du procédé, on fait réagir un dérivé de 2-pyrrolidine-2-yl-1H-indole de formule générale (V) 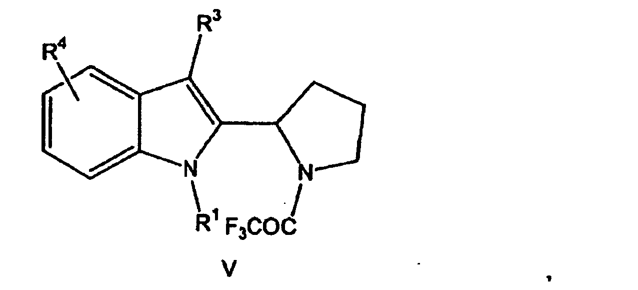 dans laquelle
R¹, R³ et R⁴ ont la définition indiquée ci-dessus dans cette revendication ;
avec le carbonate de potassium ou l'hydroxyde de sodium.

10. Procédé de production d'un dérivé substitué de 2-pyrrolidine-2-yl-1H-indole de formule générale (V) 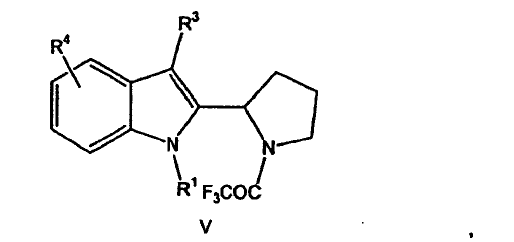 dans laquelle
R¹ représente un reste alkyle, cycloalkyle, alkyl-cycloalkyle, alkyl-aryle ou alkyl-hétérocyclyle ;
R³ représente un reste alkyle, aryle ou hétérocyclyle ;
R⁴ représente H, F, Cl, Br, I, un reste -CN, OR⁴⁰, alkyle, cycloalkyle ou NO₂ ; et
R⁴⁰ représente H, un reste alkyle, cycloalkyle, alkyl-cycloalkyle, aryle ou alkyl-aryle ;
où
le terme "cycloalkyle" désigne un reste hydrocarboné alicyclique saturé ou non saturé, le reste pouvant être non substitué ou substitué une fois ou plusieurs fois identiques ou différentes et, éventuellement, condensé au benzène et comprenant aussi des cycles aliphatiques bicycliques, tricycliques ou polycycliques,
"alkyle" désignant des restes hydrocarbonés acycliques saturés ou non saturés ("alcanyle", "alcényle" et "alcynyle") qui peuvent être ramifiés ou linéaires ainsi que non substitués ou substitués une fois ou substitués plusieurs fois identiques ou différentes ;
et "hétérocyclyle" désignant un reste organique monocyclique ou polycyclique dans lequel au moins un cycle contient un hétéroatome ou 2, 3, 4 ou 5 hétéroatomes identiques ou différents, qui est ou qui sont choisis dans le groupe comprenant N, O et S, le reste étant saturé ou non saturé et non substitué ou substitué une fois, ou substitué plusieurs fois identiques ou différentes,
**caractérisé en ce que**
dans une étape (D) du procédé, on fait réagir un dérivé de 2-pyrrolidine-2-yl-1H-indole de formule générale (IV) 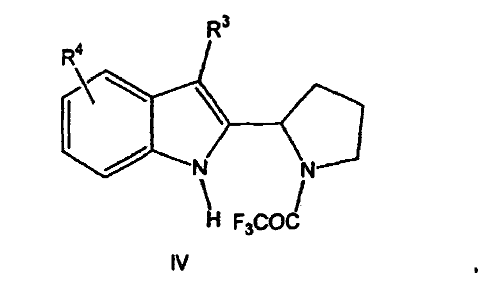 dans laquelle
R³ et R⁴ sont tels que définis ci-dessus dans cette revendication ;
avec un composé de formule générale (VI)
R¹-Hal VI
dans laquelle
R¹ est tel que défini ci-dessus dans cette revendication ;
et
Hal représente Cl, Br ou I.

11. Procédé suivant la revendication 9, **caractérisé en ce que** l'étape (D) du procédé selon la revendication 10 est mise en oeuvre avant l'étape (C) du procédé.

12. Procédé suivant la revendication 8 ou 11,
**caractérisé en ce que** les étapes suivantes sont mises en oeuvre avant l'étape (B) du procédé ou l'étape (D) du procédé:
(E) réaction d'un ester alkylique de N-trifluoracétyl-proline (VII) 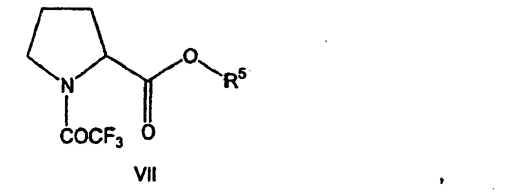 où
R⁵ désigne un reste alkyle ;
avec un dérivé d'aniline de formule générale (VIII) 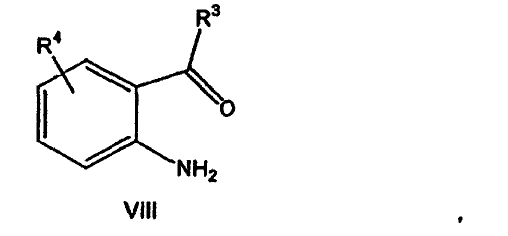 dans laquelle
R³ désigne un reste alkyle, aryle ou hétérocyclyle ;
R⁴ représente H, F, Cl, Br, I, un reste -CN, OR⁴⁰, alkyle, cycloalkyle ou NO₂ ; et
R⁴⁰ désigne H, un reste alkyle, cycloalkyle, alkyl-cycloalkyle, aryle ou alkyl-aryle ;
et
(F) transformation subséquente du produit (IX) venant de l'étape (E) du procédé 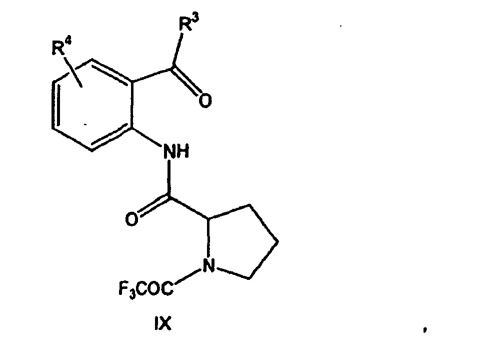
où
R³ et R⁴ sont tels que définis ci-dessus dans cette revendication ;
par couplage de McMurry en un dérivé de 2-pyrrolidine-2-yl-1H-indole de formule générale (IV) 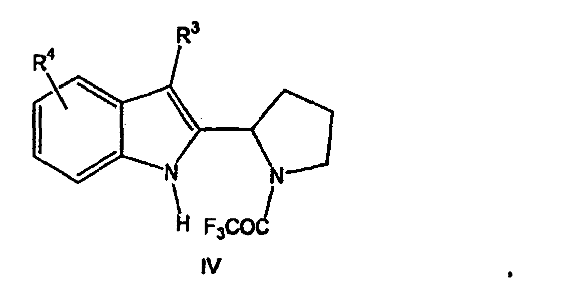 dans laquelle
R³ et R⁴ sont tels que définis ci-dessus dans cette revendication.

13. Médicament contenant au moins un composé de formule générale (I) 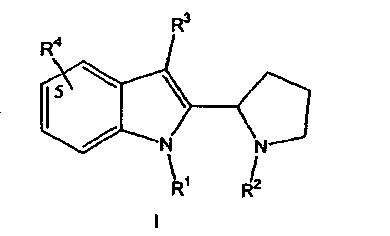 sous forme de son racémate, de ses stéréoisomères purs, ou sous forme de mélanges des stéréoisomères, dans un rapport de mélange quelconque ; sous la forme représentée ou sous forme de ses acides ou de ses bases ou sous forme de ses sels, ou sous forme de ses produits de solvatation, formule dans laquelle
R¹ représente H, un reste alkyle, cycloalkyle, alkyl-cycloalkyle, alkyl-aryle ou alkyl-hétérocyclyle ;
R² représente H, un reste alkyle, cycloalkyle, alkyl-cycloalkyle, alkyl-aryle, alkyl-hétérocyclyle, C(=O)-R²⁰ ou SO₂-R²¹ ;
R³ représente un reste alkyle, aryle ou hétérocyclyle ;
R⁴ représente H, F, Cl, Br, I, -CN, OR⁴⁰, un reste alkyle, cycloalkyle ou NO₂ ;
R²⁰ représente un reste alkyle, cycloalkyle, alkyl-cycloalkyle, aryle, alkyl-aryle, hétérocyclyle, alkyl-hétérocyclyle, OR²⁰⁰ ou NHR²⁰¹ ;
R²¹ représente un reste alkyle, aryle, alkyl-aryle, hétérocyclyle ou alkyl-hétérocyclyle ;
R⁴⁰ représente H, un reste alkyle, cycloalkyle, alkyl-cycloalkyle, aryle ou alkyl-aryle ;
R²⁰⁰ représente un reste alkyle, cycloalkyle, alkyl-cycloalkyle, aryle, alkyl-aryle, hétérocyclyle ou alkyl-hétérocyclyle ; et
R²⁰¹ représente un reste alkyle, aryle, alkyl-aryle, hétérocyclyle ou alkyl-hétérocyclyle ;
où
le terme "cycloalkyle" désigne un reste hydrocarboné alicyclique saturé ou non saturé, le reste pouvant être non substitué ou substitué une fois ou plusieurs fois identiques ou différentes et, éventuellement, condensé au benzène et comprenant aussi des cycles aliphatiques bicycliques, tricycliques ou polycycliques,
"alkyle" désignant des restes hydrocarbonés acycliques saturés ou non saturés ("alcanyle", "alcényle" et "alcynyle") qui peuvent être ramifiés ou linéaires ainsi que non substitués ou substitués une fois ou substitués plusieurs fois identiques ou différentes ;
et "hétérocyclyle" désignant un reste organique monocyclique ou polycyclique dans lequel au moins un cycle contient un hétéroatome ou 2, 3, 4 ou 5 hétéroatomes identiques ou différents, qui est ou qui sont choisis dans le groupe comprenant N, O et S, le reste étant saturé ou non saturé et non substitué ou substitué une fois, ou substitué plusieurs fois identiques ou différentes.

14. Utilisation d'un dérivé substitué de 2-pyrrolidine-2-yl-1H-indole de formule générale (I) 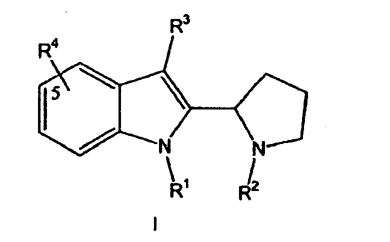 sous forme de son racémate, de ses stéréoisomères purs, ou sous forme de mélanges des stéréoisomères, dans un rapport de mélange quelconque ; sous la forme représentée ou sous forme de ses acides ou de ses bases ou sous forme de ses sels, ou sous forme de ses produits de solvatation, formule dans laquelle
R¹ représente H, un reste alkyle, cycloalkyle, alkyl-cycloalkyle, alkyl-aryle ou alkyl-hétérocyclyle ;
R² représente H, un reste alkyle, cycloalkyle, alkyl-cycloalkyle, alkyl-aryle, alkyl-hétérocyclyle, C(=O)-R²⁰ ou SO₂-R²¹ ;
R³ représente un reste alkyle, aryle ou hétérocyclyle ;
R⁴ représente H, F, Cl, Br, I, -CN, OR⁴⁰, un reste alkyle, cycloalkyle ou NO₂ ;
R²⁰ représente un reste alkyle, cycloalkyle, alkyl-cycloalkyle, aryle, alkyl-aryle, hétérocyclyle, alkyl-hétérocyclyle, OR²⁰⁰ ou NHR²⁰¹;
R²¹ représente un reste alkyle, aryle, alkyl-aryle, hétérocyclyle ou alkyl-hétérocyclyle ;
R⁴⁰ représente H, un reste alkyle, cycloalkyle, alkyl-cycloalkyle, aryle ou alkyl-aryle ;
R²⁰⁰ représente un reste alkyle, cycloalkyle, alkyl-cycloalkyle, aryle, alkyl-aryle, hétérocyclyle ou alkyl-hétérocyclyle ; et
R²⁰¹ représente un reste alkyle, aryle, alkyl-aryle, hétérocyclyle ou alkyl-hétérocyclyle ;
où
le terme "cycloalkyle" désigne un reste hydrocarboné alicyclique saturé ou non saturé, le reste pouvant être non substitué ou substitué une fois ou plusieurs fois identiques ou différentes et, éventuellement, condensé au benzène et comprenant aussi des cycles aliphatiques bicycliques, tricycliques ou polycycliques,
"alkyle" désignant des restes hydrocarbonés acycliques saturés ou non saturés ("alcanyle", "alcényle" et "alcynyle") qui peuvent être ramifiés ou linéaires ainsi que non substitués ou substitués une fois ou substitués plusieurs fois identiques ou différentes ;
et "hétérocyclyle" désignant un reste organique monocyclique ou polycyclique dans lequel au moins un cycle contient un hétéroatome ou 2, 3, 4 ou 5 hétéroatomes identiques ou différents, qui est ou qui sont choisis dans le groupe comprenant N, O et S, le reste étant saturé ou non saturé et non substitué ou substitué une fois, ou substitué plusieurs fois identiques ou différentes ; pour la préparation d'un remède destiné à inhiber la NO-synthase.

15. Utilisation d'un dérivé substitué de 2-pyrrolidine-2-yl-1H-indole de formule générale (I) 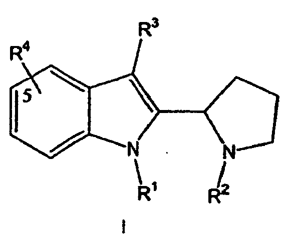 sous forme de son racémate, de ses stéréoisomères purs, ou sous forme de mélanges des stéréoisomères, dans un rapport de mélange quelconque ; sous la forme représentée ou sous forme de ses acides ou de ses bases ou sous forme de ses sels, ou sous forme de ses produits de solvatation, formule dans laquelle
R¹ représente H, un reste alkyle, cycloalkyle, alkyl-cycloalkyle, alkyl-aryle ou alkyl-hétérocyclyle ;
R² représente H, un reste alkyle, cycloalkyle, alkyl-cycloalkyle, alkyl-aryle, alkyl-hétérocyclyle, C(=O)-R²⁰ ou SO₂-R²¹;
R³ représente un reste alkyle, aryle ou hétérocyclyle ;
R⁴ représente H, F, Cl, Br, I, -CN, OR⁴⁰, un reste alkyle, cycloalkyle ou NO₂;
R²⁰ représente un reste alkyle, cycloalkyle, alkyl-cycloalkyle, aryle, alkyl-aryle, hétérocyclyle, alkyl-hétérocyclyle, OR²⁰⁰ ou NHR²⁰¹;
R²¹ représente un reste alkyle, aryle, alkyl-aryle, hétérocyclyle ou alkyl-hétérocyclyle ;
R⁴⁰ représente H, un reste alkyle, cycloalkyle, alkyl-cycloalkyle, aryle ou alkyl-aryle ;
R²⁰⁰ représente un reste alkyle, cycloalkyle, alkyl-cycloalkyle, aryle, alkyl-aryle, hétérocyclyle ou alkyl-hétérocyclyle ; et
R²⁰¹ représente un reste alkyle, aryle, alkyl-aryle, hétérocyclyle ou alkyl-hétérocyclyle ;
où
le terme "cycloalkyle" désigne un reste hydrocarboné alicyclique saturé ou non saturé, le reste pouvant être non substitué ou substitué une fois ou plusieurs fois identiques ou différentes et, éventuellement, condensé au benzène et comprenant aussi des cycles aliphatiques bicycliques, tricycliques ou polycycliques,
"alkyle" désignant des restes hydrocarbonés acycliques saturés ou non saturés ("alcanyle", "alcényle" et "alcynyle") qui peuvent être ramifiés ou linéaires ainsi que non substitués ou substitués une fois ou substitués plusieurs fois identiques ou différentes ;
et "hétérocyclyle" désignant un reste organique monocyclique ou polycyclique dans lequel au moins un cycle contient un hétéroatome ou 2, 3, 4 ou 5 hétéroatomes identiques ou différents, qui est ou qui sont choisis dans le groupe comprenant N, O et S, le reste étant saturé ou non saturé et non substitué ou substitué une fois, ou substitué plusieurs fois identiques ou différentes ; pour la préparation d'un remède destiné à la prophylaxie et au traitement de la migraine.

16. Utilisation d'un dérivé substitué de 2-pyrrolidine-2-yl-1H-indole de formule générale (I) 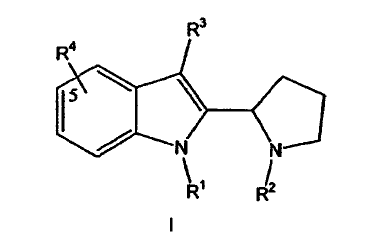 sous forme de son racémate, de ses stéréoisomères purs, ou sous forme de mélanges des stéréoisomères, dans un rapport de mélange quelconque ; sous la forme représentée ou sous forme de ses acides ou de ses bases ou sous forme de ses sels, ou sous forme de ses produits de solvatation, formule dans laquelle
R¹ représente H, un reste alkyle, cycloalkyle, alkyl-cycloalkyle, alkyl-aryle ou alkyl-hétérocyclyle ;
R² représente H, un reste alkyle, cycloalkyle, alkyl-cycloalkyle, alkyl-aryle, alkyl-hétérocyclyle, C (=O) -R²⁰ ou SO₂-R²¹ ;
R³ représente un reste alkyle, aryle ou hétérocyclyle ;
R⁴ représente H, F, Cl, Br, I, -CN, OR⁴⁰, un reste alkyle, cycloalkyle ou NO₂ ;
R²⁰ représente un reste alkyle, cycloalkyle, alkyl-cycloalkyle, aryle, alkyl-aryle, hétérocyclyle, alkyl-hétérocyclyle, OR²⁰⁰ ou NHR²⁰¹ ;
R²¹ représente un reste alkyle, aryle, alkyl-aryle, hétérocyclyle ou alkyl-hétérocyclyle ;
R⁴⁰ représente H, un reste alkyle, cycloalkyle, alkyl-cycloalkyle, aryle ou alkyl-aryle ;
R²⁰⁰ représente un reste alkyle, cycloalkyle, alkyl-cycloalkyle, aryle, alkyl-aryle, hétérocyclyle ou alkyl-hétérocyclyle ; et
R²⁰¹ représente un reste alkyle, aryle, alkyl-aryle, hétérocyclyle ou alkyl-hétérocyclyle ;
où
le terme "cycloalkyle" désigne un reste hydrocarboné alicyclique saturé ou non saturé, le reste pouvant être non substitué ou substitué une fois ou plusieurs fois identiques ou différentes et, éventuellement, condensé au benzène et comprenant aussi des cycles aliphatiques bicycliques, tricycliques ou polycycliques,
"alkyle" désignant des restes hydrocarbonés acycliques saturés ou non saturés ("alcanyle", "alcényle" et "alcynyle") qui peuvent être ramifiés ou linéaires ainsi que non substitués ou substitués une fois ou substitués plusieurs fois identiques ou différentes ;
et "hétérocyclyle" désignant un reste organique monocyclique ou polycyclique dans lequel au moins un cycle contient un hétéroatome ou 2, 3, 4 ou 5 hétéroatomes identiques ou différents, qui est ou qui sont choisis dans le groupe comprenant N, O et S, le reste étant saturé ou non saturé et non substitué ou substitué une fois, ou substitué plusieurs fois identiques ou différentes ;
pour la préparation d'un médicament destiné au traitement du choc septique, de la sclérose en plaques, de la maladie de Parkinson, de la maladie d'Alzheimer, de la maladie de Huntington, d'inflammations, d'une douleur inflammatoire, de l'ischémie cérébrale, du diabète, de la méningite, de l'artériosclérose et/ou pour la cicatrisation de plaies.
